# EUROPEAN PATENT APPLICATION

(11) **EP 4 636 090 A1**
(43) Date of publication of application: **22.10.2025**
(21) Application number: 23902830.1
(22) Date of filing: 15.12.2023
(51) Int. Cl.: C12N 15/63, C12N 15/113, C12N 5/10, C12N 15/67

(54) **UTR FOR IMPROVING TRANSLATION EFFICIENCY AND/OR STABILITY OF RNA MOLECULE AND USE THEREOF**

(30) Priority: 16.12.2022 WO PCT/CN2022/139593
(71) Applicant: Shenzhen Shenxin Biotechnology Co., Ltd., Shenzhen, Guangdong 518000 (CN)
(72) Inventor: HUANG, Hui, Shenzhen, Guangdong 518000 (CN); LI, Linxian, Shenzhen, Guangdong 518000 (CN)
(74) Representative: Nederlandsch Octrooibureau
(86) International application number: PCT/CN2023/139184
(87) International publication number: WO 2024/125637

(57) **Abstract**

Provided is a recombinant RNA molecule, which comprises a nucleotide sequence encoding a polypeptide of interest and 5'-UTR and/or 3'-UTR. The RNA molecule has improved translation efficiency and/or stability, and improves the expression level of a polypeptide and/or protein. Also provided are a vector encoding the recombinant RNA molecule, a pharmaceutical composition comprising the recombinant RNA molecule, and a method for treating or preventing a disease by using the recombinant RNA molecule.

## Description

### Technical Field

The present disclosure relates to the field of biotechnology, and relates to 5' and/or 3' untranslated regions (UTRs) for improving the translation efficiency and/or stability of an RNA molecule, a nucleic acid molecule comprising the UTR, a vector comprising the UTR or nucleic acid molecule, a pharmaceutical composition comprising the UTR, nucleic acid molecule or vector; and use of the UTR, nucleic acid molecule, vector and pharmaceutical composition.

### Background

mRNA (messenger RNA) is a single-stranded RNA molecule which is synthesized using the genomic DNA in the nucleus as template, subsequently transported to the cytoplasm, and translated into specific proteins in ribosomes to effect biological functions. mRNA has the potential to encode any protein, and due to its advantages like economical, safe, rapid, and flexible production, mRNA-based therapeutics exhibit broad application potential across diverse disease treatments, including the prevention of infectious disease, and the treatment of various diseases including cancer, and rare diseases.

mRNA vaccines have achieved clinical application in the prevention of infectious diseases, exhibiting several distinct advantages as compared to recombinant protein subunit vaccines, inactivated vaccines, or DNA vaccines. Firstly, as mRNA cannot infect the host or integrate into genomic DNA, the safety of mRNA is significantly increased. Secondly, the use of modified nucleosides can reduce the intrinsic immunogenicity of mRNA molecules and mitigate their degradation by the host, further improving the safety and stability of mRNA vaccines. Additionally, mRNA vaccines have the potential for high efficiency, rapid development, low-cost manufacturing, and simplified safety management due to the the high-yield synthesis of mRNA *in vitro.*

The untranslated region (UTR) of an mRNA regulates the translation, degradation, and localization of the gene, comprising a stem-loop structure, an upstream initiation codon, an upstream open reading frame, an internal ribosome entry site, and various cis-element that bind to RNA-binding proteins.

The UTRs play a critical role in the post-transcriptional regulation of gene expression, including modulating the nuclear export, translation efficiency, subcellular localization, and stability of mRNAs. UTRs may also have other functions, such as the specific incorporation of modified amino acid selenocysteine at the UGA codon in the mRNA encoding a selenoprotein, which is a process mediated by conserved stem-loop structures within the 3'-UTR.

A person skilled in the art knows that the translation efficiency of mRNA molecules directly affects the dosage and interval of an mRNA medicament, particularly an mRNA vaccine, finally influencing the bioavailability and determining the clinical utility thereof. Although there have been technical solutions for increasing the translation efficiency and stability of mRNA, such as the incorporation of UTRs, there remains a need for the further improvements in increasing the translation efficiency.

Therefore, there is a need for the identification or designing of UTRs capable of achieving higher translation efficiency and/or stability of mRNA.

### Summary

To address the aforementioned issues, the inventors have identified a plurality of 5'-UTRs and/or 3'-UTRs capable of enhancing the translation efficiency and/or stability of mRNA molecules. These UTRs are universal core elements that impart enhanced translation efficiency to mRNA molecules containing the UTRs, significantly increasing the expression of target genes and/or mRNA stability, showing broad applicability in the industrial production of mRNA-based therapeutics.

In a first aspect, provided is a recombinant RNA molecule, comprising: (1) a first nucleotide sequence encoding a polypeptide and/or protein of interest; and (2) a second nucleotide sequence comprising a 5'-untranslated region (5'-UTR); wherein the 5'-UTR comprises at least one polynucleotide selected from: (a) a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) a fragment of the 5'-UTR in (a); (c) a variant of the 5'-UTR in (a); and (d) a variant of the fragment in (b); and wherein the first nucleotide sequence and the second nucleotide sequence are not naturally occurring in the same RNA molecule.

In some embodiments, the gene is a human gene.

In some embodiments, the second nucleotide sequence comprises at least one of the following polynucleotides: (e) a 5'-UTR derived from at least one gene of genes PPIA, HPX, FTCD, CDK5RAP3, HSPA8, HBA1, and HBB; (f) a fragment of the 5'-UTR in (e); (g) a variant of the 5'-UTR in (e); and (h) a variant of the fragment in (f).

In some embodiments, the second nucleotide sequence comprises at least one of the following polynucleotides: an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21; preferably, the variant of the RNA encoded by a polynucleotide having a sequence as set forth in at least one of SEQ ID NOs: 1-21, the fragment of the RNA encoded by a polynucleotide having a sequence as set forth in at least one of SEQ ID NOs: 1-21, and the variant of the fragment of the RNA encoded by a polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 1-21.

In some embodiments, the second nucleotide sequence comprises at least one of: 5'-UTRs of at least two genes from the genes in (a); fragments of 5'-UTRs of at least two genes from the genes in (a); variants of 5'-UTRs of at least two genes from the genes in (a), and variants of the fragments of 5'-UTRs of at least two genes from the genes in (a).

In some embodiments, the second nucleotide sequence comprises at least one of: at least two copies of the 5'-UTR in (a); at least two copies of the fragment of the 5'-UTR in (a); at least two copies of the variant of the 5'-UTR in (a) and at least two copies of the variant of the fragment of the 5'-UTR in (a).

In some embodiments, the recombinant RNA molecule further comprises at least one of a promoter, a 5'-cap structure, a 3'-UTR, and a poly(A) tail.

In some embodiments, the recombinant RNA molecule further comprises at least one of a 5'-cap structure, a 3'-UTR, and a poly(A) tail.

In some embodiments, the 5'-cap structure comprises at least one of m⁷GpppG, m₂^{7,3'-O}GpppG, m⁷Gppp(5')N1 and m⁷Gppp(m^{2'-O})N1. In some embodiments, the 3'-UTR comprises at least one of: i) a 3'-UTR derived from at least one gene of albumin gene, α-globin gene, β-globin gene, tyrosine hydroxylase gene, lipoxygenase gene, and collagen α gene; ii) a variant of the 3'-UTR in i); iii) at least one of the 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1, a fragment, a variant, and a variant of the fragment thereof; preferably, at least one of an RNA encoded by a polynucleotide having a sequence as set forth in at least one of SEQ ID NOs: 22-49, a fragment of the RNA encoded by a polynucleotide having a sequence as set forth in at least one of SEQ ID NOs: 22-49, a variant of the RNA encoded by a polynucleotide having a sequence as set forth in at least one of SEQ ID NOs: 22-49, and a variant of the fragment of the RNA encoded by a polynucleotide having a sequence as set forth in at least one of SEQ ID NOs: 22-49; preferably, the fragment of the RNA encoded by a polynucleotide having a sequence as set forth in at least one of SEQ ID NOs: 22-49, the variant of the RNA encoded by a polynucleotide having a sequence as set forth in at least one of SEQ ID NOs: 22-49, and the variant of the fragment of the RNA encoded by a polynucleotide having a sequence as set forth in at least one of SEQ ID NOs: 22-49 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 22-49; iv) at least two copies of one of the polynucleotides in i), ii), or iii); or v) at least two polynucleotides selected from the group consisting of polynucleotides in i)-iii).

In some embodiments, the nucleotides forming the poly(A) tail comprise at least 20, at least 40, at least 80, at least 100, or at least 120 A nucleotides; preferably, the nucleotides forming the poly(A) tail comprise at least 20, at least 40, at least 80, at least 100, or at least 120 consecutive A nucleotides. In some embodiments, the nucleotides forming the poly(A) tail comprises one or more nucleotides other than A nucleotide; optionally, the nucleotides forming the poly(A) tail comprises two or more consecutive nucleotides other than A nucleotide.

In a second aspect, provided is another recombinant RNA molecule, comprising: (1) a first nucleotide sequence encoding a polypeptide and/or protein of interest; and (2) a second nucleotide sequence comprising a 3'-untranslated region (3'-UTR); wherein the 3'-UTR comprises at least one polynucleotide selected from: (a) a 3'-UTR derived from at least one gene selected from genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (b) a fragment of the 3'-UTR in (a); (c) a variant of the 3'-UTR in (a); and (d) a variant of the fragment in (b); wherein the first nucleotide sequence and the second nucleotide sequence are not naturally occurring in the same RNA molecule.

In some embodiments, the gene is a human gene.

In some embodiments, the second nucleotide sequence comprises at least one of the following polynucleotides: (e) a 3'-UTR derived from at least one gene of genes MPND, FBXW10, FBXW12, and PGLYRP1; (f) a fragment of the 3'-UTR in (e); (g) a variant of the 3'-UTR in (e); and (h) a variant of the fragment in (f). In some embodiments, the second nucleotide sequence comprises at least one of the following polynucleotides: an RNA encoded by a polynucleotide having a sequence as set forth in at least one of SEQ ID NOs: 22-48; a fragment of the RNA encoded by a polynucleotide having a sequence as set forth in at least one of SEQ ID NOs: 22-48; a variant of the RNA encoded by a polynucleotide having a sequence as set forth in at least one of SEQ ID NOs: 22-48; and a variant of the fragment of the RNA encoded by a polynucleotide having a sequence as set forth in at least one of SEQ ID NOs: 22-48; preferably, the variant of the RNA encoded by a polynucleotide having a sequence as set forth in at least one of SEQ ID NOs: 22-48, the fragment of the RNAencoded by a polynucleotide having a sequence as set forth in at least one of SEQ ID NOs: 22-48, and the variant of the fragment of the RNA encoded by a polynucleotide having a sequence as set forth in at least one of SEQ ID NOs: 22-48 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 22-48.

In some embodiments, the second nucleotide sequence comprises at least one of: 3'-UTRs of at least two genes from the genes in (a); fragments of 3'-UTRs of at least two genes from the genes in (a); variants of 3'-UTRs of at least two genes from the genes in (a) and fragments of variants of 3'-UTRs of at least two genes from the genes in (a).

In some embodiments, the second nucleotide sequence comprises at least one of: at least two copies of the 3'-UTR in (a); at least two copies of the fragment of the 3'-UTR in (a); at least two copies of the variant of the 3'-UTR in (a) and at least two copies of the variant of the fragment of the 3'-UTR in (a).

In some embodiments, the recombinant RNA molecule further comprises at least one of a promoter, a 5'-cap structure, a 5'-UTR, and a poly(A) tail.

In some embodiments, the recombinant RNA molecule further comprises at least one of a 5'-cap structure, a 3'-UTR, and a poly(A) tail.

In some embodiments, the 5'-cap structure comprises at least one of m⁷GpppG, m₂^{7,3'-O}GpppG, m⁷Gppp(5')N1 or m⁷Gppp(m^{2'-O})N1.

In some embodiments, the 5'-UTR comprises:
i) at least one of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; a fragment, a variant or a variant of a fragment thereof; preferably, an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21; preferably, the variant of the RNA encoded by a polynucleotide having a sequence as set forth in at least one of SEQ ID NOs: 1-21, the fragment of the RNAencoded by a polynucleotide having a sequence as set forth in at least one of SEQ ID NOs: 1-21, and the variant of the fragment of the RNA encoded by a polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 1-21; ii) at least two copies of one of the polynucleotides in i); or iii) at least two polynucleotides in i).

In some embodiments, the nucleotides forming the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides. In some embodiments, the nucleotides forming the poly(A) tail comprises one or more nucleotides other than A nucleotide.

In a third aspect, provided is a DNA molecule encoding the recombinant RNA molecule according to the present disclosure.

In a fourth aspect, provided is a vector comprising the DNA molecule according to the present disclosure.

In a fifth aspect, provided is a host cell comprising the recombinant RNA molecule, the DNA molecule or the vector according to present disclosure.

In a sixth aspecrt, provided is a lipid nanoparticle comprising the recombinant RNA molecule according to the present disclosure.

In a seventh aspect, provided is a pharmaceutical composition comprising the recombinant RNA molecule, the DNA molecule, the vector, the host cell, or the lipid nanoparticle according to the present disclosure, and a pharmaceutically acceptable carrier.

In an eighth aspect, provided is a vector comprising a first nucleotide sequence encoding a 5'-UTR and/or a second nucleotide sequence encoding a 3'-UTR, wherein:
the first nucleotide sequence comprises at least one of the following polynucleotides:
   (a)a polynucleotide encoding a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) a polynucleotide encoding a fragment of the 5'-UTR in (a); (c) a polynucleotide encoding a variant of the 5'-UTR in (a); and (d) a polynucleotide encoding a variant of the fragment in (b);
the second nucleotide sequence comprises at least one of the following polynucleotides:
   (e) a polynucleotide encoding a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (f) a polynucleotide encoding a fragment of the 3'-UTR in (e); (g) a polynucleotide encoding a variant of the 3'-UTR in (e); and (h) a polynucleotide encoding a variant of the fragment in (f);

In some embodiments, the gene is a human gene.

In some embodiments, the first nucleotide sequence comprises a 5'-UTR derived from at least one gene of genes PPIA, HPX, FTCD, CDK5RAP3, HSPA8, HBA1, and HBB, or a variant thereof.

In some embodiments, the second nucleotide sequence comprises a 3'-UTR derived from at least one gene of genes MPND, FBXW10, FBXW12, and PGLYRP1, or a variant thereof.

In some embodiments, the vector comprises the first nucleotide sequence and the second nucleotide sequence.

In some embodiments, the first nucleotide sequence comprises:
a polynucleotide having a sequence as set forth in at least one of SEQ ID NOs: 1-21; a fragment of the polynucleotide having a sequence as set forth in at least one of SEQ ID NOs: 1-21; a variant of the polynucleotide having a sequence as set forth in at least one of SEQ ID NOs: 1-21; and a variant of the fragment of the polynucleotide having a sequence as set forth in at least one of SEQ ID NOs: 1-21; preferably, the variant of the polynucleotide having a sequence as set forth in at least one of SEQ ID NOs: 1-21, the fragment of the polynucleotide having a sequence as set forth in at least one of SEQ ID NOs: 1-21, and the variant of the fragment of the polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% homology to the polynucleotide having a sequence as set forth in at least one of SEQ ID NOs: 1-21; ii) two copies of one of the polynucleotides in i); or iii) at least two polynucleotides in i).

In some embodiments, the second nucleotide sequence comprises: (1) a polynucleotide encoding a 3'-UTR derived from at least one gene of albumin gene, α-globin gene, β-globin gene, tyrosine hydroxylase gene, lipoxygenase gene, and collagen α gene; (2) a polynucleotide encoding a variant of the 3'-UTR in (1); (3) at least one of a polynucleotide having a sequence as set forth in at least one of SEQ ID NOs: 22-48; a fragment of the polynucleotide having a sequence as set forth in at least one of SEQ ID NOs: 22-48; a variant of the polynucleotide having a sequence as set forth in at least one of SEQ ID NOs: 22-48; and a variant of the fragment of the polynucleotide having a sequence as set forth in at least one of SEQ ID NOs: 22-48; preferably, the variant of the polynucleotide having a sequence as set forth in at least one of SEQ ID NOs: 22-48, the fragment of the polynucleotide having a sequence as set forth in at least one of SEQ ID NOs: 22-48, and the variant of the fragment of the polynucleotide having a sequence as set forth in at least one of SEQ ID NOs: 22-48 have at least 70%, 80%, 85%, 90%, 91%, 92%, 93%, 94%, 95%, 96%, 97%, 98%, or 99% identity to the polynucleotide having a sequence as set forth in at least one of SEQ ID NOs: 22-48; (4) two copies of one of the polynucleotides in (1), (2) or (3); or (5) at least two polynucleotides selected from the group consisting of polynucleotides in (1)-(3).

In some embodiments, the vector further comprises a polynucleotide encoding a poly(A) tail. In some embodiments, the nucleotides forming the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides. In some embodiments, the nucleotides forming the poly(A) tail comprises one or more nucleotides other than A nucleotide.

In a ninth aspect, provided is use of the recombinant RNA molecule, the DNA molecule, the vector, the host cell, the lipid nanoparticle, or the pharmaceutical composition according to the present disclosure for the manufacture of a medicament; preferably, the medicament is used for gene therapy, gene vaccination, or protein replacement therapy.

In some embodiments, the medicament is a nucleic acid medicament, wherein the nucleic acid comprises at least one of RNA, messenger RNA (mRNA), DNA, plasmid, ribosomal RNA (rRNA), single-guide RNA (sgRNA), and Cas9 mRNA.

In some embodiments, the medicament is used for the treatment and/or prevention of a disease; preferably, the disease is selected from the group consisting of: a rare disease, an infectious disease, a cancer, a genetic disease, an autoimmune disease, a diabete disease, a neurodegenerative disease, a cardiovascular disease, a renal vascular disease, and a metabolic disease; preferably, the cancer comprises one or more of lung cancer, gastric cancer, liver cancer, esophageal cancer, colon cancer, pancreatic cancer, brain cancer, lymphoma, leukemia, or prostate cancer; the genetic disease comprises one or more of hemophilia, thalassemia, and Gaucher's disease.

### Brief Description of the Drawings

Fig. 1A shows the flowchart for the construction of plasmid D.
Fig. 1B shows the schematic diagram for the construction and modification of plasmid D.
Fig. 2 shows the map of plasmid luciferase-pcDNA3.
Fig. 3 shows the map of plasmid B.
Fig. 4 shows the map of plasmid C.
Fig. 5 shows the map of plasmid D.
Fig. 6 shows the luciferase expression in HEK293 cells transfected with mRNAs containing different 5'-UTRs in Example 5.
Fig. 7 shows the luciferase expression in HEK293 cells transfected with mRNAs containing different 3'-UTRs in Example 6.
Fig. 8 shows the in vivo expression of mRNAs with identical 5'-UTR but different 3'-UTRs in mice.
Fig. 9 shows the in vivo expression of mRNAs with identical 3'-UTR but different 5'-UTRs in mice.

### Detailed Description

### I. Definitions

All patents, patent applications, scientific publications, manufacturer's instructions, and guidelines cited herein, above or below, are hereby incorporated by reference in their entireties. Nothing herein shall be construed as an admission that the present disclosure is not entitled to antedate such disclosure.

Unless otherwise specified, the scientific and technical terms used herein have the meanings commonly understood by those skilled in the art. Furthermore, the terminology related to protein and nucleic acid chemistry, molecular biology, cell and tissue culture, and microbiology used herein is widely used in the related fields (see, for example, Molecular Cloning: A Laboratory Manual, 2nd Edition, J. Sambrook et al. eds., Cold Spring Harbor Laboratory Press, Cold Spring Harbor 1989). In addition, to facilitate a better understanding of the present disclosure, definitions and explanations of relevant terms are provided below.

As used herein, the expressions "comprising", "including", "containing", and "having" are open-ended and mean that the specified elements, steps, or components are included but do not exclude other unlisted elements, steps, or components. The expression "consisting of" excludes any unspecified elements, steps, or components. The expression "consisting essentially of" means that the scope is limited to the specified elements, steps, or components, plus any optionally present elements, steps, or components that do not significantly affect the basic and novel characteristics of the claimed subject matter. It is understood that the expressions "consisting essentially of" and "consisting of" are encompassed within the meaning of the expression "comprising".

As used herein, unless the context clearly indicates otherwise, the singular forms "a", "an", and "the" used in the context describing the disclosure (particularly in the context of the claims) and similar references shall be interpreted to cover both the singular and plural. The terms "one or more" or "at least one" encompasses 1, 2, 3, 4, 5, 6, 7, 8, 9, or more.

The numerical ranges described herein should be understood to include any and all subranges encompassed therein. For example, the range "1 to 10" should be interpreted not only to include the explicitly stated values of 1 and 10 but also to include any individual value within the range of 1 to 10 (e.g., 2, 3, 4, 5, 6, 7, 8, and 9) and subranges (e.g., 1-2, 1.5-2.5, 1-3, 1.5-3.5, 2.5-4, 3-4.5, and the like). This principle also applies to ranges that specify only one numerical value as the minimum or maximum.

Unless otherwise specified, all methods described herein may be performed in any suitable order.

As used herein, the term "wild-type" refers to a sequence that is naturally occurring and has not been artificially modified, including naturally occurring mutants. The term "fragment" or "fragment of a nucleic acid" refers to a portion of a nucleic acid. For example, a nucleic acid that is truncated at the 5' and/or 3' end. A fragment of a nucleic acid comprises at least 50%, 60%, 70%, or 80% of the nucleic acid. Preferably, a fragment of a nucleic acid comprises at least 70% or 80% of the nucleic acid. More preferably, it comprises at least 90%, 95%, 96%, 97%, 98%, or 99% of the nucleotide residues. Typically, it may be a shorter portion of the full-length nucleic acid.

As used herein, the term "variant" in the context of nucleic acids refers to a nucleic acid variant in which at least one nucleotide differs from a reference nucleic acid (also referred to as the "parent"). Compared to the reference nucleic acid, a variant nucleic acid includes single or multiple nucleotide deletions, additions, mutations, and/or insertions, wherein deletion comprises the removal of one or more nucleotides from the reference nucleic acid; addition comprises the fusion of one or more nucleotides (e.g., 1, 2, 3, 5, 10, 20, 30, 50, or more nucleotides) to the 5' and/or 3' ends of the reference nucleic acid; mutation may comprise, but not limited to, substitution (e.g., removal of at least one nucleotide and insertion of another nucleotide in its place, such as transversion and transition); insertion comprises the addition of at least one nucleotide. The term "nucleic acid variant" as used herein encompasses both naturally occurring variants and engineered variants. Therefore, a "nucleic acid variant" as defined herein may be derived, isolated, related, based on, or homologous to the reference nucleic acid sequence. A "nucleic acid variant" may optionally have at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99% sequence identity to the corresponding naturally occurring (wild-type) nucleic acid or a homolog, fragment, or derivative thereof; preferably at least 70%, more preferably at least 80%, even more preferably at least 85%, even more preferably at least 90%, and most preferably at least 95% or even 97% sequence identity. It is understood that, for nucleic acid molecules, the term "variant" includes degenerate nucleic acid sequences, wherein a degenerate nucleic acid sequence according to the present disclosure is a nucleic acid that differs from the reference nucleic acid in its codon sequence due to the degeneracy of the genetic code.

In some embodiments, the nucleic acid variant is a variant of the 5'-UTR, a variant of the 3'-UTR, or a variant of the polyA tail. In some embodiments, the mutations introduced into the nucleic acid variant enable the variant to avoid enzymatic cleavage by preventing recognition by nucleases, to avoid binding with microRNAs, or to prevent the formation of complex secondary structures such as hairpins or G-quadruplexes. For example, the nucleic acid variant is a variant derived from the 3'-UTR of the ALB gene (GenBank accession number NM_000477.7), wherein one "A" in the 3'-UTR of the ALB gene is mutated to a "C" to prevent recognition by nucleases and enzymatic cleavage.

As used herein, the term "% identity" or "% similarity" refers to the percentage of identical nucleotides or amino acids in the optimal alignment between the sequences being compared. Differences between the two sequences may be distributed across local regions (segments) or the entire length of the sequences being compared. Typically, the identity between two sequences is determined after optimal alignment of segments or "comparison windows". Optimal alignment can be performed manually or using algorithms known in the art. Algorithms known in the art include, but not limited to, the local homology algorithm described by Smith and Waterman, 1981, Ads App. Math. 2, 482 and Neddleman and Wunsch, 1970, J. Mol. Biol. 48, 443; the similarity search method described by Pearson and Lipman, 1988, Proc. Natl Acad. Sci. USA 88, 2444; or the use of computer programs such as GAP, BESTFIT, FASTA, BLAST P, BLAST N, and TFASTA from Wisconsin Genetics Software Package, Genetics Computer Group, 575 Science Drive, Madison, Wis. For example, the percentage identity of two sequences can be determined using the BLASTN or BLASTP algorithms publicly available on the website of the National Center for Biotechnology Information (NCBI).

The "% identity" or "% similarity" may be obtained as follows: by determining the number of identical positions in the sequences being compared, dividing this number by the total number of positions compared (e.g., the number of positions in the reference sequence), and multiplying the result by 100 to obtain the % identity. In some embodiments, the degree of identity is given for regions that are at least about 50%, at least about 55%, at least about 60%, at least about 65%, at least about 70%, at least about 75%, at least about 80%, at least about 85%, at least about 90%, at least about 95%, or about 100%. In some embodiments, the degree of identity is given for the entire length of the reference sequence. Alignment to determine sequence identity may be performed using tools known in the art, preferably using optimal sequence alignment, for example, using Align with standard settings, preferably EMBOSS::needle, Matrix: Blosum62, Gap Open 10.0, Gap Extend 0.5.

Preferably, a fragment or variant of a specific nucleic acid or a nucleic acid having a specific degree of identity to the specific nucleic acid preferably possesses at least one functional characteristic of the specific nucleic acid and is preferably functionally equivalent to the specific nucleic acid, for example, exhibiting characteristics identical or similar to those of the specific nucleic acid.

As used herein, "nucleotide" comprises deoxyribonucleotides, ribonucleotides, derivatives of deoxyribonucleotides, and derivatives of ribonucleotides. As used herein, a "ribonucleotide" is a building block of ribonucleic acid (RNA), consisting of a base, a five-carbon sugar, and a phosphate group, and refers to a nucleotide having a hydroxyl group at the 2' position of the β-D-ribofuranosyl group. A "deoxyribonucleotide" is a building block of deoxyribonucleic acid (DNA), also consisting of a base, a five-carbon sugar, and a phosphate group, and refers to a nucleotide in which the hydroxyl group at the 2' position of the β-D-ribofuranosyl group is replaced by hydrogen, which is the primary chemical component of chromosomes.

"Nucleotide" is typically denoted by a single letter representing its base: "A" or "A nucleotide" refers to adenine deoxyribonucleotide or adenine ribonucleotide containing adenine, "C" or "C nucleotide" refers to cytosine deoxyribonucleotide or cytosine ribonucleotide containing cytosine, "G" or "G nucleotide" refers to guanine deoxyribonucleotide or guanine ribonucleotide containing guanine, "U" or "U nucleotide" refers to uridine ribonucleotide containing uracil, and "T" or "T nucleotide" refers to thymidine deoxyribonucleotide containing thymine.

As used herein, the term "nucleic acid" generally refers to any compound which is a polymer of deoxyribonucleotides (deoxyribonucleic acid, abbreviated as DNA) or ribonucleotides (ribonucleic acid, abbreviated as RNA), or a combination thereof. Additionally, nucleic acids herein also include derivatives of nucleic acids. The term "derivatives of nucleic acids" includes chemical derivatization of nucleic acids at the base, sugar, or phosphate of the nucleotides, as well as nucleic acids containing non-natural nucleotides and nucleotide analogs. Furthermore, nucleic acids herein may exist in the form of single-stranded or double-stranded linear or covalently closed circular molecules.

The terms "polynucleotide sequence", "nucleic acid sequence", and "nucleotide sequence" may be used interchangeably to denote the order of nucleotides in a polynucleotide. Those skilled in the art will understand that the DNA coding strand (sense strand) and the RNA encoded thereby can be considered to have the same nucleotide sequence, with deoxythymidine in the DNA coding strand sequence corresponding to uridine in the encoded RNA sequence. The RNA encoded by DNA refers to the RNA corresponding to the DNA, i.e., the polynucleotide in which all T nucleotides in the DNA are replaced with U nucleotides.

A polynucleotide may comprise one or multiple segments (nucleic acid fragments) (e.g., 1, 2, 3, 4, 5, 6, 7, 8 segments). For example, a polynucleotide may comprise a segment encoding a polypeptide of interest (e.g., the polypeptides and polypeptide antigens described herein). In specific embodiments, the polynucleotide may comprise a segment encoding a polypeptide of interest as well as a regulatory segment (including but not limited to those for transcriptional and translational regulation). In an embodiment, the regulatory segment comprises polynucleotides corresponding to one or more regulatory elements, including but not limited to: a promoter, a 5' untranslated region (5'-UTR), a 3' untranslated region (3'-UTR), and a poly(A) tail.

The term "promoter" refers to a polynucleotide located upstream of the 5' end of the coding region in a gene, which contains conserved sequences required for the specific binding of RNA polymerase and the initiation of transcription, and can activate RNA polymerase, enable RNA polymerase to accurately bind to the template DNA and initiate transcription with specificity. Promoters may be derived from viruses, bacteria, fungi, plants, insects, and animals. Representative examples of promoters comprise T7 phage promoter, T3 phage promoter, SP6 promoter, lac operon-promoter, tac promoter, SV40 late promoter, SV40 early promoter, RSV-LTR promoter, CMV IE promoter, SV40 early promoter, SV40 late promoter, and CMV IE promoter. As used herein, the term "5' untranslated region" or "5'-UTR" refers to the RNA sequence in mRNA that is located upstream of the coding sequence and is not translated into protein. In a gene, the 5'-UTR typically starts at the transcription initiation site and ends at the nucleotide upstream of the translation initiation codon of the coding sequence. The 5'-UTR may contain elements that regulate gene expression, such as ribosome binding sites, 5'-terminal oligopyrimidine tracts, and translation initiation signals like the Kozak sequence. mRNA may undergo post-transcriptional modification through the addition of a 5' cap. Therefore, the 5'-UTR in mature mRNA may also refer to the RNA sequence between the 5' cap and the initiation codon. As used herein, the term "3' untranslated region" or "3'-UTR" refers to the RNA sequence in mRNA that is located downstream of the coding sequence and is not translated into protein. The 3'-UTR in mRNA lies between the termination codon of the coding sequence and the poly(A) sequence, for example, starting from the nucleotide downstream of the termination codon and ending at the nucleotide upstream of the poly(A) sequence.

As used herein, "5' or 3'-UTR derived from gene A" refers to the 5' or 3'-UTR from the mRNA of gene A. The 5' or 3'-UTR derived from gene A may be the entire 5' or 3'-UTR of the mRNA of gene A, or it may be a portion of the 5' or 3'-UTR of the mRNA of gene A.

As used herein, the terms "polyadenylate", "poly(A) sequence", and "poly(A) tail" can be used interchangeably. Naturally occurring poly(A) sequences typically consist of adenine ribonucleotides. According to the present disclosure, the term "modified poly(A) sequence" refers to a poly(A) sequence that includes nucleotides or nucleotide segments other than adenine ribonucleotide. The poly(A) sequence is usually located at the 3' end of mRNA, for example, at the 3' end (downstream) of the 3'-UTR. As used herein, the term "5' cap structure": the 5' cap structure is typically located at the 5' end of mature mRNA. In some embodiments, the 5' cap structure is linked to the 5' end of mRNA via a 5'-5' triphosphate bond. The 5' cap structure is usually formed by a modified (e.g., methylated) ribonucleotide, particularly a derivative of guanine nucleotide. For example, m7GpppN (cap 0 or "cap0", formed by the 5' phosphate group of hnRNA reacting with the 5'-phosphate group of m7GTP under the action of guanylyltransferase to form a 5',5'-phosphodiester bond), where N is the terminal 5' nucleotide of the nucleic acid carrying the 5' cap structure. In some embodiments, the 5' cap structure comprises but not limited to, cap 0, cap 1 (a cap structure formed by further methylation of the 2'-OH group of the sugar moiety of the first nucleotide of hnRNA on the basis of cap 0, also referred to as "cap1"), cap 2 (a cap structure formed by further methylation of the 2'-OH group of the sugar moiety of the second nucleotide of hnRNA on the basis of cap 1, also referred to as "cap2"), cap 4, cap 0 analog, cap 1 analog, cap 2 analog, or cap 4 analog.

As used herein, the term "expression" encompasses the transcription and/or translation of a nucleotide sequence. Therefore, expression may involve the production of transcripts and/or polypeptides. The term "transcription" refers to the process of transcribing the genetic code from a DNA sequence into RNA (transcript). The term "*in vitro* transcription" refers to the synthesis of RNA, particularly mRNA, in a cell-free system (e.g., in an appropriate cell extract) (see, for example, Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). Vectors used to produce transcripts are also referred to as "transcription vectors" which contain the regulatory sequences required for transcription. The term "transcription" encompasses *"in vitro* transcription".

As used herein, the term "polypeptide" refers to a polymer comprising two or more amino acids covalently linked by peptide bonds. A "protein" may comprise one or more polypeptides, wherein the polypeptides interact with each other through covalent or non-covalent means.

As used herein, the term "host cell" refers to a cell used to receive, maintain, replicate, or express a polynucleotide or vector. The term "host cell" includes prokaryotic cells (e.g., *Escherichia coli*.) or eukaryotic cells (e.g., yeast cells and insect cells), for example, cells derived from a human, mouse, hamster, pig, goat, or primates. Cells may be derived from various tissue types and include primary cells and cell lines. Specific examples include keratinocytes, peripheral blood leukocytes, bone marrow stem cells, and embryonic stem cells. In other embodiments, the host cell is an antigen-presenting cell, particularly a dendritic cell, monocyte, or macrophage. Nucleic acid may exist in the host cell as a single copy or in several copies. In some embodiments, the host cell may be a cell in which the polypeptide of the present disclosure is expressed.

As used herein, the term "recombination" or "recombinant" means "produced by genetic engineering". Preferably, in the context of the present disclosure, a "recombinant substance", such as a recombinant RNA molecule, is non-naturally occurring. The term "naturally occurring" or "naturally existing" as used herein refers to the fact that a substance can be found in nature. For example, a peptide or nucleic acid that exists in an organism (including viruses) and can be isolated from natural sources without intentional artificial modification in experiments is naturally occurring.

In the context of the present disclosure, the term "plasmid" generally refers to a circular DNA molecule, but it may also encompass linearized DNA molecules. Specifically, the term "plasmid" also includes molecules obtained by linearizing a circular plasmid, for example, by digesting the circular plasmid with a restriction enzyme, thereby converting the circular plasmid molecule into a linear molecule. Plasmids can replicate, i.e., amplify independently of the genetic information stored as chromosomal DNA in cells, and can be used for cloning, i.e., for amplifying genetic information in bacterial cells. Preferably, the DNA plasmid is a medium-copy or high-copy plasmid, more preferably a high-copy plasmid. Examples of such high-copy plasmids include, for example, pUC and pTZ plasmids or any other plasmids containing a replication origin that supports high plasmid copy numbers (e.g., pMB1, pColE1).

The term "treatment" as used herein generally refers to achieving a desired pharmacological and/or physiological effect. Accordingly, the treatment of the present disclosure may involve the treatment of a certain disease state but may also encompass prophylactic treatment in terms of the complete or partial prevention of a disease or a symptom thereof. Preferably, the term "treatment" should be understood as therapeutic in partially or completely curing a disease and/or adverse effects and/or symptoms attributable to the disease. Treatment may also be prophylactic or preventive treatment, that is, measures taken to prevent a disease, such as to prevent infection and/or the onset of a disease.

Certain elements of the disclosure will be described herein. These elements are presented together with specific embodiments; however, it should be understood that they may be combined in any manner and in any number to give additional embodiments. The examples and preferred embodiments described herein should not be construed as limiting the disclosure solely to the specific embodiments. The specification should be understood to support and include those which combine the specific embodiments with any number of the disclosed and/or preferred elements. Furthermore, unless the context indicates otherwise, all permutations and combinations of the elements described herein should be considered as disclosed by the specification of the disclosure. For example, if in an embodiment, the 5'-UTR of a recombinant nucleic acid molecule comprises a polynucleotide with a sequence as set forth in SEQ ID NO: 1, and if in another embodiment, the 3'-UTR of the recombinant nucleic acid molecule comprises a polynucleotide with a sequence as set forth in SEQ ID NO: 22, the following embodiment is also the one directed in the disclosure: a recombinant nucleic acid molecule wherein the 5'-UTR comprises a polynucleotide with a sequence as set forth in SEQ ID NO: 1, and the 3'-UTR comprises a polynucleotide with a sequence as set forth in SEQ ID NO: 22.

### II. 5'-UTRs improving translation

The inventors have unexpectedly found that the translation efficiency of the encoded sequence can be improved by incorporating the 5'-UTR of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, or CDK7 into an mRNA.

The full names of the above genes are listed as follows.
PPIA: peptidylprolyl isomerase A;
HPX: hemopexin;
FTCD: formimidoyltransferase cyclodeaminase;
CDK5RAP3: CDK5 regulatory subunit associated protein 3;
HSPA8: heat shock protein family A (Hsp70) member 8;
HBA1: hemoglobin subunit alpha 1;
HBB: hemoglobin subunit beta;
MYSM1: Myb like, SWIRM and MPN domains 1;
LENG1: leukocyte receptor cluster member 1;
TMSB4X: thymosin beta 4 X-linked;
CASP4: caspase 4;
IFNA1: interferon alpha 1;
PGLYRP1: peptidoglycan recognition protein 1;
UCHL1: ubiquitin C-terminal hydrolase L1;
CPAMD8: C3 and PZP like, alpha-2-macroglobulin domain containing 8;
TTR: transthyretin;
APOA2: apolipoprotein A2;
GH1: growth hormone 1);
DTYMK: deoxythymidylate kinase;
APOC2: apolipoprotein C2;
CDK7: cyclin-dependent kinase 7.

Accordingly, provided is a 5'-UTR, comprising at least one polynucleotide selected from the following:
(a) a 5'-UTR derived from at least one gene selected from genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7;
(b) a fragment of the 5'-UTR described in (a);
(c) a variant of the 5'-UTR in (a); and
(d) a variant of the fragment in (b).

In some embodiments, the gene is a gene from a eukaryotic organism.

In some embodiments, the gene is a gene from a chordate.

In some embodiments, the gene is a gene from a vertebrate.

In some embodiments, the gene is a gene from a mammal.

In some embodiments, the gene is a gene from a primate.

In some embodiments, the genes are each independently a human (*Homo sapiens*) gene, a *Pan paniscus* gene, a *Pan troglodytes* gene, or a *Gorilla gorilla gorilla* gene.

In some embodiments, the gene is a human gene.

In some embodiments, the gene is human PPIA gene, human HPX gene, *Pan paniscus* HPX gene, human FTCD gene, human CDK5RAP3 gene, *Gorilla gorilla gorilla* CDK5RAP3 gene, human HSPA8 gene, human HBA1 gene, human HBB gene, human MYSM1 gene, *Pan paniscus* MYSM1 gene, human LENG1 gene, human TMSB4X gene, human CASP4 gene, human IFNA1 gene, human PGLYRP1 gene, *Gorilla gorilla gorilla* PGLYRP1 gene, human UCHL1 gene, human CPAMD8 gene, *Pan troglodytes* CPAMD8 gene, human TTR gene, *Gorilla gorilla gorilla* TTR gene, human APOA2 gene, human GH1 gene, human DTYMK gene, *Pan paniscus* DTYMK gene, human APOC2 gene, *Pan troglodytes* APOC2 gene, and human CDK7 gene.

In some embodiments, PPIA gene has the GenBank Accession Number of BC137057.1; HPX gene has the GenBank Accession Number of AH002827.2; FTCD gene has the GenBank Accession Number of NM_006657.3; CDK5RAP3 gene has the GenBank Accession Number of AK223387.1; HSPA8 gene has the GenBank Accession Number of NM_006597.6; HBA1 gene has the GenBank Accession Number of NM_000558.5; HBB gene has the GenBank Accession Number of NM_000518.5; MYSM1 gene has the GenBank Accession Number of NM_001085487.2; LENG1 gene has the GenBank Accession Number of NM_024316.3; TMSB4X gene has the GenBank Accession Number of NM_021109.4; CASP4 gene has the GenBank Accession Number of NM_001225.3; IFNA1 gene has the GenBank Accession Number of NM_024013.3; PGLYRP1 gene has the GenBank Accession Number of NM_005091.2; UCHL1 gene has the GenBank Accession Number of NG_012931.1; CPAMD8 gene has the GenBank Accession Number of NG_054892.1; TTR gene has the GenBank Accession Number of NM_000371.3; APOA2 gene has the GenBank Accession Number of NM_001643.2; GH1 gene has the GenBank Accession Number of NM_000515.5; DTYMK gene has the GenBank Accession Number of NM_001165031.1; APOC2 gene has the GenBank Accession Number of NM_000483.4; CDK7 gene has the GenBank Accession Number of AY130859.1.

In some embodiments, the polynucleotides enconding 5'-UTRs of human genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7 are as shown in Table 1.

**Table 1: Identified 5'-UTRs Improving Translation Efficiency**

| **5'-UTRs and Source Genes** | **Nucleotide Sequences Encoding 5'-UTRs** |
|---|---|
| 5UTR-NO2 | ACTCTTCTGGTCCCCACAGACTCAGAGAGAACCCACC (SEQ ID NO:1) |
| Homo sapiens isolate Novin-B mutant hemoglobin subunit alpha 1 (HBA1) | |
| 5UTR-NO4 | GACGGACGGGGTCAGGTCCCATC (SEQ ID NO:2) |
| Homo sapiens Myb like, SWIRM and MPN domains 1 (MYSM1) | |
| 5UTR-NO5 | CAGCTACGACGCC (SEQ ID NO:3) |
| Homo sapiens leukocyte receptor cluster member 1(LENG1) | |
| 5UTR-NO7 | AGGCACAGACACCAAGGACAGAGACGCTGGCTAGGCCG CCCTCCCCACTGTTACCAAC (SEQ ID NO:4) |
| Homo sapiens apolipoprotein A2 (APOA2) | |
| 5UTR-NO10 | AGGATTCCAAGGCCCAACTCCCCGAACCACTCAGGGTC CTGTGGACAGCTCACCTAGCTGCA (SEQ ID NO:5) |
| Homo sapiens growth hormone 1 (GH1) | |
| 5UTR-NO11 | ACATTTGCTTCTGACACAACTGTGTTCACTAGCAACCTCA AACAGACACC (SEQ ID NO:6) |
| Homo sapiens hemoglobin subunit beta (HBB) | |
| 5UTR-NO12 | AACTCTATATAGGGAGTTCAACTGGTCACCCAGAGCTGTC CTGTGGCCTCTGCAGCTCAGC (SEQ ID NO:7) |
| Homo sapiens hemopexin (HPX) | |
| 5UTR-NO13 | CTCATTGAACTCGCCTGCAGCTCTTGGGTTTTTTGTGGCT TCCTTCGTTATTGGAGCCAGGCCTACACCCCAGCAACC (SEQ ID NO:8) |
| Homo sapiens heat shock protein family A (Hsp70) member 8 (HSPA8) | |
| 5UTR-NO14 | GAACGTGGTATAAAAGGGGCGGGAGGCCAGGCTCGTGC CGTTTTGCAGACGCCACCGCCGAGGAAAACCGTGTACTA TTAGCC (SEQ ID NO:9) |
| Homo sapiens peptidylprolyl isomerase A (PPIA) | |
| 5UTR-NO16 | GACAACTCGGTGGTGGCCACTGCGCAGACCAGACTTCG CTCGTACTCGTGCGCCTCGCTTCGCTTTTCCTCCGCAAC C (SEQ ID NO:10) |
| Homo sapiens thymosin beta 4 X-linked (TMSB4X) | |
| 5UTR-NO17 | ATACATAGTTTACTTTCATTTTTGACTCTGAGGCTCTTTCC AACGCTGTAAAAAAGGACAGAGGCTGTTCCCT (SEQ ID NO:11) |
| Homo sapiens caspase 4 (CASP4) | |
| 5UTR-NO18 | CGGAGGCTCGGCCACAACGCCACTGGATTGGTGGTAGG GCGGGGCGGGCCACAGTCTCCAGCCTGAAGCGGAAGT GGAGGAAAG (SEQ ID NO:12) |
| Homo sapiens CDK5 regulatory subunit associated protein 3 (CDK5RAP3) | |
| 5UTR-NO20 | AGAACCTAGAGCCCAAGGTTCAGAGTCACCCATCTCAGC AAGCCCAGAAGTATCTGCAATATCTACG (SEQ ID NO:13) |
| Homo sapiens interferon alpha 1 (IFNA1) | |
| 5UTR-NO22 | CCGGGCGCTCCTAGCGGCCTCCCGGACCCTGCCGCCCT GCCACT (SEQ ID NO:14) |
| Homo sapiens peptidoglycan recognition protein 1 (PGLYRP1) | |
| 5UTR-NO23 | AGTGCGTCTGGCCGGCGCTTTATAGCTGCAGCCTGGGC GGCTCCGCTAGCTGTTTTTCGTCTTCCCTAGGCTATTTCT GCCGGGCGCTCCGCGAAG (SEQ ID NO:15) |
| Homo sapiens ubiquitin C-terminal hydrolase L1 (UCHL1) | |
| 5UTR-NO25 | GCCCCAGCTTGGCCGGGCGCGGAGCGGGGCGC (SEQ ID NO:16) |
| Homo sapiens C3 and PZP like, alpha-2-macroglobulin domain containing 8 (CPAMD8) | |
| 5UTR-NO26 | GAGACCCCAGCGGCGGGCGGTGGACAGTC (SEQ ID NO:17) |
| Homo sapiens deoxythymidylate kinase (DTYMK) | |
| 5UTR-NO29 | CTTCCATCTGCCCAGAGAGGAGCATCTGGATCAAGGTGC TGGCC (SEQ ID NO:18) |
| Homo sapiens formimidoyltransferase cyclodeaminase (FTCD) | |
| 5UTR-NO34 | GTTGACTAAGTCAATAATCAGAATCAGCAGGTTTGCAGTC AGATTGGCAGGGATAAGCAGCCTAGCTCAGGAGAAGTGA GTATAAAAGCCCCAGGCTGGGAGCAGCCATCACAGAAGT CCACTCATTCTTGGCAGG (SEQ ID NO:19) |
| Homo sapiens transthyretin (TTR) | |
| 5UTR-NO37 | GTTGTGGCTGTGGAGCGGAAGTGGGTCTCAACCACTATA AATCCTCTCTGTGCCCGTCCGGAGCTGGTGAGGACAGC CTGCCAGAGTCTGGTCTCTGGACACT (SEQ ID NO:20) |
| Homo sapiens apolipoprotein C2 (APOC2) | |
| 5UTR-NO42 | GGAACGCCAACCGCCTGGGCCTAGCGCAGCTTCCTCCG CCCACCACGGAAGTGAGGCGGGGATACTAAAGCGACGG AGCCCGGTGGACGGAAGTGGGTGTTGGAGGCTTTAAGG TAGCTTTAAATTCGTGTTGTCCTGGGAGCTCGCCCTTTTC GGCTGGAGTCGGGCTTTACGGCGCCGG (SEQ ID NO:21) |
| Homo sapiens cyclin-dependent kinase 7 (CDK7) | |

Accordingly, in some embodiments, the 5'-UTR comprises at least one of the following polynucleotides: an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21. In some embodiments, the fragment, the variant, or the variant of the fragment of the RNA encoded by a polynucleotide having a sequence as set forth in at least one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in at least one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21. It should be understood that "a fragment, variant, or variant of a fragment of A" herein is an abbreviation for "a fragment of A, a variant of A, or a variant of a fragment of A". For example, "a fragment, a variant, and a variant of the fragment of the RNA encoded by a polynucleotide having a sequence as set forth in at least one of SEQ ID NOs: 1-21" refers to a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21.

In a preferred embodiment, the gene is selected from at least one of PPIA, HPX, FTCD, CDK5RAP3, HSPA8, HBA1, and HBB. In some embodiments, the 5'-UTR comprises at least one of the following polynucleotides:1) an RNA encoded by a polynucleotide having a sequence as set forth in SEQ ID NO: 9, 7, 18, 12, 8, 1, or 6; 2) a fragment of the RNA in 1); 3) a variant of the RNA in 1); and a variant of the fragment in 2). In some embodiments, the fragment, the variant, or the variant of the fragment of the RNA encoded by a polynucleotide having a sequence as set forth in SEQ ID NO: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

The 5'-UTR of the present disclosure may comprise two or more tandem 5'-UTRs of the above genes, fragments of 5'-UTRs of the above genes, variants of 5'-UTRs of the above genes, or variants of fragments of 5'-UTRs of the above genes.

In some embodiments, the 5'-UTR comprises at least one of the following polynucleotides: (a) 5'-UTRs derived from at least two genes of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) fragments of the 5'-UTRs of at least two genes in (a); (c) variants of the 5'-UTRs of at least two genes in (a); and (d) variants of the fragments in (b). In some embodiments, the 5'-UTR comprises at least one of the following polynucleotides: (e) 5'-UTRs derived from at least two genes of genes PPIA, HPX, FTCD, CDK5RAP3, HSPA8, HBA1, and HBB; (f) fragments of the 5'-UTRs in (e); (g) variants of the 5'-UTRs in (e); and (h) variants of the fragments in (f).

In some embodiments, the 5'-UTR comprises at least one of the following polynucleotides: RNAs encoded by at least two polynucleotides having a sequence as set forth in SEQ ID NOs: 1-21; fragments of RNAs encoded by at least two polynucleotides having a sequence as set forth in SEQ ID NOs: 1-21; variants of RNAs encoded by at least two polynucleotides having a sequence as set forth in SEQ ID NOs: 1-21; and variants of fragments of RNAs encoded by at least two polynucleotides having a sequence as set forth in SEQ ID NOs: 1-21. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the 5'-UTR comprises at least one of the following polynucleotides: RNAs encoded by at least two polynucleotides having a sequence as set forth in SEQ ID NOs: 9, 7, 18, 12, 8, 1, and 6; fragments of RNAs encoded by at least two polynucleotides having a sequence as set forth in SEQ ID NOs: 9, 7, 18, 12, 8, 1, and 6; variants of RNAs encoded by at least two polynucleotides having a sequence as set forth in SEQ ID NOs: 9, 7, 18, 12, 8, 1, and 6; and variants of fragments of RNAs encoded by at least two polynucleotides having a sequence as set forth in SEQ ID NOs: 9, 7, 18, 12, 8, 1, and 6. In some embodiments, the fragment, the variant, or the variant of the fragment of the RNA encoded by a polynucleotide having a sequence as set forth in SEQ ID NOs: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NOs: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

In some embodiments, the 5'-UTR comprises at least one of the following polynucleotides: a): at least two copies of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; b) at least two copies of a fragment of the 5'-UTR of at least one gene from the genes in a); c) at least two copies of a variant of the 5'-UTR of at least one gene from the genes in a); and d) at least two copies of a variant of the fragment of the 5'-UTR of at least one gene from the genes in a). In some embodiments, the 5'-UTR comprises at least two copies of a 5'-UTR derived from at least one gene of genes PPIA, HPX, FTCD, CDK5RAP3, HSPA8, HBA1, and HBB. Preferably, at least two copies are two copies, three copies, four copies, five copies, six copies, seven copies, eight copies, or nine copies.

In some embodiments, the 5'-UTR comprises at least one of the following polynucleotides: at least two copies of the RNA encoded by a polynucleotide having a sequence as set forth in one of SEQ ID NOs: 1-21; at least two copies of a fragment of the RNA encoded by a polynucleotide having a sequence as set forth in one of SEQ ID NOs: 1-21; at least two copies of a variant of the RNA encoded by a polynucleotide having a sequence as set forth in one of SEQ ID NOs: 1-21; and at least two copies of a variant of the fragment of the RNA encoded by a polynucleotide having a sequence as set forth in one of SEQ ID NOs: 1-21. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the 5'-UTR comprises at least one of the following polynucleotides: at least two copies of the RNA encoded by a polynucleotide having a sequence as set forth in one of SEQ ID NOs: 9, 7, 18, 12, 8, 1, and 6; at least two copies of a fragment of the RNA encoded by a polynucleotide having a sequence as set forth in one of SEQ ID NOs: 9, 7, 18, 12, 8, 1, and 6; at least two copies of a variant of the RNA encoded by a polynucleotide having a sequence as set forth in one of SEQ ID NOs: 9, 7, 18, 12, 8, 1, and 6; and at least two copies of a variant of the fragment of the RNA encoded by a polynucleotide having a sequence as set forth in one of SEQ ID NOs: 9, 7, 18, 12, 8, 1, and 6. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 9, 7, 18, 12, 8, 1, and 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in one of SEQ ID NOs: 9, 7, 18, 12, 8, 1, and 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

### III. 3'-UTRs improving translation

The inventors have unexpectedly found that the translation efficiency of the encoded sequence can be increased by incorporating the 3'-UTR derived from genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1 or NFKB2 into an mRNA.

The full names of the aforementioned genes are listed as follows.
MPND: MPN domain containing;
FBXW10: F-box and WD repeat domain containing 10;
FBXW12: F-box and WD repeat domain containing 12;
PGLYRP1: peptidoglycan recognition protein 1;
HPX: hemopexin;
CDK7: cyclin dependent kinase 7;
APOC2: apolipoprotein C2;
PFN1: profilin 1;
RBP4: retinol binding protein 4;
FTCD: formimidoyltransferase cyclodeaminase;
NAAA: N-acylethanolamine acid amidase;
ALB: albumin;
GSDMD: gasdermin D;
FBXL8: F-box and leucine rich repeat protein 8;
ORM1: orosomucoid 1;
CASP4: caspase 4;
CHMP2A: charged multivesicular body protein 2A;
LENG1: leukocyte receptor cluster member 1;
MYCBPAP: MYCBP associated protein;
APOC1: apolipoprotein C1;
GAPDH: glyceraldehyde-3-phosphate dehydrogenase;
HSPA8: heat shock protein family A (Hsp70) member 8;
APOA2: apolipoprotein A2;
UCHL1: ubiquitin C-terminal hydrolase L1;
TSG101: tumor susceptibility 101;
NAE1: NEDD8 activating enzyme E1 subunit 1;
NFKB2: nuclear factor kappa B subunit 2.

Accordingly, provided is a 3'-UTR, comprising at least one polynucleotide selected from the following:
(a) a 3'-UTR derived from at least one gene selected from genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2;
(b) a fragment of the 3'-UTR in (a);
(c) a variant of the 3'-UTR in (a); and
(d) a variant of the fragment in (b).

In some embodiments, the gene is a gene from a eukaryotic organism.

In some embodiments, the gene is a gene from a chordate.

In some embodiments, the gene is a gene from a vertebrate.

In some embodiments, the gene is a gene from a mammal.

In some embodiments, the gene is a gene from a primate.

In some embodiments, the genes are each independently a human (*Homo sapiens*) gene, a *Pan paniscus* gene, a *Pan troglodytes* gene, or a *Gorilla gorilla gorilla* gene.

In some embodiments, the gene is a human gene.

In some embodiments, the gene is at least one of human MPND gene, *Pan paniscus* MPND gene, human FBXW10 gene, human FBXW12 gene, *Pan paniscus* FBXW12 gene, human PGLYRP1 gene, human HPX gene, human CDK7 gene, human APOC2 gene, human PFN1 gene, human RBP4 gene, human FTCD gene, human NAAA gene, human ALB gene, human GSDMD gene, human FBXL8 gene, *Pan paniscus* FBXL8 gene, *Gorilla gorilla gorilla* FBXL8 gene, human ORM1 gene, human CASP4 gene, human CHMP2A gene, *Pan paniscus* CHMP2A gene, human LENG1 gene, *Pan paniscus* LENG1 gene, human MYCBPAP gene, human APOC1 gene, human GAPDH gene, human HSPA8 gene, human APOA2 gene, human UCHL1 gene, human TSG101 gene, human NAE1 gene, *Pan paniscus* NAE1 gene, human NFKB2 gene, and *Pan paniscus* NFKB2 gene.

In some embodiments, MPND gene has the GenBank Accession Number of NM_001300862.1; FBXW10 gene has the GenBank Accession Number of NM_001267586.2; FBXW12 gene has the GenBank Accession Number of NM_001159929.1; PGLYRP1 gene has the GenBank Accession Number of NM_005091.3; HPX gene has the GenBank Accession Number of AH002827.2; CDK7 gene has the GenBank Accession Number of AY130859.1; APOC2 gene has the GenBank Accession Number of NM_000483.5; PFN1 gene has the GenBank Accession Number of NM_005022.4; RBP4 gene has the GenBank Accession Number of NM_006744.4; FTCD gene has the GenBank Accession Number of NM_006657.3; NAAA gene has the GenBank Accession Number of NM_001363719.2; ALB gene has the GenBank Accession Number of NM_000477.7; GSDMD gene has the GenBank Accession Number of NM_024736.7; FBXL8 gene has the GenBank Accession Number of NM_018378.2; ORM1 gene has the GenBank Accession Number of NM_000607.4; CASP4 gene has the GenBank Accession Number of NM_001225.3; CHMP2A gene has the GenBank Accession Number of NM_198426.3; LENG1 gene has the GenBank Accession Number of NM_024316.3; MYCBPAP gene has the GenBank Accession Number of NM_032133.6; APOC1 gene has the GenBank Accession Number of NG_012859.1; GAPDH gene has the GenBank Accession Number of NM_002046.7; HSPA8 gene has the GenBank Accession Number of NM_006597.6; APOA2 gene has the GenBank Accession Number of NM_001643.2; UCHL1 gene has the GenBank Accession Number of NG_012931.1; TSG101 gene has the GenBank Accession Number of NM_006292.4; NAE1 gene has the GenBank Accession Number of NM_003905.4; NFKB2 gene has the GenBank Accession Number of NM_001261403.3.

In some embodiments, the polynucleotides enconding 3'-UTRs of human genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2 are as shown in Table 2.

**Table 2: Identified 3'-UTRs Improving Translation Efficiency**

| **3'UTRs and Source Genes** | **Nucleotide Sequences Encoding 3'-UTRs** |
|---|---|
| 3UTR-NO4 | ACCAGCCTTGGGAAATTACAATGTTTTACAATAAACAGAAAGCC AAGCGG (SEQ ID NO:22) |
| Homo sapiens F-box and WD repeat domain containing 10 (FBXW10) | |
| 3UTR-NO5 | TATGGAAACTAACAAAATTGACCTTGCATCATCTTCTGCAATGTA GTAAAGAAATTCTATTTGC (SEQ ID NO:23) |
| Homo sapiens F-box and WD repeat domain containing 12 (FBXW12) | |
| 3UTR-NO6 | GCCTTCCAGGGCAGGGTGGGCTCCAGTTGTCTTGAGGGTCCG GATGGGCTCAGGTAATAAAGAAACGGAAGCAGCAGCCAGCC (SEQ ID NO:24) |
| Homo sapiens MPN domain containing (MPND) | |
| 3UTR-NO8 | GGCCCTGCTGATCCGCACCCCATTCCTCCCCTCCCATGGCCAA AAACCCCACTGT (SEQ ID NO:25) |
| Homo sapiens peptidoglycan recognition protein 1 (PGLYRP1) | |
| 3UTR-NO10 | GTGCCCCTGCCACTCCGAGATAACCAGTGGATGCCCAGGATCT TTTACCACAACCCCTCTGTAATAAAAGAGATTTGACACT (SEQ ID NO: 26) |
| Homo sapiens charged multivesicular body protein 2A (CHMP2A) | |
| 3UTR-NO11 | CTCCTGAGGGGGTACAGGAGAGGCCGCTGCTGCCAGCCGTCA TATAAAACTATTTATTCATAAATATTTTCCAAAAT (SEQ ID NO:27) |
| Homo sapiens leukocyte receptor cluster member 1 (LENG1) | |
| 3UTR-NO13 | CTCTCGGGACCAAGCAACCTTCTGGAAAACGGGTTAATAAATAA ATCAATAAAGAACCTTCAAGTTTCTACT (SEQ ID NO:28) |
| Homo sapiens MYCBP associated protein (MYCBPAP) | |
| 3UTR-NO14 | GGGGCCTTCTGACATGAGTCTGGCCTGGCCCCACCTCCTAGTT CCTCATAATAAAGACAGATTGCTTCTTCGCTTCTCACTGAGGGG CCTTCTGACATGAGTCTGGCCTGGCCCCACCTCCCCAGTTTCT CATAATAAAGACAGATTGCTTCTTCACTTGAATCAAGGGACCT (SEQ ID NO:29) |
| Homo sapiens hemopexin (HPX) | |
| 3UTR-NO15 | AGAGAACACTGGACAACATTTTACTACTGAGGGAAATAGCCAAA AAGGCAAATAATGGAAAAATAGTAAACATTAAGTAAATGCTGTAG AAGTGAGTTTGTAAATATTCTACACATGTAAAATATGTAAAACTAT GGGTTATTTTTATTAAATGTATTTTAAAATAAAAATTTAATTCTGGT TTTTCTGATTAGAGTGCAAAAGTGAGAAAAGTTCAATACTCTTG AAATGTAGAATTGAAAATGCATTAGGGAAAACTTAATAAAAATTAT TACCAGTTATTTGGAAGATCTG (SEQ ID NO:30) |
| Homo sapiens cyclin dependent kinase 7 (CDK7) | |
| 3UTR-NO16 | GGACCTGAAGGGTGACATCCCAGGAGGGGCCTCTGAAATTTC CCACACCCCAGCGCCTGTGCTGAGGACTCCCTCCATGTGGCC CCAGGTGCCACCAATAAAAATCCTACAGAAAATTC (SEQ ID NO:31) |
| Homo sapiens apolipoprotein C1 (APOC1) | |
| 3UTR-NO17 | CAGCCAGACCCCCCATCAGTGGACAAGGGGAGAGTCCCCTAC TCCCCTGATCCCCCAGGTTCAGACTGAGCTCCCCCTTCCCAGT AGCTCTTGCATCCTCCTCCCAACTCTAGCCTGATTCTTTTCAATA AAAAATACAATTCAAGTTGCTTCTCATGGATGGCACTGCTTTTCT GAGGACTCAAGGGCCAAGATGGAGGGGCTGACTCAGTCCAGC CAACATTTAATGAGCACCTACTTTATGTATGGAGCTCTAACCCAT GGGTCCATGGGAATAAAGCAGTGAATAGTAACAATAAATAATCG TAACA (SEQ ID NO:32) |
| Homo sapiens apolipoprotein C2 (APOC2) | |
| 3UTR-NO19 | GACCCCTGGACCACCAGCCCCAGCAAGAGCACAAGAGGAAGA GAGAGACCCTCACTGCTGGGGAGTCCCTGCCACACTCAGTCC CCCACCACACTGAATCTCCCCTCCTCACAGTTGCCATGTAGAC CCCTTGAAGAGGGGAGGGGCCTAGGGAGCCGCACCTTGTCAT GTACCATCAATAAAGTACCCTGTGCTCAACC (SEQ ID NO:33) |
| Homo sapiens glyceraldehyde-3-phosphate dehydrogenase (GAPDH) | |
| 3UTR-NO22 | GCCAACCAAGTGTAGATGTAGCATTGTTCCACACATTTAAAACA TTTGAAGGACCTAAATTCGTAGCAAATTCTGTGGCAGTTTTAAA AAGTTAAGCTGCTATAGTAAGTTACTGGGCATTCTCAATACTTGA ATATGGAACATATGCACAGGGGAAGGAAATAACATTGCACTTTAT AAACACTGTATTGTAAGTGGAAAATGCAATGTCTTAAATAAAACT ATTTAAAATTGGCACCATA (SEQ ID NO:34) |
| Homo sapiens heat shock protein family A (Hsp70) member 8 (HSPA8) | |
| 3UTR-NO23 | CAGGACACAGCCTTGGATCAGGACAGAGACTTGGGGGCCATC CTGCCCCTCCAACCCGACATGTGTACCTCAGCTTTTTCCCTCA CTTGCATCAATAACTTCTGTGTTTGGAACAGCTAA (SEQ ID NO:35) |
| Homo sapiens orosomucoid 1 (ORM1) | |
| 3UTR-NO24 | CCTCGTCTGTCCCTTCCCCTTCACCGCTCCCCACAGCTTTGCA CCCCTTTCCTCCCCATACACACACAAACCATTTTATTTTTTGGGC CATTACCCCATACCCCTTATTGCTGCCAAAACCACATGGGCTGG GGGCCAGGGCTGGATGGACAGACACCTCCCCCTACCCATATC CCTCCCGTGTGTGGTTGGAAAACTTTTGTTTTTGGGTTTTTT TTTTCTGAATAAAAAAGATTCTACTAACAA (SEQ ID NO:36) |
| Homo sapiens profilin 1 (PFN1) | |
| 3UTR-NO25 | CAATATCAAGAATCTAGTTTCATCTGAGAACTTCTGATTAGCTCT CAGTCTTCAGCTCTATTTATCTTAGGAGTTTAATTTGCCCTTCTC TCCCCATCTTCCCTCAGTTCCCATAAAACCTTCATTACACATAAA GATACACGTGGGGGTCAGTGAATCTGCTTGCCTTTCCTGAAAG TTTCTGGGGCTTAAGATTCCAGACTCTGATTCATTAAACTATAGT CACCCGTGTCCTGTGA (SEQ ID NO:37) |
| Homo sapiens retinol binding protein 4 (RBP4) | |
| 3UTR-NO32 | CGAAACCCCAGGGACAGCAGGACCTTCGACGCTGGAAGGATA GCCTCGCTTCCCAGGTGCAGCTCCCGGCAGGTCTGGACAGTT CCACAGAGCACGGTACTGGCCCTGTCCTCAGTTCCCGTCCAC CCTTCAACCAGTGGCTCCTGGGAGACTTTTCCTCCCTGCCTCA GCTCCCTGCCCAGCCAGCCTCCTGTCTCCAGAGAAGTTCTCC ATTAAAAAATAATTTAGCAAATC (SEQ ID NO:38) |
| Homo sapiens formimidoyltransferase cyclodeaminase (FTCD) | |
| 3UTR-NO33 | AAGCAGGCCAGGTGCGGTGGCTCACGCCTGTAATCCCAGCCC TTTGGGAGGCCAAGGCGGGTGGATCACTTGAGGTCAGGAGTT CAAGACCAGCCTGGCCAACATGGTGAAACCTCGTCTCTACTAA AAATACAAAAATCAACCAGGCATGACGGTGCACGCCTGTTATCA CAGCTACTTGGGAAGCAGCGGCTGGAGAATTGCTTGAACCTG GGAAGTCGAGGCTGCAAGTGAGCTGAGATCATGCCATGGCAG TCTAGCCTGGGCAACAGAGCGAGACTCTGTTTCA (SEQ ID NO:39) |
| Homo sapiens N-acylethanolamine acid amidase (NAAA) | |
| 3UTR-NO36 | AGTGTCCAGACCATTGTCTTCCAACCCCAGCTGGCCTCTAGAA CACCCACTGGCCAGTCCTAGAGCTCCTGTCCCTACCCACTCTT TGCTACAATAAATGCTGAATGAATCC (SEQ ID NO:40) |
| Homo sapiens apolipoprotein A2 (APOA2) | |
| 3UTR-NO37 | CATCACATTTAAAAGCATCTCAGCCTACCATGAGAATAAGAGAA AGAAAATGAAGATCAACAGCTTATTCATCTGTTTTTCTTTTTCGT TGGTGTAAAGCCAACACCCTGTCTAAAAAACATAAATTTCTTTAA TCATTTTGCCTCTTTTCTCTGTGCTTCAATTAATAAAAAATGGAA AGAATCTAATAGAGTGGTACAGCACTGTTATTTTTCAAAGATGTG TTGCTATCCTGAAAATTCTGTAGGTTCTGTGGAAGTTCCAGTGT TCTCTCTTATTCCACTTCGGTAGAGGATTTCTAGTTTCTTGTGG GCTAATTAAATAAATCATTAATACTCTTCTAA (SEQ ID NO:41) |
| Homo sapiens albumin (ALB) | |
| 3UTR-NO38 | TGCTCTGTGGGAGGGACTTTGCTGATTTCCCCTCTTCCCTTCA ACATGAAAATATATACCCCCCCATGCAGTCTAAAATGCTTCAGTA CTTGTGAAACACAGCTGTTCTTCTGTTCTGCAGACACGCCTTC CCCTCAGCCACACCCAGGCACTTAAGCACAAGCAGAGTGCAC AGCTGTCCACTGGGCCATTGTGGTGTGAGCTTCAGATGGTGAA GCATTCTCCCCAGTGTATGTCTTGTATCCGATATCTAACGCTTTA AATGGCTACTTTGGTTTCTGTCTGTAAGTTAAGACCTTGGATGT GGTTTAATTGTTTGTCCTCAAAAGGAATAAAACTTTTCTGCTGAT AAGAT (SEQ ID NO:42) |
| Homo sapiens ubiquitin C-terminal hydrolase L1 (UCHL1) | |
| 3UTR-NO39 | CTTCTCTGATACCAGCTGGAGGTTGAGCTCTTCTTAAAGTATTC TTCTCTTCCTTTTATCAGTAGGTGCCCAGAATAAGTTATTGCAGT TTATCATTCAAGTGTAAAATATTTTGAATCAATAATATATTTTCTGT TTTCTTTTGGTAAAGACTGGCTTTTATTAATGCACTTTCTATCCTC TGTAAACTTTTTGTGCTGAATGTTGGGACTGCTAAATAAAATTTG TTGCAT (SEQ ID NO:43) |
| Homo sapiens tumor susceptibility 101 (TSG101) | |
| 3UTR-NO41 | AGTAAGCAAGCACCTTAAGTAGTGTGTTAATGATTGAAACTGTA ATTGCCTTCGGGTTGTGCTTTAGTCTGTAAAATTCTAAAGGAGA GCTGCTAAATTGTTTTCTTAATAAACATTTTTCTCATTTGTA (SEQ ID NO:44) |
| Homo sapiens NEDD8 activating enzyme E1 subunit 1 (NAE1) | |
| 3UTR-NO46 | AAATGGAAGCCACAAGCAGCCCAGCCCTCCTTAATCAACTTCA AGGAGCACCTTCATTAGTACAGCTTGCATATTTAACATTTTGTAT TTCAATAAAAGTGAAGACAAACGA (SEQ ID NO:45) |
| Homo sapiens caspase 4 (CASP4) | |
| 3UTR-NO47 | CCTGCTGCCTGCCCCCAGCCCCCTTCCCGGACCCCCTGTACA GCGTCCCCACCTATTTCAAATCTTATTTAACACCCCACACCCAC CCCTCAGTTGGGACAAATAAAGGATTCTCATGGGAAGGGGAGG ACCCCTCCTTCCCAACTT (SEQ ID NO:46) |
| Homo sapiens nuclear factor kappa B subunit 2 (NFKB2) | |
| 3UTR-NO48 | CCTGTGCCCGGGCATGGCCTGGCAGCTCTCCAGCAGGGCAGA GTGTTTGCCCACCAGCTGCTAGCCCTAGGAAGGCCAGGAGCC CAGTAGCCATGTGGCCAGTCTACCATG (SEQ ID NO:47) |
| Homo sapiens gasdermin D (GSDMD) | |
| 3UTR-NO49 | TTGGGCGACTTCTCTCCCCCGTCCCCGTGGACACGCCCCACC CGCTCGGTCCTGGACACACTGCCCCCCTCTCTTGCCTCCACC CCTCTGCGGACTCTGCAGCTCCGCGGCCCCGGCGCAGGGAG AGGGAGGGCACGGGCGCGGGCCGGGCCTCAAGG (SEQ ID NO:48) |
| Homo sapiens F-box and leucine rich repeat protein 8 (FBXL8) | |
| 3UTR-NO50 | CGGGTGGCATCCCTGTGACCCCTCCCCAGTGCCTCTCCTGGC CCTGGAAGTTGCCACTCCAGTGCCCACCAGCCTTGTCCTAATA AAATTAAGTTGCATC (SEQ ID NO:49) |
| Homo sapiens growth hormone 1 (GH1) | |

Accordingly, in some embodiments, the 3'-UTR comprises at least one of the following polynucleotides: an RNA encoded by a polynucleotide having a sequence as set forth in at least one of SEQ ID NOs: 22-48; a fragment of the RNA encoded by a polynucleotide having a sequence as set forth in at least one of SEQ ID NOs: 22-48; a variant of the RNA encoded by a polynucleotide having a sequence as set forth in at least one of SEQ ID NOs: 22-48; and a variant of the fragment of the RNA encoded by a polynucleotide having a sequence as set forth in at least one of SEQ ID NOs: 22-48. In some embodiments, the fragment, the variant, or the variant of the fragment of the RNA encoded by a polynucleotide having a sequence as set forth in at least one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-48. In some embodiments, the fragment, the variant, and the variant of the fragment of the RNA encoded by a polynucleotide having a sequence as set forth in at least one of SEQ ID NOs: 22-48 have insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In a preferred embodiment, the gene is selected from at least one of genes MPND, FBXW10, FBXW12, and PGLYRP1. In some embodiments, the 3'-UTR comprises at least one of the following polynucleotides: an RNA encoded by a polynucleotide having a sequence as set forth in SEQ ID NO: 24, 22, 23, or 25; a fragment of the RNA encoded by a polynucleotide having a sequence as set forth in SEQ ID NO: 24, 22, 23, or 25; a variant of the RNA encoded by a polynucleotide having a sequence as set forth in SEQ ID NO: 24, 22, 23, or 25; and a variant of the fragment of the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

The 3'-UTR sequence of the present disclosure may comprise two or more tandem 3'-UTRs of the above genes, fragments of 3'-UTRs of the above genes, variants of 3'-UTRs of the above genes, or variants of fragments of 3'-UTRs of the above genes.

In some embodiments, the 3'-UTR comprises at least one of the following polynucleotides: (a) 3'-UTRs derived from at least two genes of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (b) fragments of the 3'-UTRs of at least two genes in (a); (c) variants of the 3'-UTRs of at least two genes in (a); and (d) variants of the fragments in (b). In some embodiments, the 3'-UTR comprises at least one of the following polynucleotides: (e) 3'-UTRs derived from at least two genes of genes MPND, FBXW10, FBXW12, and PGLYRP1; (f) fragments of the 3'-UTRs in (e); (g) variants of the 3'-UTRs in (e); and (h) variants of the fragments in (f).

In some embodiments, the 3'-UTR comprises at least one of the following polynucleotides: RNAs encoded by at least two polynucleotides having a sequence as set forth in SEQ ID NOs: 22-48; fragments of RNAs encoded by at least two polynucleotides having a sequence as set forth in SEQ ID NOs: 22-48; variants of RNAs encoded by at least two polynucleotides having a sequence as set forth in SEQ ID NOs: 22-48; and variants of fragments of RNAs encoded by at least two polynucleotides having a sequence as set forth in SEQ ID NOs: 22-48. In some embodiments, the fragment, the variant, or the variant of the fragment of the RNA encoded by a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-48. In some embodiments, the fragment, the variant, or the variant of the fragment of the RNA encoded by a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In some embodiments, the 3'-UTR comprises at least one of the following polynucleotides: RNAs encoded by at least two polynucleotides having a sequence as set forth in SEQ ID NOs: 24, 22, 23, and 25; fragments of RNAs encoded by at least two polynucleotides having a sequence as set forth in SEQ ID NOs: 24, 22, 23, and 25; variants of RNAs encoded by at least two polynucleotides having a sequence as set forth in SEQ ID NOs: 24, 22, 23, and 25; and variants of fragments of RNAs encoded by at least two polynucleotides having a sequence as set forth in SEQ ID NOs: 24, 22, 23, and 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NOs: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NOs: 24, 22, 23, or 25.

In some embodiments, the 3'-UTR comprises at least one of the following polynucleotides: a): at least two copies of a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; b) at least two copies of a fragment of the 3'-UTR of at least one gene from the genes in a); c) at least two copies of a variant of the 3'-UTR of at least one gene from the genes in a); and d) at least two copies of a variant of the fragment of the 3'-UTR of at least one gene from the genes in a). In some embodiments, the 3'-UTR comprises at least one of the following polynucleotides: e) at least two copies of a 3'-UTR derived from at least one gene of genes MPND, FBXW10, FBXW12, and PGLYRP1; f) at least two copies of a fragment of the 3'-UTR of at least one gene from the genes in e); g) at least two copies of a variant of the 3'-UTR of at least one gene from the genes in e); and h) at least two copies of a variant of the fragment in f). Preferably, at least two copies are two copies, three copies, four copies, five copies, six copies, seven copies, eight copies, or nine copies.

In some embodiments, the 3'-UTR comprises at least one of the following polynucleotides: at least two copies of the RNA encoded by a polynucleotide having a sequence as set forth in one of SEQ ID NOs: 22-48; at least two copies of a fragment of the RNA encoded by a polynucleotide having a sequence as set forth in one of SEQ ID NOs: 22-48; at least two copies of a variant of the RNA encoded by a polynucleotide having a sequence as set forth in one of SEQ ID NOs: 22-48; and at least two copies of variant of the fragment of the RNA encoded by a polynucleotide having a sequence as set forth in one of SEQ ID NOs: 22-48. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-48. In some embodiments, the fragment, the variant, or the variant of the fragment of the RNA encoded by a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In some embodiments, the 3'-UTR comprises at least one of the following polynucleotides: at least two copies of an RNA encoded by a polynucleotide having a sequence as set forth in one of SEQ ID NOs: 24, 22, 23, and 25; at least two copies of a fragment of the RNA encoded by a polynucleotide having a sequence as set forth in one of SEQ ID NOs: 24, 22, 23, and 25; at least two copies of a variant of the RNA encoded by a polynucleotide having a sequence as set forth in one of SEQ ID NOs: 24, 22, 23, and 25; and at least two copies of variant of the fragment of the RNA encoded by a polynucleotide having a sequence as set forth in one of SEQ ID NOs: 24, 22, 23, and 25. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 24, 22, 23, and 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in one of SEQ ID NOs: 24, 22, 23, and 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

### IV. Recombinant RNA molecule comprising 5' and/or 3'-UTRs of the present disclosure

Provided is a recombinant RNA molecule comprising the 5' and/or 3'-UTRs improving the translation of the coding sequence identified in the present disclosure.

In some embodiments, provided is a recombinant RNA molecule, comprising: a first nucleotide sequence encoding a polypeptide and/or protein of interest; and a second nucleotide sequence comprising a 5'-untranslated region (5'-UTR); wherein the 5'-UTR comprises at least one polynucleotide selected from: (a) a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) a fragment of the 5'-UTR in (a); (c) a variant of the 5'-UTR in (a); and (d) a variant of the fragment in (b); and wherein the first nucleotide sequence and the second nucleotide sequence are not naturally occurring in the same RNA molecule.

In some embodiments, the gene is a human gene. In some embodiments, the first nucleotide sequence encodes at least one polypeptide of interest, for example, one, two, three, four, five, six, seven, eight, nine, or ten polypeptides of interest. In some embodiments, the first nucleotide sequence encodes at least one protein of interest, for example, one, two, three, four, five, six, seven, eight, nine, or ten proteins of interest. In some embodiments, the first nucleotide sequence encodes at least one polypeptide of interest and at least one protein of interest, for example, one, two, three, four, five, six, seven, eight, nine, or ten polypeptides of interest, and one, two, three, four, five, six, seven, eight, nine, or ten proteins of interest.

In some embodiments, the second nucleotide sequence comprises at least one of the following polynucleotides: an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21. In some embodiments, the fragment, the variant, or the variant of the fragment of the RNA encoded by a polynucleotide having a sequence as set forth in at least one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in at least one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In a preferred embodiment, the gene is selected from at least one of PPIA, HPX, FTCD, CDK5RAP3, HSPA8, HBA1, and HBB. In some embodiments, the second nucleotide sequence comprises at least one of the following polynucleotides:1) an RNA encoded by a polynucleotide having a sequence as set forth in SEQ ID NO: 9, 7, 18, 12, 8, 1, or 6; 2) a fragment of the RNA in 1); 3) a variant of the RNA in 1); and 4) a variant of the fragment in 2). In some embodiments, the fragment, the variant, or the variant of the fragment of the RNA encoded by a polynucleotide having a sequence as set forth in SEQ ID NO: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

The second nucleotide sequence may comprise two or more tandem 5'-UTRs of the above genes, fragments of 5'-UTRs of the above genes, variants of 5'-UTRs of the above genes, or variants of fragments of 5'-UTRs of the above genes.

In some embodiments, the second nucleotide sequence comprises at least one of the following polynucleotides: (a) 5'-UTRs derived from at least two genes of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) fragments of the 5'-UTRs of at least two genes in (a); (c) variants of the 5'-UTRs of at least two genes in (a); and (d) variants of the fragments in (b). In some embodiments, the second nucleotide sequence comprises at least one of the following polynucleotides: (e) 5'-UTRs derived from at least two genes of genes PPIA, HPX, FTCD, CDK5RAP3, HSPA8, HBA1, and HBB; (f) fragments of the 5'-UTRs in (e); (g) variants of the 5'-UTRs in (e); and (h) variants of the fragments in (f).

In some embodiments, the second nucleotide sequence comprises at least one of the following polynucleotides: RNAs encoded by at least two polynucleotides having a sequence as set forth in SEQ ID NOs: 1-21; fragments of RNAs encoded by at least two polynucleotides having a sequence as set forth in SEQ ID NOs: 1-21; variants of RNAs encoded by at least two polynucleotides having a sequence as set forth in SEQ ID NOs: 1-21; and variants of fragments of RNAs encoded by at least two polynucleotides having a sequence as set forth in SEQ ID NOs: 1-21. In some embodiments, the second nucleotide sequence comprises RNAs encoded by at least two polynucleotides having a sequence as set forth in SEQ ID NOs: 1-21; fragments of RNAs encoded by at least two polynucleotides having a sequence as set forth in SEQ ID NOs: 1-21; variants of RNAs encoded by at least two polynucleotides having a sequence as set forth in SEQ ID NOs: 1-21; or variants of fragments of RNAs encoded by at least two polynucleotides having a sequence as set forth in SEQ ID NOs: 1-21. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the second nucleotide sequence comprises at least one of the following polynucleotides: RNAs encoded by at least two polynucleotides having a sequence as set forth in SEQ ID NOs: 9, 7, 18, 12, 8, 1, and 6; fragments of RNAs encoded by at least two polynucleotides having a sequence as set forth in SEQ ID NOs: 9, 7, 18, 12, 8, 1, and 6; variants of RNAs encoded by at least two polynucleotides having a sequence as set forth in SEQ ID NOs: 9, 7, 18, 12, 8, 1, and 6; and variants of fragments of RNAs encoded by at least two polynucleotides having a sequence as set forth in SEQ ID NOs: 9, 7, 18, 12, 8, 1, and 6. In some embodiments, the fragment, the variant, or the variant of the fragment of the RNA encoded by a polynucleotide having a sequence as set forth in SEQ ID NOs: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NOs: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in EQ ID NO: 9, 7, 18, 12, 8, 1, or 6.

In some embodiments, the second nucleotide sequence comprises at least one of the following polynucleotides: a): at least two copies of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; b) at least two copies of a fragment of the 5'-UTR of at least one gene from the genes in a); c) at least two copies of a variant of the 5'-UTR of at least one gene from the genes in a); and d) at least two copies of a variant of the fragment of the 5'-UTR of at least one gene from the genes in a). In some embodiments, the second nucleotide sequence comprises at least two copies of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7. In some embodiments, the 5'-UTR sequence comprises at least one of the following polynucleotides: a): at least two copies of a 5'-UTR derived from at least one gene of genes PPIA, HPX, FTCD, CDK5RAP3, HSPA8, HBA1, and HBB; b) at least two copies of a fragment of the 5'-UTR of at least one gene from the genes in a); c) at least two copies of a variant of the 5'-UTR of at least one gene from the genes in a); and d) at least two copies of a variant of the fragment of the 5'-UTR of at least one gene from the genes in a). In some embodiments, the 5'-UTR sequence comprises at least two copies of a 5'-UTR derived from at least one gene of genes PPIA, HPX, FTCD, CDK5RAP3, HSPA8, HBA1, and HBB. Preferably, at least two copies are two copies, three copies, four copies, five copies, six copies, seven copies, eight copies, or nine copies.

In some embodiments, the second nucleotide sequence comprises at least one of the following polynucleotides: at least two copies of the RNA encoded by a polynucleotide having a sequence as set forth in one of SEQ ID NOs: 1-21; at least two copies of a fragment of the RNA encoded by a polynucleotide having a sequence as set forth in one of SEQ ID NOs: 1-21; at least two copies of a variant of the RNA encoded by a polynucleotide having a sequence as set forth in one of SEQ ID NOs: 1-21; and at least two copies of variant of the fragment of the RNA encoded by a polynucleotide having a sequence as set forth in one of SEQ ID NOs: 1-21. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the second nucleotide sequence comprises at least one of the following polynucleotides: at least two copies of the RNA encoded by a polynucleotide having a sequence as set forth in one of SEQ ID NOs: 9, 7, 18, 12, 8, 1, and 6; at least two copies of a fragment of the RNA encoded by a polynucleotide having a sequence as set forth in one of SEQ ID NOs: 9, 7, 18, 12, 8, 1, and 6; at least two copies of a variant of the RNA encoded by a polynucleotide having a sequence as set forth in one of SEQ ID NOs: 9, 7, 18, 12, 8, 1, and 6; and at least two copies of variant of the fragment of the RNA encoded by a polynucleotide having a sequence as set forth in one of SEQ ID NOs: 9, 7, 18, 12, 8, 1, and 6. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 9, 7, 18, 12, 8, 1, and 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in one of SEQ ID NOs: 9, 7, 18, 12, 8, 1, and 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

In some embodiments, the recombinant RNA molecule is an mRNA molecule. In some embodiments, the recombinant RNA molecule further comprises at least one of a promoter, a 5' cap structure, a 3'-UTR, and a poly(A) tail. In some embodiments, the recombinant RNA molecule further comprises at least one of a 5' cap structure, a 3'-UTR, and a poly(A) tail.

In some embodiments, the 5'-cap structure comprises but not limited to at least one of m⁷GpppG, m₂^{7,3'-O}GpppG, m⁷Gppp(5')N1, and m⁷Gppp(m^{2'-O})N1. "m7G" represents 7-methylguanosine cap nucleoside; "ppp" represents a triphosphate linkage between the 5' carbon of the cap nucleoside and the first nucleotide of the primary RNA transcript; "N1" represents the most 5' nucleotide; "G" represents guanosine nucleoside; "m⁷" represents a methyl group at the 7-position of guanine; and "m^{2'-O}" represents a methyl group at the 2'-O position of the nucleotide.

In some embodiments, the 3'-UTR comprises at least one of: i) a polynucleotide of a 3'-UTR derived from at least one gene of albumin gene, α-globin gene, β-globin gene, tyrosine hydroxylase gene, lipoxygenase gene, and collagen α gene or a variant thereof; ii) a variant of the 3'-UTR in i); iii) a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1, a fragment, a variant, or a variant of a fragment thereof; preferably, at least one of an RNA encoded by a polynucleotide having a sequence as set forth in SEQ ID NOs: 22-49, a fragment of the RNA encoded by a polynucleotide having a sequence as set forth in SEQ ID NOs: 22-49, a variant of the RNA encoded by a polynucleotide having a sequence as set forth in SEQ ID NOs: 22-49, and a variant of the fragment of the RNA encoded by a polynucleotide having a sequence as set forth in SEQ ID NOs: 22-49. In some embodiments, the fragment, the variant, or the variant of the fragment of the RNA encoded by a polynucleotide having a sequence as set forth in SEQ ID NOs: 22-49 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NOs: 22-49; iv) at least two copies of one of the polynucleotides in i), ii), or iii); or v) at least two polynucleotides selected from the group consisting of polynucleotides in i)-iii). Preferably, at least two copies are two copies, three copies, four copies, five copies, six copies, seven copies, eight copies, or nine copies.

In some embodiments, the nucleotides of the poly(A) tail comprise at least 20, at least 40, at least 80, at least 100, or at least 120 A nucleotides; the nucleotides of the poly(A) tail comprise at least 20, at least 40, at least 80, at least 100, or at least 120 consecutive A nucleotides. In some embodiments, the poly(A) tail comprises one or more nucleotides other than A nucleotide. In some embodiments, the poly(A) tail comprises two or more consecutive nucleotides other than A nucleotide, wherein the first and last nucleotides of the sequence having two or more consecutive nucleotides are nucleotides other than A nucleotide. Preferably, the poly(A) tail has a segmented configuration, that is, m consecutive A nucleotides are connected to n consecutive A nucleotides via a linker sequence composed of p nucleotides other than A nucleotide, wherein m, n, and p are positive integers. Preferably, m is 30, n is 70, and p is 10.

In some embodiments, the recombinant RNA molecule comprises a first nucleotide sequence encoding a polypeptide and/or protein of interest; and a second nucleotide sequence comprising a 3'-UTR; wherein the second nucleotide sequence comprises at least one of the following polynucleotides: (a) a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (b) a fragment of the 3'-UTR in (a); (c) a variant of the 3'-UTR in (a); and (d) a variant of the fragment in (b); and wherein the first nucleotide sequence and the second nucleotide sequence are not naturally occurring in the same RNA molecule.

In some embodiments, the gene is a human gene.

In some embodiments, the first nucleotide sequence encodes at least one polypeptide of interest, for example, one, two, three, four, five, six, seven, eight, nine, or ten polypeptides of interest. In some embodiments, the first nucleotide sequence encodes at least one protein of interest, for example, one, two, three, four, five, six, seven, eight, nine, or ten proteins of interest. In some embodiments, the first nucleotide sequence encodes at least one polypeptide of interest and at least one protein of interest, for example, one, two, three, four, five, six, seven, eight, nine, or ten polypeptides of interest, and one, two, three, four, five, six, seven, eight, nine, or ten proteins of interest.

In some embodiments, the second nucleotide sequence comprises at least one of the following polynucleotides: an RNA encoded by a polynucleotide having a sequence as set forth in at least one of SEQ ID NOs: 22-48; a fragment of the RNA encoded by a polynucleotide having a sequence as set forth in at least one of SEQ ID NOs: 22-48; a variant of the RNA encoded by a polynucleotide having a sequence as set forth in at least one of SEQ ID NOs: 22-48; and a variant of the fragment of the RNA encoded by a polynucleotide having a sequence as set forth in at least one of SEQ ID NOs: 22-48. In some embodiments, the fragment, the variant, or the variant of the fragment of the RNA encoded by a polynucleotide having a sequence as set forth in at least one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in of SEQ ID NOs: 22-48. In some embodiments, the fragment, the variant or the variant of the fragment of the RNA encoded by a polynucleotide having a sequence as set forth in at least one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In a preferred embodiment, the gene is selected from at least one of MPND, FBXW10, FBXW12, and PGLYRP1. In some embodiments, the second nucleotide sequence comprises at least one of the following polynucleotides: an RNA encoded by a polynucleotide having a sequence as set forth in SEQ ID NO: 24, 22, 23, or 25; a fragment of the RNA encoded by a polynucleotide having a sequence as set forth in SEQ ID NO: 24, 22, 23, or 25; a variant of the RNA encoded by a polynucleotide having a sequence as set forth in SEQ ID NO: 24, 22, 23, or 25; and a variant of the fragment of the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

The 3'-UTR in the second nucleotide sequence in the recombinant RNA molecule of the present disclosure may comprise two or more tandem 3'-UTRs of the above genes, fragments of 3'-UTRs of the above genes, variants of 3'-UTRs of the above genes, or variants of fragments of 3'-UTRs of the above genes.

In some embodiments, the second nucleotide sequence comprises at least one of the following polynucleotides: (a) 3'-UTRs derived from at least two genes of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (b) fragments of the 3'-UTRs of at least two genes in (a); (c) variants of the 3'-UTRs of at least two genes in (a); and (d) variants of the fragments in (b). In some embodiments, the 3'-UTR comprises at least one of the following polynucleotides: (e) 3'-UTRs derived from at least two genes of genes MPND, FBXW10, FBXW12, and PGLYRP1; (f) fragments of the 3'-UTRs in (e); (g) variants of the 3'-UTRs in (e); and (h) variants of the fragments in (f).

In some embodiments, the second nucleotide sequence comprises at least one of the following polynucleotides: RNAs encoded by at least two polynucleotides having a sequence as set forth in SEQ ID NOs: 22-48; fragments of RNAs encoded by at least two polynucleotides having a sequence as set forth in SEQ ID NOs: 22-48; variants of RNAs encoded by at least two polynucleotides having a sequence as set forth in SEQ ID NOs: 22-48; and variants of fragments of RNAs encoded by at least two polynucleotides having a sequence as set forth in SEQ ID NOs: 22-48. In some embodiments, the fragment, the variant, or the variant of the fragment of the RNA encoded by a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-48. In some embodiments, the fragment, the variant, or the variant of the fragment of the RNA encoded by a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In some embodiments, the second nucleotide sequence comprises at least one of the following polynucleotides: RNAs encoded by at least two polynucleotides having a sequence as set forth in SEQ ID NOs: 24, 22, 23, and 25; fragments of RNAs encoded by at least two polynucleotides having a sequence as set forth in SEQ ID NOs: 24, 22, 23, and 25; variants of RNAs encoded by at least two polynucleotides having a sequence as set forth in SEQ ID NOs: 24, 22, 23, and 25; and variants of fragments of RNAs encoded by at least two polynucleotides having a sequence as set forth in SEQ ID NOs: 24, 22, 23, and 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded encoded by a polynucleotide having a sequence as set forth in SEQ ID NOs: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NOs: 24, 22, 23, or 25.

In some embodiments, the second nucleotide sequence comprises at least one of the following polynucleotides: a): at least two copies of a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; b) at least two copies of a fragment of the 3'-UTR of at least one gene from the genes in a); c) at least two copies of a variant of the 3'-UTR of at least one gene from the genes in a); and d) at least two copies of a variant of the fragment of the 3'-UTR of at least one gene from the genes in a). In some embodiments, the second nucleotide sequence comprises nucleotides of at least two copies of a 3'-UTR derived from at least one gene of genes MPND, FBXW10, FBXW12, and PGLYRP1. Preferably, at least two copies are two copies, three copies, four copies, five copies, six copies, seven copies, eight copies, or nine copies. In some embodiments, the second nucleotide sequence comprises at least one of the following polynucleotides: at least two copies of the RNA encoded by a polynucleotide having a sequence as set forth in one of SEQ ID NOs: 22-48; at least two copies of a fragment of the RNA encoded by a polynucleotide having a sequence as set forth in one of SEQ ID NOs: 22-48; at least two copies of a variant of the RNA encoded by a polynucleotide having a sequence as set forth in one of SEQ ID NOs: 22-48; and at least two copies of variant of the fragment of the RNA encoded by a polynucleotide having a sequence as set forth in one of SEQ ID NOs: 22-48. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-48. In some embodiments, the fragment, the variant, or the variant of the fragment of the RNA encoded by a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In some embodiments, the second nucleotide sequence comprises at least one of the following polynucleotides: at least two copies of the RNA encoded by a polynucleotide having a sequence as set forth in one of SEQ ID NOs: 24, 22, 23, and 25; at least two copies of a fragment of the RNA encoded by a polynucleotide having a sequence as set forth in one of SEQ ID NOs: 24, 22, 23, and 25; at least two copies of a variant of the RNA encoded by a polynucleotide having a sequence as set forth in one of SEQ ID NOs: 24, 22, 23, and 25; and at least two copies of variant of the fragment of the RNA encoded by a polynucleotide having a sequence as set forth in one of SEQ ID NOs: 24, 22, 23, and 25. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 24, 22, 23, and 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in one of SEQ ID NOs: 24, 22, 23, and 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

In some embodiments, the recombinant RNA molecule is an mRNA molecule. In some embodiments, the recombinant RNA molecule further comprises at least one of a promoter, a 5' cap structure, a 3'-UTR, and a poly(A) tail.

In some embodiments, the recombinant RNA molecule further comprises at least one of a 5' cap structure, a 3'-UTR, and a poly(A) tail.

In some embodiments, the 5'-cap structure comprises but not limited to at least one of m⁷GpppG, m₂^{7,3'-O}GpppG, m⁷Gppp(5')N1, and m⁷Gppp(m^{2'-O})N1. "m7G" represents 7-methylguanosine cap nucleoside; "ppp" represents a triphosphate linkage between the 5' carbon of the cap nucleoside and the first nucleotide of the primary RNA transcript; "N1" represents the most 5' nucleotide; "G" represents guanosine nucleoside; "m⁷" represents a methyl group at the 7-position of guanine; and "m^{2'-O}" represents a methyl group at the 2'-O position of the nucleotide.

In some embodiments, the 5'-UTR comprises: i) at least one of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; a fragment, a variant or a variant of a fragment thereof; preferably, the RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21; the variant of the RNA encoded by a polynucleotide having a sequence as set forth in at least one of SEQ ID NOs: 1-21, the fragment of the RNA encoded by a polynucleotide having a sequence as set forth in at least one of SEQ ID NOs: 1-21, and the variant of the fragment of the RNA encoded by a polynucleotide having a sequence as set forth in at least one of SEQ ID NOs: 1-21 have at least 40%, 50%, 60%, 70%, 80%, 85%, 90%, 91%, 92%, 93%, 94%, 95%, 96%, 97%, 98%, or 99% homology to the RNA encoded by a polynucleotide having a sequence as set forth in at least one of SEQ ID NOs: 1-21; ii) at least two copies of one of the polynucleotides in i); or iii) at least two polynucleotides in i). Preferably, at least two copies are two copies, three copies, four copies, five copies, six copies, seven copies, eight copies, or nine copies.

In some embodiments, the nucleotides of the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides. In some embodiments, the poly(A) tail comprises one or more nucleotides other than A nucleotide. In some embodiments, the poly(A) tail comprises two or more consecutive nucleotides other than A nucleotide, wherein the first and last nucleotides of the sequence having two or more consecutive nucleotides are nucleotides other than A nucleotide. Preferably, the poly(A) tail has a segmented configuration, that is, m consecutive A nucleotides are connected to n consecutive A nucleotides via a linker sequence composed of p nucleotides other than A nucleotide, wherein m, n, and p are positive integers. Preferably, m is 30, n is 70, and p is 10.

In some embodiments, the DNA sequence corresponding to the poly(A) tail is as set forth in SEQ ID NO: 53.

In some embodiments, the recombinant RNA molecule has no more than 50,000 nt. Preferably, the recombinant RNA molecule has no more than 40,000 nt, 30,000 nt, 20,000 nt, 10,000 nt, 9,000 nt, 8,000 nt, or 6,000 nt. In some embodiments, the recombinant RNA molecule has 500 nt - 50,000 nt. In some embodiments, the recombinant RNA molecule has 1,000 nt - 40,000 nt, 1,000 nt - 30,000 nt, 1,500 nt - 10,000 nt, or 1,500 nt - 8,000 nt.

In some embodiments, the recombinant RNA molecule comprises a first nucleotide sequence encoding a polypeptide and/or protein of interest, a second nucleotide sequence comprising a 5'-UTR, and a third nucleotide sequence comprising a 3'-UTR, wherein the second nucleotide sequence comprises at least one of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7, a fragment, a variant, and a variant of a fragment thereof, wherein the third nucleotide sequence comprises at least one of a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1, a fragment, a variant, and a variant of a fragment thereof, and wherein the first nucleotide sequence and at least one of the second nucleotide sequence and the third nucleotide sequence are not naturally occurring in the same RNA molecule.

As compared to individual 5'-UTR or 3'-UTR derived from at least one of the aforementioned genes, a fragment, a variant, or a variant of a fragment thereof, the combination of a 5'-UTR derived from at least one of the aforementioned genes, a fragment, a variant, or a variant of a fragment thereof, with a 3'-UTR derived from at least one of the aforementioned genes, a fragment, a variant, or a variant of a fragment thereof, can further enhance the translation efficiency and/or stability of the mRNA molecule, increasing the expression level of the polypeptide and/or protein of interest, for example, by 1-fold, 2-fold, 3-fold, 4-fold, 5-fold, 6-fold, 7-fold, 8-fold, 9-fold, or 10-fold or more.

It is to be understood that "A, a fragment, variant, or variant of a fragment thereof" herein is an abbreviation for "A, a fragment of A, a variant of A, or a variant of a fragment of A". For example, "5'-UTR derived from at least one gene of genes PPIA, HPX, and FTCD, a fragment, a variant or a variant of a fragment thereof" means 5'-UTR derived from at least one gene of genes PPIA, HPX, and FTCD; a fragment of 5'-UTR derived from at least one gene of genes PPIA, HPX, and FTCD; a variant of 5'-UTR derived from at least one gene of genes PPIA, HPX, and FTCD; or a variant of a fragment of 5'-UTR derived from at least one gene of genes PPIA, HPX, and FTCD. "At least one of A, a fragment, variant, and variant of a fragment thereof" is an abbreviation for at least one of a group composed of "A, a fragment of A, a variant of A, and a variant of a fragment of A". For example, "at least one of 5'-UTR derived from at least one gene of genes PPIA, HPX, and FTCD, a fragment, a variant and a variant of a fragment thereof" means at least one of 5'-UTR derived from at least one gene of genes PPIA, HPX, and FTCD; a fragment of 5'-UTR derived from at least one gene of genes PPIA, HPX, and FTCD; a variant of 5'-UTR derived from at least one gene of genes PPIA, HPX, and FTCD; and a variant of a fragment of 5'-UTR derived from at least one gene of genes PPIA, HPX, and FTCD.

In some embodiments, the gene is a human gene.

In some embodiments, the first nucleotide sequence encodes at least one polypeptide of interest, for example, one, two, three, four, five, six, seven, eight, nine, or ten polypeptides of interest. In some embodiments, the first nucleotide sequence encodes at least one protein of interest, for example, one, two, three, four, five, six, seven, eight, nine, or ten proteins of interest. In some embodiments, the first nucleotide sequence encodes at least one polypeptide of interest and at least one protein of interest, for example, one, two, three, four, five, six, seven, eight, nine, or ten polypeptides of interest, and one, two, three, four, five, six, seven, eight, nine, or ten proteins of interest.

In some embodiments, the second nucleotide sequence comprises at least one of the following polynucleotides: the RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21. In some embodiments, the second nucleotide sequence comprises one of the following polynucleotides: the RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21. In some embodiments, the fragment, the variant, or the variant of the fragment of the RNA encoded by a polynucleotide having a sequence as set forth in at least one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in at least one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In a preferred embodiment, the gene is selected from at least one of PPIA, HPX, FTCD, CDK5RAP3, HSPA8, HBA1, and HBB. In some embodiments, the second nucleotide sequence comprises at least one of the following polynucleotides: 1) the RNA encoded by a polynucleotide having a sequence as set forth in SEQ ID NO: 9, 7, 18, 12, 8, 1, or 6; 2) a fragment of the RNA in 1); 3) a variant of the RNA in 1); and 4) a variant of the fragment in 2). In some embodiments, the second nucleotide sequence comprises one of the following polynucleotides: 1) the RNA encoded by a polynucleotide having a sequence as set forth in SEQ ID NO: 9, 7, 18, 12, 8, 1, or 6; 2) a fragment of the RNA in 1); 3) a variant of the RNA in 1); and 4) a variant of the fragment in 2). In some embodiments, the fragment, the variant, or the variant of the fragment of the RNA encoded by a polynucleotide having a sequence as set forth in SEQ ID NO: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

The 5'-UTR in the second nucleotide sequence in the recombinant RNA molecule of the present disclosure may comprise two or more tandem 5'-UTRs of the above genes, fragments of 5'-UTRs of the above genes, variants of 5'-UTRs of the above genes, or variants of fragments of 5'-UTRs of the above genes.

In some embodiments, the second nucleotide sequence comprises at least one of the following polynucleotides: (a) 5'-UTRs derived from at least two genes of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) fragments of the 5'-UTRs of at least two genes in (a); (c) variants of the 5'-UTRs of at least two genes in (a); and (d) variants of the fragments in (b).

In some embodiments, the second nucleotide sequence comprises one of the following polynucleotides: (a) 5'-UTRs derived from at least two genes of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) fragments of the 5'-UTRs of at least two genes in (a); (c) variants of the 5'-UTRs of at least two genes in (a); and (d) variants of the fragments in (b). In some embodiments, the second nucleotide sequence comprises at least one of the following polynucleotides: (e) 5'-UTRs derived from at least two genes of genes PPIA, HPX, FTCD, CDK5RAP3, HSPA8, HBA1, and HBB; (f) fragments of the 5'-UTRs in (e); (g) variants of the 5'-UTRs in (e); and (h) variants of the fragments in (f). In some embodiments, the second nucleotide sequence comprises one of the following polynucleotides: (e) 5'-UTRs derived from at least two genes of genes PPIA, HPX, FTCD, CDK5RAP3, HSPA8, HBA1, and HBB; (f) fragments of the 5'-UTRs in (e); (g) variants of the 5'-UTRs in (e); and (h) variants of the fragments in (f).

In some embodiments, the second nucleotide sequence comprises at least one of the following polynucleotides: RNAs encoded by at least two polynucleotides having a sequence as set forth in SEQ ID NOs: 1-21; fragments of RNAs encoded by at least two polynucleotides having a sequence as set forth in SEQ ID NOs: 1-21; variants of RNAs encoded by at least two polynucleotides having a sequence as set forth in SEQ ID NOs: 1-21; and variants of fragments of RNAs encoded by at least two polynucleotides having a sequence as set forth in SEQ ID NOs: 1-21. In some embodiments, the second nucleotide sequence comprises RNAs encoded by at least two polynucleotides having a sequence as set forth in SEQ ID NOs: 1-21; fragments of RNAs encoded by at least two polynucleotides having a sequence as set forth in SEQ ID NOs: 1-21; variants of RNAs encoded by at least two polynucleotides having a sequence as set forth in SEQ ID NOs: 1-21; or variants of fragments of RNAs encoded by at least two polynucleotides having a sequence as set forth in SEQ ID NOs: 1-21. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the second nucleotide sequence comprises at least one of the following polynucleotides: RNAs encoded by at least two polynucleotides having a sequence as set forth in SEQ ID NOs: 9, 7, 18, 12, 8, 1, and 6; fragments of RNAs encoded by at least two polynucleotides having a sequence as set forth in SEQ ID NOs: 9, 7, 18, 12, 8, 1, and 6; variants of RNAs encoded by at least two polynucleotides having a sequence as set forth in SEQ ID NOs: 9, 7, 18, 12, 8, 1, and 6; and variants of fragments of RNAs encoded by at least two polynucleotides having a sequence as set forth in SEQ ID NOs: 9, 7, 18, 12, 8, 1, and 6. In some embodiments, the fragment, the variant, or the variant of the fragment of the RNA encoded by a polynucleotide having a sequence as set forth in SEQ ID NOs: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NOs: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

In some embodiments, the second nucleotide sequence comprises at least one of the following polynucleotides: (a) at least two copies of 5'-UTRs derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) at least two copies of a fragment of the 5'-UTR of at least one gene in (a); (c) at least two copies of a variant of the 5'-UTR of at least one gene in (a); and (d) at least two copies of a variant of the fragment of the 5'-UTR of at least one gene in (a).

In some embodiments, the second nucleotide sequence comprises at least two copies of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7. In some embodiments, the 5'-UTR sequence comprises at least two copies of a 5'-UTR derived from at least one gene of genes PPIA, HPX, FTCD, CDK5RAP3, HSPA8, HBA1, and HBB; at least two copies of a fragment of the 5'-UTR of at least one gene from genes PPIA, HPX, FTCD, CDK5RAP3, HSPA8, HBA1, and HBB; at least two copies of a variant of the 5'-UTR of at least one gene from genes PPIA, HPX, FTCD, CDK5RAP3, HSPA8, HBA1, and HBB; or at least two copies of a variant of a fragment of the 5'-UTR of at least one gene from genes PPIA, HPX, FTCD, CDK5RAP3, HSPA8, HBA1, and HBB. In some embodiments, the 5'-UTR sequence comprises at least two copies of a 5'-UTR derived from one gene of genes PPIA, HPX, FTCD, CDK5RAP3, HSPA8, HBA1, and HBB; at least two copies of a fragment of the 5'-UTR of one gene from genes PPIA, HPX, FTCD, CDK5RAP3, HSPA8, HBA1, and HBB; at least two copies of a variant of the 5'-UTR of one gene from genes PPIA, HPX, FTCD, CDK5RAP3, HSPA8, HBA1, and HBB; or at least two copies of a variant of a fragment of the 5'-UTR of one gene from genes PPIA, HPX, FTCD, CDK5RAP3, HSPA8, HBA1, and HBB. Preferably, at least two copies are two copies, three copies, four copies, five copies, six copies, seven copies, eight copies, or nine copies.

In some embodiments, the second nucleotide sequence comprises at least one of the following polynucleotides: at least two copies of the RNA encoded by a polynucleotide having a sequence as set forth in one of SEQ ID NOs: 1-21; at least two copies of a fragment of the RNA encoded by a polynucleotide having a sequence as set forth in one of SEQ ID NOs: 1-21; at least two copies of a variant of the RNA encoded by a polynucleotide having a sequence as set forth in one of SEQ ID NOs: 1-21; and at least two copies of a variant of the fragment of the RNA encoded by a polynucleotide having a sequence as set forth in one of SEQ ID NOs: 1-21. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 1-21. In some embodiments, the fragment, the variant, or the variant of the fragment of the RNA encoded by a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In some embodiments, the second nucleotide sequence comprises at least one of the following polynucleotides: at least two copies of the RNA encoded by a polynucleotide having a sequence as set forth in one of SEQ ID NOs: 9, 7, 18, 12, 8, 1, and 6; at least two copies of a fragment of the RNA encoded by a polynucleotide having a sequence as set forth in one of SEQ ID NOs: 9, 7, 18, 12, 8, 1, and 6; at least two copies of a variant of the RNA encoded by a polynucleotide having a sequence as set forth in one of SEQ ID NOs: 9, 7, 18, 12, 8, 1, and 6; and at least two copies of a variant of the fragment of the RNA encoded by a polynucleotide having a sequence as set forth in one of SEQ ID NOs: 9, 7, 18, 12, 8, 1, and 6. Preferably, at least two copies are two copies, three copies, four copies, five copies, six copies, seven copies, eight copies, or nine copies. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 9, 7, 18, 12, 8, 1, and 6 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in one of SEQ ID NOs: 9, 7, 18, 12, 8, 1, and 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

In some embodiments, the third nucleotide sequence comprises at least one of an RNA encoded by a polynucleotide having a sequence as set forth in at least one of SEQ ID NOs: 22-49; a fragment of the RNA encoded by a polynucleotide having a sequence as set forth in at least one of SEQ ID NOs: 22-49; a variant of the RNA encoded by a polynucleotide having a sequence as set forth in at least one of SEQ ID NOs: 22-49; and a variant of the fragment of the RNA encoded by a polynucleotide having a sequence as set forth in at least one of SEQ ID NOs: 22-49. In some embodiments, the third nucleotide sequence comprises at least one of the RNA encoded by a polynucleotide having a sequence as set forth in at least one of SEQ ID NOs: 22-48; a fragment of the RNA encoded by a polynucleotide having a sequence as set forth in at least one of SEQ ID NOs: 22-48; a variant of the RNA encoded by a polynucleotide having a sequence as set forth in at least one of SEQ ID NOs: 22-48; and a variant of the fragment of the RNA encoded by a polynucleotide having a sequence as set forth in at least one of SEQ ID NOs: 22-48. In some embodiments, the fragment, the variant, or the variant of the fragment of the RNA encoded by a polynucleotide having a sequence as set forth in one of SEQ ID NOs: 22-49 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-49. In some embodiments, the fragment, the variant, or the variant of the fragment of the RNA encoded by a polynucleotide having a sequence as set forth in one of SEQ ID NOs: 22-49 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-49.

In a preferred embodiment, the gene is selected from at least one of MPND, FBXW10, FBXW12, and PGLYRP1. In some embodiment, the gene is selected from at least one of MPND, FBXW10, FBXW12, and PGLYRP1. In some embodiments, the third nucleotide sequence comprises at least one of the following polynucleotides: the RNA encoded by a polynucleotide having a sequence as set forth in SEQ ID NO: 24, 22, 23, or 25; a fragment of the RNA encoded by a polynucleotide having a sequence as set forth in SEQ ID NO: 24, 22, 23, or 25; a variant of the RNA encoded by a polynucleotide having a sequence as set forth in SEQ ID NO: 24, 22, 23, or 25; and a variant of the fragment of the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

The 3'-UTR in the third nucleotide sequence in the recombinant molecule of the present disclosure may comprise two or more tandem 3'-UTRs of the above genes, fragments of 3'-UTRs of the above genes, variants of 3'-UTRs of the above genes, or variants of fragments of 3'-UTRs of the above genes.

In some embodiments, the third nucleotide sequence comprises at least one of the following polynucleotides: (a) 3'-UTRs derived from at least two genes of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1; (b) fragments of the 3'-UTRs of at least two genes in (a); (c) variants of the 3'-UTRs of at least two genes in (a); and (d) variants of the fragments of the 3'-UTRs of at least two genes in (a). In some embodiments, the third nucleotide sequence comprises at least one of the following polynucleotides: (e) 3'-UTRs derived from at least two genes of genes MPND, FBXW10, FBXW12, and PGLYRP1; (f) fragments of the 3'-UTRs in (e); (g) variants of the 3'-UTRs in (e); and (h) variants of the fragments in (f).

In some embodiments, the third nucleotide sequence comprises at least one of the following polynucleotides: RNAs encoded by at least two polynucleotides having a sequence as set forth in SEQ ID NOs: 22-49; fragments of RNAs encoded by at least two polynucleotides having a sequence as set forth in SEQ ID NOs: 22-49; variants of RNAs encoded by at least two polynucleotides having a sequence as set forth in SEQ ID NOs: 22-49; and variants of fragments of RNAs encoded by at least two polynucleotides having a sequence as set forth in SEQ ID NOs: 22-49. In some embodiments, the third nucleotide sequence comprises at least one of the following polynucleotides: RNAs encoded by at least two polynucleotides having a sequence as set forth in SEQ ID NOs: 22-48; fragments of RNAs encoded by at least two polynucleotides having a sequence as set forth in SEQ ID NOs: 22-48; variants of RNAs encoded by at least two polynucleotides having a sequence as set forth in SEQ ID NOs: 22-48; and variants of fragments of RNAs encoded by at least two polynucleotides having a sequence as set forth in SEQ ID NOs: 22-48. In some embodiments, the fragment, the variant, or the variant of the fragment of the RNA encoded by a polynucleotide having a sequence as set forth in one of SEQ ID NOs: 22-49 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-49. In some embodiments, the fragment, the variant, or the variant of the fragment of the RNA encoded by a polynucleotide having a sequence as set forth in one of SEQ ID NOs: 22-49 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-49.

In some embodiments, the third nucleotide sequence comprises at least one of the following polynucleotides: RNAs encoded by at least two polynucleotides having a sequence as set forth in SEQ ID NOs: 24, 22, 23, and 25; fragments of RNAs encoded by at least two polynucleotides having a sequence as set forth in SEQ ID NOs: 24, 22, 23, and 25; variants of RNAs encoded by at least two polynucleotides having a sequence as set forth in SEQ ID NOs: 24, 22, 23, and 25; and variants of fragments of RNAs encoded by at least two polynucleotides having a sequence as set forth in SEQ ID NOs: 24, 22, 23, and 25. In some embodiments, the fragment, the variant, or the variant of the fragment of the RNA encoded by a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NOs: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NOs: 24, 22, 23, or 25.

In some embodiments, the third nucleotide sequence comprises at least one of the following polynucleotides: a): at least two copies of a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1; b) at least two copies of a fragment of the 3'-UTR of at least one gene from the genes in a); c) at least two copies of a variant of the 3'-UTR of at least one gene from the genes in a); and d) at least two copies of a variant of the fragment of the 3'-UTR of at least one gene from the genes in a). In some embodiments, the second nucleotide sequence comprises at least two copies of a 3'-UTR derived from at least one gene of genes MPND, FBXW10, FBXW12, and PGLYRP1. Preferably, at least two copies are two copies, three copies, four copies, five copies, six copies, seven copies, eight copies, or nine copies. In some embodiments, the third nucleotide sequence comprises at least one of the following polynucleotides: at least two copies of the RNA encoded by a polynucleotide having a sequence as set forth in one of SEQ ID NOs: 22-49; at least two copies of a fragment of the RNA encoded by a polynucleotide having a sequence as set forth in one of SEQ ID NOs: 22-49; at least two copies of a variant of the RNA encoded by a polynucleotide having a sequence as set forth in one of SEQ ID NOs: 22-49; and at least two copies of variant of the fragment of the RNA encoded by a polynucleotide having a sequence as set forth in one of SEQ ID NOs: 22-49. In some embodiments, the third nucleotide sequence comprises at least one of the following polynucleotides: at least two copies of the RNA encoded by a polynucleotide having a sequence as set forth in one of SEQ ID NOs: 22-49; at least two copies of a fragment of the RNA encoded by a polynucleotide having a sequence as set forth in one of SEQ ID NOs: 22-49; at least two copies of a variant of the RNA encoded by a polynucleotide having a sequence as set forth in one of SEQ ID NOs: 22-49; and at least two copies of variant of the fragment of the RNA encoded by a polynucleotide having a sequence as set forth in one of SEQ ID NOs: 22-49. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 22-49 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in one of SEQ ID NOs: 22-49. In some embodiments, the fragment, the variant, or the variant of the fragment of the RNA encoded by a polynucleotide having a sequence as set forth in one of SEQ ID NOs: 22-49 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-49.

In some embodiments, the third nucleotide sequence comprises at least one of the following polynucleotides: at least two copies of the RNA encoded by a polynucleotide having a sequence as set forth in one of SEQ ID NOs: 24, 22, 23, and 25; at least two copies of a fragment of the RNA encoded by a polynucleotide having a sequence as set forth in one of SEQ ID NOs: 24, 22, 23, and 25; at least two copies of a variant of the RNA encoded by a polynucleotide having a sequence as set forth in one of SEQ ID NOs: 24, 22, 23, and 25; and at least two copies of variant of the fragment of the RNA encoded by a polynucleotide having a sequence as set forth in one of SEQ ID NOs: 24, 22, 23, and 25. Preferably, at least two copies are two copies, three copies, four copies, five copies, six copies, seven copies, eight copies, or nine copies. In some embodiments, the fragment, the variant, or the variant of the fragment of the RNA encoded by a polynucleotide having a sequence as set forth in one of SEQ ID NOs: 24, 22, 23, and 25 has at least 40%, 50%, 60%, 70%, 80%, 85%, 90%, 91%, 92%, 93%, 94%, 95%, 96%, 97%, 98%, or 99% identity to the RNA encoded by a polynucleotide having a sequence as set forth in SEQ ID NO: 24, 22, 23, or 25. In some embodiments, the fragment, the variant, or the variant of the fragment of the RNA encoded by a polynucleotide having a sequence as set forth in one of SEQ ID NOs: 24, 22, 23, and 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

In some embodiments, the 5'-UTR has a nucleotide sequence as set forth in SEQ ID NO: 55.

In some embodiments, the recombinant RNA molecule comprises a first nucleotide sequence encoding a polypeptide and/or protein of interest, a 3'-UTR according to any of the above embodiments, and a 5'-UTR having a nucleotide sequence as set forth in SEQ ID NO: 55.

In some embodiments, the recombinant RNA molecule comprises a first nucleotide sequence encoding a polypeptide and/or protein of interest, a 3'-UTR having a sequence as set forth in one of SEQ ID NOs: 22-48, and a 5'-UTR having a nucleotide sequence as set forth in SEQ ID NO: 55.

In some embodiments, the recombinant RNA molecule comprises a first nucleotide sequence encoding a polypeptide and/or protein of interest; a 3'-UTR derived from at least one gene of genes FBXW10, FBXW12, MPND, and PGLYRP1; and a 5'-UTR having a nucleotide sequence as set forth in SEQ ID NO: 55.

In some embodiments, the recombinant RNA molecule comprises a first nucleotide sequence encoding a polypeptide and/or protein of interest; a 3'-UTR having a nucleotide sequence as set forth in one of SEQ ID NOs: 22-25; and a 5'-UTR having a nucleotide sequence as set forth in SEQ ID NO: 55.

In some embodiments, the 3'-UTR is a 3'-UTR derived from gene COP1 (CARD only protein).

In some embodiments, the 3'-UTR has a nucleotide sequence as set forth in SEQ ID NO: 56.

In some embodiments, the recombinant RNA molecule comprises a first nucleotide sequence encoding a polypeptide and/or protein of interest, a 5'-UTR according to any of the above embodiments, and a 3'-UTR having a nucleotide sequence as set forth in SEQ ID NO: 56.

In some embodiments, the recombinant RNA molecule comprises a first nucleotide sequence encoding a polypeptide and/or protein of interest, a 5'-UTR having a sequence as set forth in one of SEQ ID NOs: 1-21, and a 3'-UTR of gene COP1 (CARD only protein).

In some embodiments, the recombinant RNA molecule comprises a first nucleotide sequence encoding a polypeptide and/or protein of interest, a 5'-UTR having a sequence as set forth in one of SEQ ID NOs: 1-21, and a 3'-UTR having a sequence as set forth in SEQ ID NO: 56.

In some embodiments, the recombinant RNA molecule comprises a first nucleotide sequence encoding a polypeptide and/or protein of interest; a 5'-UTR derived from at least one gene of genes PPIA, HPX, FTCD, CDK5RAP3, HSPA8, HBA1, and HBB; and a 3'-UTR derived from gene COP1.

In some embodiments, the recombinant RNA molecule comprises a first nucleotide sequence encoding a polypeptide and/or protein of interest, a 5'-UTR having a sequence as set forth in one of SEQ ID NOs: 1, 6-9, 12, and 18, and a 3'-UTR having a sequence as set forth in SEQ ID NO: 56.

In some embodiments, the recombinant RNA molecule is an mRNA molecule. In some embodiments, the recombinant RNA molecule further comprises at least one of a 5' cap structure, and a poly(A) tail.

In some embodiments, the 5'-cap structure comprises but not limited to at least one of m⁷GpppG, m₂^{7,3'-O}GpppG, m⁷Gppp(5')N1, and m⁷Gppp(m^{2'-O})N1. "m7G" represents 7-methylguanosine cap nucleoside; "ppp" represents a triphosphate linkage between the 5' carbon of the cap nucleoside and the first nucleotide of the primary RNA transcript; "N1" represents the most 5' nucleotide; "G" represents guanosine nucleoside; "m⁷" represents a methyl group at the 7-position of guanine; and "m^{2'-O}" represents a methyl group at the 2'-O position of the nucleotide.

In some embodiments, the nucleotides of the poly(A) tail comprise at least 20, at least 40, at least 80, at least 100, or at least 120 A nucleotides. Preferably, the nucleotides of the poly(A) tail comprise at least 20, at least 40, at least 80, at least 100, or at least 120 consecutive A nucleotides. In some embodiments, the poly(A) tail comprises one or more nucleotides other than A nucleotide. In some embodiments, the poly(A) tail comprises two or more consecutive nucleotides other than A nucleotide, wherein the first and last nucleotides of the sequence having two or more consecutive nucleotides are nucleotides other than A nucleotide. Preferably, the poly(A) tail has a segmented configuration, that is, m consecutive A nucleotides are connected to n consecutive A nucleotides via a linker sequence composed of p nucleotides other than A nucleotide, wherein m, n, and p are positive integers. Preferably, m is 30, n is 70, and p is 10.

In some embodiments, the DNA sequence corresponding to the poly(A) tail is as set forth in SEQ ID NO: 53.

In some embodiments, the recombinant RNA molecule has no more than 50,000 nt. Preferably, the recombinant RNA molecule has no more than 40,000 nt, 30,000 nt, 20,000 nt, 10,000 nt, 9,000 nt, 8,000 nt, or 6,000 nt. In some embodiments, the recombinant RNA molecule has 500 nt - 50,000 nt. In some embodiments, the recombinant RNA molecule has 1,000 nt - 40,000 nt, 1,000 nt - 30,000 nt, 1,500 nt - 10,000 nt, or 1,500 nt - 8,000 nt.

In some embodiments, the recombinant RNA molecule according to any one of the above embodiments comprises a modified nucleotide. In some embodiments, the recombinant RNA molecule comprises at least one of a modified uridine, a modified cytidine, a modified adenosine, and a modified guanosine.

In some embodiments, the modified nucleotide is a modified uridine. In some embodiments, 0.1%-100% of the uridines in the recombinant RNA molecule are modified. Preferably, 80% - 100% of the uridines are modified. Preferably, 100% of the uridines are modified. Exemplary modified uridines comprise pseudouridine (ψ), N1-methylpseudouridine, pyridin-4-one ribonucleoside, 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho⁵U), 5-allylamino-uridine, 5-halo-uridine (e.g., 5-iodo-uridine or 5-bromo-uridine), 3-methyl-uridine (m³U), 5-methoxy-uridine (mo⁵U), uridine-5-oxyacetic acid (cmo⁵U), uridine-5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propargyl-uridine, 1-propargyl-pseudouridine, 5-taurinomethyl-uridine (τm⁵U), 1-taurinomethyl-pseudouridine, 5-taurinomethyl-2-thio-uridine (τm⁵s²U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s²U), α-thio-uridine, 2'-O-methyl-uridine (Um), 5,2'-O-dimethyl-uridine (m⁵Um), 2'-O-methyl-pseudouridine (Ψm), 2-thio-2'-O-methyl-uridine (s²Um), 5-methoxycarbonylmethyl-2'-O-methyl-uridine (mcm⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-arabino-uridine (2'-F-ara-uridine), 2'-F-uridine, 2'-OH-arabino-uridine, 5-(2-carbomethoxyvinyl)uridine, and 5-[3-(1-E-propenylamino)uridine.

In some embodiments, the modified nucleoside is a modified cytidine. In some embodiments, 0.1% - 100% of the cytidines in the recombinant RNA molecule are modified. Preferably, 80% - 100% of the cytidines are modified. More preferably, 100% of the cytidines are modified. Exemplary modified cytidines comprise, but not limited to 5-aza-cytidine, 6-aza-cytidine, pseudoisocytidine, 3-methyl-cytidine (m3C), N4-acetyl-cytidine (ac4C), 5-formyl-cytidine (f5C), N4-methyl-cytidine (m4C), 5-methyl-cytidine (m5C), 5-halo-cytidine (e.g., 5-iodo-cytidine), 5-hydroxymethyl-cytidine (hm5C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k2C), alpha-thio-cytidine, 2'-O-methyl-cytidine (Cm), 5,2'-O-dimethyl-cytidine (m5Cm), N4-acetyl-2'-O-methyl-cytidine (ac4Cm), N4,2'-O-dimethyl-cytidine (m4Cm), 5-formyl-2'-O-methyl-cytidine (f5Cm), N4,N4,2'-O-trimethyl-cytidine (m42Cm), 1-thio-cytidine, 2'-F-arabino-cytidine, 2'-F-cytidine, and 2'-OH-arabino-cytidine.

In some embodiments, the modified nucleoside is a modified adenosine. In some embodiments, 0.1% - 100% of the adenosines in the recombinant RNA molecule are modified. In preferred embodiments, 80% - 100% of the adenosines are modified. In more preferred embodiments, 100% of the adenosines are modified. Exemplary modified adenosines comprise, but not limited to 2-aminopurine, 2,6-diaminopurine, 2-amino-6-halopurine (e.g., 2-amino-6-chloropurine), 6-halopurine (e.g., 6-chloropurine), 2-amino-6-methylpurine, 8-azidoadenosine, 7-deazaadenine, 7-deaza-8-azaadenine, 7-deaza-2-aminopurine, 7-deaza-8-aza-2-aminopurine, 7-deaza-2,6-diaminopurine, 7-deaza-8-aza-2,6-diaminopurine, 1-methyladenosine (m1A), 2-methyladenine (m2A), N6-methyladenosine (m6A), 2-methylthio-N6-methyladenosine (ms2m6A), N6-isopentenyladenosine (i6A), 2-methylthio-N6-isopentenyladenosine (ms2i6A), N6-(cis-hydroxyisopentenyl)adenosine (io6A), 2-methylthio-N6-(cis-hydroxyisopentenyl)adenosine (ms2io6A), N6-glycinylcarbamoyladenosine (g6A), N6-threonylcarbamoyladenosine (t6A), N6-methyl-N6-threonylcarbamoyladenosine (m6t6A), 2-methylthio-N6-threonylcarbamoyladenosine (ms2g6A), N6,N6-dimethyladenosine (m62A), N6-hydroxynorvalylcarbamoyladenosine (hn6A), 2-methylthio-N6-hydroxynorvalylcarbamoyladenosine (ms2hn6A), N6-acetyladenosine (ac6A), 7-methyladenine, 2-methylthioadenine, 2-methoxyadenine, alpha-thioadenosine, 2'-O-methyladenosine (Am), N6,2'-O-dimethyladenosine (m6Am), N6,N6,2'-O-trimethyladenosine (m62Am), 1,2'-O-dimethyladenosine (m1Am), 2'-O-ribosyladenosine (phosphate) (Ar(p)), 2-amino-N6-methylpurine, 1-thioadenosine, 8-azidoadenosine, 2'-F-arabinoadenosine, 2'-F-adenosine, 2'-OH-arabinoadenosine, and N6-(19-amino-pentaoxanonadecyl)adenosine.

In some embodiments, the modified nucleoside is a modified guanosine. In some embodiments, 0.1% - 100% of the guanosines in the recombinant RNA molecule are modified. In preferred embodiments, 80% - 100% of the guanosines are modified. In more preferred embodiments, 100% of the guanosines are modified. Exemplary modified guanosines comprise, but not limited to inosine (I), 1-methylinosine (m1I), wyosine (imG), methylwyosine (mimG), 4-demethylwyosine (imG-14), isowyosine (imG2), wybutosine (yW), peroxywybutosine (o2yW), hydroxywybutosine (OHyW), undermodified hydroxywybutosine (OHyW*), 7-deazaguanosine, queuosine (Q), epoxyqueuosine (oQ), galactosyl-queuosine (galQ), mannosyl-queuosine (manQ), 7-cyano-7-deazaguanosine (preQ0), 7-aminomethyl-7-deazaguanosine (preQ1), archaeosine (G+), 7-deaza-8-azaguanosine, 6-thioguanosine, 6-thio-7-deazaguanosine, 6-thio-7-deaza-8-azaguanosine, 7-methylguanosine (m7G), 6-thio-7-methylguanosine, 7-methylinosine, 6-methoxyguanosine, 1-methylguanosine (m1G), N2-methylguanosine (m2G), N2,N2-dimethylguanosine (m22G), N2,7-dimethylguanosine (m2,7G), N2,N2,7-trimethylguanosine (m2,2,7G), 8-oxoguanosine, 7-methyl-8-oxoguanosine, 1-methyl-6-thioguanosine, N2-methyl-6-thioguanosine, N2,N2-dimethyl-6-thioguanosine, alpha-thioguanosine, 2'-O-methylguanosine (Gm), N2-methyl-2'-O-methylguanosine (m2Gm), N2,N2-dimethyl-2'-O-methylguanosine (m22Gm), 1-methyl-2'-O-methylguanosine (m1Gm), N2,7-dimethyl-2'-O-methylguanosine (m2,7Gm), 2'-O-methylinosine (lm), 1,2'-O-dimethylinosine (m1lm), 2'-O-ribosylguanosine (phosphate) (Gr(p)), 1-thioguanosine, O6-methylguanosine, 2'-F-arabinoguanosine, and 2'-F-guanosine.

In some embodiments, in any of the foregoing embodiments, the polypeptide and/or protein of interest refers to a therapeutically or pharmaceutically active polypeptide or protein having therapeutic or prophylactic effects, of which the function in or near cells is required or beneficial, for example, the proteins may include those whose deficiency or defective forms lead to disease occurrence, wherein provision of such proteins may regulate or prevent disease; or the proteins whose presence in or near cells is physiologically advantageous. The polypeptide or protein may comprise the full-length protein or a functional variant thereof.

In any of the foregoing embodiments, the nucleotide sequence encoding the polypeptide and/or protein of interest or the expressed peptide and/or protein comprises or is one or more of: (a) an antigen; (b) a therapeutic protein or polypeptide, a fragment thereof, or a fragment or a variant thereof; and (c) other polypeptide or protein.

In some embodiments, the peptide and/or protein expressed by the nucleotide sequence encoding the polypeptide and/or protein of interest comprises or is an antigen.

In some embodiments, the antigen expressed by the nucleotide sequence encoding the polypeptide and/or protein of interest is derived from one or more of: (1) a pathogenic antigen, a fragment, a variant, or a variant of the fragment thereof; (2) a tumor antigen, a fragment, a variant, or a variant of the fragment thereof; (3) an allergic antigen, a fragment, a variant, or a variant of the fragment thereof; (4) an autoimmune self-antigen, a fragment, a variant, or a variant of the fragment thereof.

In some embodiments, the pathogenic antigen is derived from a pathogenic organism capable of eliciting an immune response in a subject (e.g., a mammalian subject, more particularly a human). In some embodiments, the pathogenic organism comprises or is one or more of: a bacteria, a virus, a fungi, and a protozoa (e.g., unicellular organism, multicellular organism).

In some embodiments, the pathogenic antigen comprises or is a surface antigen, a fragment, a variant thereof, or a variant of the fragment thereof, such as a protein on the surface of a virus, a bacteria, or a protozoan, a fragment (e.g., an external portion of the surface antigen), a variant, or a variant of the fragment thereof.

In some embodiments, the pathogenic antigen comprises or is derived from a polypeptide or protein associated with an infectious pathogen.

In some embodiments, the pathogenic antigen is selected from, but not limited to the group consisting of: pathogen-derived antigens as described in WO2018/078053A1 (pages 21-35); pathogen-derived antigens as described in WO2019/077001A1 (page 57, paragraph 3 to page 63, paragraph 2); pathogen-derived antigens as described in WO2013/120628A1 (page 32, line 26 to page 34, line 27); and antigens as described in WO2013/120628A1 (page 34, line 29 to page 59, line 5).

In some embodiments, the pathogenic antigen is selected from one or more pathogens of, without limitation: Sarcoptes scabiei, Babesia spp., Leishmania spp., Gnathostoma spp., Ancylostoma braziliense, Ancylostoma duodenale, Strongyloides stercoralis, Trichuris trichiura, Toxocara canis, Toxocara cati, Toxoplasma gondii, Trypanosoma brucei, Trypanosoma cruzi, Brugia malayi, Onchocerca volvulus, Wuchereria bancrofti, Taenia spp., Taenia solium, Echinococcus spp., Ascaris lumbricoides, Dientamoeba fragilis, Naegleria fowleri, Necator americanus, Paragonimus spp. (e.g., Paragonimus westermani), Clonorchis sinensis, Plasmodium spp. (e.g., Plasmodium falciparum, Plasmodium vivax, Plasmodium malariae, or Plasmodium ovale), Pneumocystis jirovecii, Metagonimus yokogawai, Trichinella spiralis, Trichomonas vaginalis, Giardia intestinalis, Schistosoma spp., Geotrichum candidum, Saccharomyces cerevisiae, Bartonella henselae, Hortaea wernickeii, Aspergillus spp., Malassezia spp., Vibrio cholerae, Acinetobacter baumannii, Paracoccidioides brasiliensis, Neisseria gonorrhoeae, Neisseria meningitidis, Pasteurella spp., Nocardia asteroides, Nocardia spp., Sporothrix schenckii, Staphylococcus spp., Streptococcus agalactiae, Streptococcus pneumoniae, Streptococcus pyogenes, Trichophyton spp., Yersinia enterocolitica, Yersinia pestis, Yersinia pseudotuberculosis, Ureaplasma urealyticum, Salmonella spp., Francisella tularensis, Fusobacterium spp., Mycobacterium leprae, Mycobacterium lepromatosis, Mycobacterium tuberculosis, Mycobacterium ulcerans, Shigella spp., Arcanobacterium haemolyticum, Bacillus anthracis, Bacillus cereus, Histoplasma capsulatum, Blastomyces dermatitidis, Bordetella pertussis, Borrelia spp., Borrelia burgdorferi, Brucella spp., Burkholderia spp. (e.g., Burkholderia cepacia, Burkholderia mallei, Burkholderia pseudomallei), Campylobacter spp., Candida spp. (e.g., Candida albicans), Chlamydophila pneumoniae, Corynebacterium diphtheriae, Coxiella burnetii, Clostridium spp. (e.g., Clostridium botulinum, Clostridium difficile, Clostridium perfringens), Clostridium tetani, Coccidioides spp., Ehrlichia chaffeensis, Ehrlichia ewingii, Ehrlichia spp., Extraintestinal pathogenic Escherichia coli, Kingella spp., Klebsiella granulomatis, Anaplasma spp. (e.g., Anaplasma phagocytophilum), Leptospira spp., Borrelia burgdorferi, Treponema pallidum, Rickettsia spp. (e.g., Rickettsia prowazekii, Rickettsia rickettsii, Rickettsia typhi), Chlamydophila psittaci, Chlamydia trachomatis, Kuru prion, Lassa virus (LASV), Legionella pneumophila, Listeria monocytogenes, Enterococcus spp., Epidermophyton spp., Escherichia coli O157:H7 and O104:H4, Fasciola hepatica and Fasciola gigantica, Enteroviruses (e.g., Coxsackievirus A, Enterovirus 71 (EV71)), Fatal familial insomnia prion, Creutzfeldt-Jakob disease prion, Epstein-Barr virus (EBV), Feline immunodeficiency virus (FIV), Flavivirus, Gerstmann-Sträussler-Scheinker syndrome prion, Guanarito virus, Haemophilus ducreyi, Haemophilus influenzae, Helicobacter pylori, Bunyaviridae, Caliciviridae, Astroviridae, Coronavirus, Crimean-Congo hemorrhagic fever virus, Cryptococcus neoformans, Cryptosporidium spp., Cytomegalovirus (CMV), BK virus, Dengue virus, Ebola virus (EBOV), Herpes simplex virus (HSV), Human immunodeficiency virus (HIV), Human papillomavirus (HPV), Influenza virus, Rabies virus, Norovirus, Nipah virus, Henipavirus (Hendra-Nipah virus), Hepatitis A virus, Hepatitis B virus (HBV), Hepatitis C virus (HCV), Hepatitis D virus, Hepatitis E virus, Human bocavirus (HBoV), Human metapneumovirus (hMPV), Human parainfluenza virus (HPIV), Japanese encephalitis virus, JC virus, Junin virus, Yellow fever virus, Middle East respiratory syndrome coronavirus (MERS-CoV), Lymphocytic choriomeningitis virus (LCMV), Machupo virus, Marburg virus, Measles virus, Molluscum contagiosum virus (MCV), Mumps virus, Parvovirus B19, Mycoplasma pneumoniae, Orthomyxovirus, Poliovirus, Rhinovirus, Rift Valley fever virus, Rotavirus, Rubella virus, Sabia virus, SARS coronavirus (e.g., SARS-CoV-2), nCoV-2019 coronavirus, Sin Nombre virus, Hantavirus, Vaccinia virus, Respiratory syncytial virus (RSV), Tick-borne encephalitis virus (TBEV), Varicella-zoster virus (VZV), Venezuelan equine encephalitis virus, West Nile virus, Western equine encephalitis virus, and Zika virus.

In some embodiments, the pathogenic antigen comprises or is one or more of:
(1)one or more proteins from SARS-CoV-2, nCoV-2019 coronavirus, or SARS-CoV (SARS-CoV-1) of: spike protein (S), envelope protein (E), membrane protein (M), or nucleocapsid protein (N); (2) one or more proteins from MERS coronavirus of: spike protein (S), spike S1 fragment (S1), envelope protein (E), membrane protein (M), or nucleocapsid protein (N); (3) one or more proteins from human papillomavirus (e.g., HPV16) of: replication protein E1, regulatory protein E2, protein E3, protein E4, protein E5, protein E6, protein E7, protein E8, major capsid protein L1, and minor capsid protein L2; (4) one or more proteins from human parainfluenza virus (HPIV/PIV) (e.g., hPIV-1, hPIV-2, hPIV-3, or hPIV-4 serotypes) of: fusion protein (F), hemagglutinin-neuraminidase, glycoprotein (G), matrix protein (M), phosphoprotein (P), nucleocapsid protein, fusion glycoproteins F0, F1 or F2, recombinant PIV3/PIV1 fusion glycoprotein, C protein, D protein, viral replicase (L), and non-structural V protein; (5) one or more proteins from human metapneumovirus (hMPV) of: fusion (F) glycoprotein, glycoprotein (G), phosphoprotein (P), and nucleocapsid protein; (6) one or more proteins from influenza virus of: hemagglutinin (HA), neuraminidase (NA), nucleoprotein (NP), M1 protein, M2 protein, NS1 protein, NS2 protein (NEP protein: nuclear export protein), PA protein, PB1 protein (polymerase basic 1 protein), PB1-F2 protein, and PB2 protein; (7) one or more proteins from rabies virus of: nucleoprotein (N), large structural protein (L), phosphoprotein (P), matrix protein (M), and glycoprotein (G); (8) one or more proteins from human immunodeficiency virus of: HIV p24 antigen, HIV envelope proteins (Gp120, Gp41, Gp160), GAG polyprotein, negative factor protein Nef, transcriptional transactivator Tat, and Brec1; (9) one or more proteins from Chlamydia trachomatis of: major outer membrane protein MOMP, probable outer membrane protein PMPC, outer membrane complex protein B OmcB, heat shock proteins Hsp60/HSP10, IncA protein, type III secretion system proteins, ribonucleotide reductase small chain protein NrdB, plasmid protein Pgp3, chlamydial outer protein N CopN, and antigens CT521, CT425, CT043, TC0052, TC0189, TC0582, TC0660, TC0726, TC0816, and TC0828; (10) one or more proteins from cytomegalovirus (CMV/HCMV) of: pp65 antigen, membrane protein pp15, tegument protein pp150, M45 protein, DNA polymerase UL54, helicase UL105, glycoproteins gM, gN, gH, gB, proteins UL83, UL94, UL99, HCMV glycoproteins (selected from gH-gL, gB, gO, gN and gM), HCMV proteins (selected from UL83, UL123, UL128, UL130 and UL131A), tegument protein pp150, phosphoprotein pp65, envelope glycoprotein M (UL100), regulatory protein IE1 (UL123), envelope proteins (UL128, UL130, UL131A), envelope glycoprotein B (UL55), structural glycoproteins N (UL73) and O (UL74); (11) one or more proteins from dengue virus of: capsid protein C, pre-membrane protein prM, membrane protein M, envelope protein E (domain I, II, III), proteins NS1, NS2A, NS2B, NS3, NS4A, 2K, NS4B, and NS5; (12) one or more proteins from EBOV virus of: EBOV glycoprotein (GP), surface EBOV GP, wild-type EBOV pro GP, mature EBOV GP, secreted wild-type EBOV pro GP, secreted mature EBOV GP, EBOV nucleoprotein (NP), RNA polymerase L, and EBOV matrix proteins (selected from VP35, VP40, VP24 and VP30); (13) one or more proteins from hepatitis B virus (HBV) of: hepatitis B surface antigen HBsAg, hepatitis B core antigen HbcAg, polymerase, protein Hbx, pre-S2 surface protein, surface protein L, large S protein, and viral proteins VP1, VP2, VP3, and VP4; (14) one or more proteins from respiratory syncytial virus (RSV) of: fusion protein F, F protein, nucleoprotein N, matrix proteins M, M2-1, M2-2, phosphoprotein P, small hydrophobic protein SH, major surface glycoprotein G, polymerase L, non-structural proteins NS1, NS2, RSV attachment protein (G), fusion (F) glycoprotein, nucleoprotein (N), phosphoprotein (P), large polymerase protein (L), matrix proteins (M, M2), small hydrophobic protein (SH), non-structural proteins (NS1, NS2), membrane-bound RSV F protein, and membrane-bound DS Cav1 (prefusion-stabilized RSV F protein); (15) one or more proteins from Mycobacterium tuberculosis of: secreted antigen SssA, chaperone protein DnaK, cell surface lipoprotein Mpt83, lipoprotein P23, phosphate transport system permease pstA, 14kDa antigen, fibronectin-binding protein C FbpC1, alanine dehydrogenase TB43, glutamine synthetase 1, ESX-1 proteins, proteins CFP10, TB10.4, MPT83, MTB12, MTB8, Rpf-like proteins, MTB32, MTB39, crystallin proteins, heat shock protein HSP65, and protein PST-S; (16) one or more proteins from yellow fever virus of: polyprotein, proteins E, M, capsid protein C, protease NS3, proteins NS1, NS2A, NS2B, NS4A, NS4B, and NS5; (17) Cyclosporin protein; and (18) one or more proteins from Zika virus of: Zika virus capsid protein (c), pre-membrane protein (prM), pr protein (pr), membrane protein (M), envelope protein (E), non-structural proteins, prME antigen, capsid protein, pre-membrane/membrane proteins, ZIKV envelope protein, ZIKV non-structural protein 1, ZIKV non-structural protein2A, ZIKV non-structural protein2B, ZIKV non-structural protein3, ZIKV non-structural protein4A, ZIKV non-structural protein4B, ZIKV non-structural protein 5 and Zika virus envelope protein (e).

In some embodiments, the tumor antigen is selected from, but not limited to, the group consisting of tumor antigens described on pages 47-51 of WO2018/078053A1.

In some embodiments, the antigen expressed by the nucleotide sequence encoding the polypeptide and/or protein of interest comprises or is an allergenic antigen and an autoimmune antigen. In some embodiments, the allergenic antigen and autoimmune antigen are derived from or selected from, but not limited to, the antigen group disclosed on pages 59 to 73 of WO2018/078053A1.

In some embodiments, the antigen expressed by the nucleotide sequence encoding the polypeptide and/or protein of interest is listed on pages 48 to 51 of WO2018/078053A1.

In some embodiments, the polypeptide and/or protein expressed by the nucleotide sequence encoding the polypeptide or protein of interest comprises or is a therapeutic protein or polypeptide.

In some embodiments, the therapeutic protein or polypeptide comprises or is one or more of: (1) a therapeutic protein or polypeptide for an enzyme replacement therapy to treat a metabolic, endocrine or amino acid disorder, or for replacing missing, defective or mutated proteins; (2) a therapeutic protein or polypeptide for treating a blood disorder, a circulatory system disorder, a respiratory disorder, an infectious disease or a immunodeficiency disorder; (3) a therapeutic protein or polypeptide for treating a cancer or neoplastic disease; (4) a therapeutic protein or polypeptide for a hormone replacement therapy; (5) a therapeutic protein or polypeptide for reprogramming somatic cells into pluripotent stem cells or totipotent stem cells; (6) a therapeutic protein or polypeptide for use as an adjuvant or an immunostimulant; (7) a therapeutic protein or polypeptide as a therapeutic antibody; (8) a therapeutic protein or polypeptide as a gene editing agent; (9) a therapeutic protein or polypeptide for treating or preventing a liver disease selected from the group consisting of liver fibrosis, cirrhosis and liver cancer; and (10) a therapeutic protein or polypeptide for treating or preventing a rare disease.

In some embodiments, the therapeutic protein or polypeptide for enzyme replacement therapy to treat a metabolic, endocrine or amino acid disorder, or for replacing missing, defective or mutated proteins, comprises or is one or more of: acid sphingomyelinase, fatty acids, aglucosidase beta, alglucosidase, alpha-galactosidase A, alpha-glucosidase, alpha-L-iduronidase, alpha-N-acetylglucosaminidase, amphiregulin, angiopoietins (Ang1, Ang2, Ang3, Ang4, ANGPTL2, ANGPTL3, ANGPTL4, ANGPTL5, ANGPTL6, ANGPTL7), ATPase, Cu(2+)-transporting beta polypeptide (ATP7B), argininosuccinate synthase (ASS1), betacellulin, beta-glucuronidase, bone morphogenetic proteins (BMP1, BMP2, BMP3, BMP4, BMP5, BMP6, BMP7, BMP8a, BMP8b, BMP10, BMP15), CLN6 protein, epidermal growth factor (EGF), epiregulin, epigen, fibroblast growth factors (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-18, FGF-19, FGF-20, FGF-21, FGF-22, FGF-23), fumarylacetoacetate hydrolase (FAH), galactocerebrosidase, ghrelin, glucocerebrosidase, GM-CSF, heparin-binding EGF-like growth factor (HB-EGF), hepatocyte growth factor (HGF), hepatopoietin, human albumin, iduronate sulfatase, integrins αVβ3, αVβ5 and α5β1, iduronate-2-sulfatase, laronidase, N-acetylgalactosamine-4-sulfatase (rhASB; galsulfase), arylsulfatase A (ARSA), arylsulfatase B (ARSB), N-acetylglucosamine-6-sulfatase, nerve growth factor (NGF), brain-derived neurotrophic factor (BDNF), neurotrophin-3 (NT-3), neurotrophin-4/5 (NT-4/5), neuregulins (NRG1, NRG2, NRG3, NRG4), neuropilins (NRP-1, NRP-2), obestatin, phenylalanine hydroxylase (PAH), phenylalanine ammonia-lyase (PAL), platelet-derived growth factors (PDGF-A, PDGF-B, PDGF-C, PDGF-D), TGFβ receptors (endoglin, TGFβ1 receptor, TGFβ2 receptor, TGFβ3 receptor), thrombopoietin (THPO; megakaryocyte growth and development factor (MGDF)), transforming growth factors (TGF-α, TGF-β1, TGF-β2, TGF-β3), vascular endothelial growth factors (VEGF-A, VEGF-B, VEGF-C, VEGF-D, VEGF-E, VEGF-F, PIGF), nesiritide, trypsin, adrenocorticotropic hormone (ACTH), atrial natriuretic peptide (ANP), cholecystokinin, gastrin, leptin, oxytocin, somatostatin, vasopressin (antidiuretic hormone), calcitonin, exenatide, growth hormone (GH), insulin, insulin-like growth factor 1 (IGF-1), mecasermin, IGF-1 analogs, pegvisomant, pramlintide, teriparatide (human parathyroid hormone 1-34), becaplermin, dibotermin alfa (bone morphogenetic protein 2), histrelin acetate (gonadotropin-releasing hormone; GnRH), octreotide, hepatocyte nuclear factor 4α (HNF4A), CCAAT/enhancer-binding protein alpha (CEBPA), fibroblast growth factor 21 (FGF21), extracellular matrix metalloproteinases or human collagenase MMP1, hepatocyte growth factor (HGF), TNF-related apoptosis-inducing ligand (TRAIL), opioid growth factor receptor-like 1 (OGFRL1), clostridial collagenase, relaxin-1 (RLN1), relaxin-2 (RLN2), relaxin-3 (RLN3), and palifermin (keratinocyte growth factor; KGF).

In some embodiments, the therapeutic protein or polypeptide for treating a metabolic or endocrine disorder is selected from the proteins or polypeptides listed in Table A (in conjunction with Table C) of WO2017/191274.

In some embodiments, the therapeutic protein or polypeptide for treating a hematologic disorder, a circulatory system disorder, a respiratory disorder, a cancer or a neoplastic disease, an infectious disease or an immunodeficiency disorder comprises or is one or more of: alteplase (tissue plasminogen activator; tPA), anistreplase, antithrombin III (AT-III), bivalirudin, darbepoetin alfa, drotrecogin alfa (activated protein C), epoetin, epoetin α, erythropoietin, factor IX, factor Vlla, factor VIII, lepirudin, protein C concentrate, reteplase (deletion mutant of tPA), streptokinase, tenecteplase, urokinase, angiostatin, anti-CD22 immunotoxin, denileukin diftitox, immunocyanin, MPS (zinc finger protein), aflibercept, endostatin, collagenase, human deoxyribonuclease I, deoxyribonuclease, hyaluronidase, papain, L-asparaginase, pegaspargase, rasburicase, human chorionic gonadotropin (hCG), human follicle-stimulating hormone (FSH), lutropin alfa, prolactin, alpha-1-proteinase inhibitor, lactase, pancrelipase (lipase, amylase, protease), adenosine deaminase (bovine pegademase, PEG-ADA), abatacept, alefacept, anakinra, etanercept, interleukin-1 (IL-1 receptor antagonist), anakinra, thymopentin, TNF-α antagonist, enfuvirtide, and thymosin α1.

In some embodiments, the therapeutic protein or polypeptide for treating a cancer or a neoplastic disease comprises or is one or more of: cytokines, chemokine, suicide gene product, immunogenic protein or peptide, apoptosis inducer, angiogenesis inhibitor, heat shock protein, tumor antigen, β-catenin inhibitor, STING pathway activator, checkpoint modulator, innate immune activator, antibody, dominant negative receptor and decoy receptor, myeloid-derived suppressor cell (MDSC) inhibitor, IDO pathway inhibitor, and protein or peptide binding to apoptosis inhibitor.

In some embodiments, the hormone in the therapeutic protein or polypeptide for hormone replacement therapy comprise or is one or more of: estrogen, corporin, progesterone, and testosterone.

In some embodiments, the therapeutic protein for reprogramming somatic cells into pluripotent or totipotent stem cells comprise or is one or more of: Oct-3/4, Sox gene family (e.g., Sox1, Sox2, Sox3, and Sox15), Klf family (e.g., Klf1, Klf2, Klf4, and Klf5), Myc family (e.g., c-Myc, L-Myc, and N-Myc), Nanog, and LIN28.

In some embodiments, the therapeutic protein or polypeptide for use as an adjuvant or an immunostimulatory protein comprises or is of one or more of: human adjuvant protein, particularly pattern recognition receptor including TLR1, TLR2, TLR3, TLR4, TLR5, TLR6, TLR7, TLR8, TLR9, TLR10, and TLR11; NOD1, NOD2, NOD3, NOD4, NOD5, NALP1, NALP2, NALP3, NALP4, NALP5, NALP6, NALP7, NALP8, NALP9, NALP10, NALP11, NALP12, NALP13, and NALP14; IPAF; NAIP; CIITA; RIG-I; MDA5; and LGP2; signal transducer of TLR signaling including adaptor protein (e.g., TRIF and CARDIF), component of small GTPase signaling (e.g., RhoA, Ras, Rac1, Cdc42, Rab, etc.), component of PIP signaling (e.g., PI3K, Src kinases, etc.), component of MyD88-dependent signaling (e.g., MyD88, IRAK1, IRAK2, IRAK4, TIRAP, TRAF6, etc.), and component of MyD88-independent signaling (e.g., TICAM1, TICAM2, TRAF6, TBK1, IRF3, TAK1, IRAK1, etc.); activated kinase (e.g., Akt, MEKK1, MKK1, MKK3, MKK4, MKK6, MKK7, ERK1, ERK2, GSK3, PKC kinase, PKD kinase, GSK3 kinase, JNK, p38 MAPK, TAK1, IKK, etc.); activated transcription factor (e.g., NF-κB, c-Fos, c-Jun, c-Myc, CREB, AP-1, Elk-1, ATF2, IRF-3, IRF-7, etc.); heat shock protein (e.g., HSP10, HSP60, HSP65, HSP70, HSP75, and HSP90); gp96; fibrinogen; fibronectin type III repeat extra-domain; component of the complement system (e.g., C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P, and CD59); and cell surface protein inducing target gene (e.g., β-defensin). In some embodiments, the human adjuvant protein comprise or is one or more of: TRIF, FLT-3 ligand, gp96, or fibronectin; cytokine that induce or enhance innate immune response (e.g., IL-1α, IL-1R1, IL-1β, IL-2, IL-6, IL-7, IL-8, IL-9, IL-12, IL-13, IL-15, IL-16, IL-17, IL-18, IL-21, IL-23, TNF-α, IFN-α, IFN-β, IFN-γ, GM-CSF, G-CSF, and M-CSF); chemokines (e.g., IL-8, IP-10, MCP-1, MIP-1α, RANTES, eotaxin, and CCL21); and cytokine released by macrophage (e.g., IL-1, IL-6, IL-8, IL-12, and TNF-α).

In some embodiments, the therapeutic protein or polypeptide for use as an adjuvant or an immunostimulant comprises or is one or more of: bacterial (adjuvant) protein, protozoan (adjuvant) protein, viral (adjuvant) protein, fungal (adjuvant) protein, and animal-derived protein.

In some embodiments, the bacterial (adjuvant) protein comprise one or more of: bacterial heat shock protein or chaperone (including Hsp60, Hsp70, Hsp90, and Hsp100); Gram-negative bacterial OmpA (outer membrane protein); OspA; bacterial porin (e.g., OmpF); bacterial toxin (e.g., pertussis toxin (PT) from Bordetella pertussis, B. pertussis adenylate cyclase toxin CyaA and CyaC, pertussis toxin PT-9K/129G mutant, B. pertussis adenylate cyclase toxin CyaA and CyaC, tetanus toxin, cholera toxin (CT), cholera toxin B subunit, cholera toxin CTK63 mutant, CTE112K mutant of CT, Escherichia coli heat-labile enterotoxin (LT), heat-labile enterotoxin with reduced toxicity (LTB) B subunit of E. coli heat-labile enterotoxin mutant (e.g., LTK63, LTR72)); phenol-soluble modulin; Helicobacter pylori neutrophil-activating protein (HP-NAP); surfactant protein D; Borrelia burgdorferi outer surface protein A lipoprotein; Mycobacterium tuberculosis Ag38 (38 kDa antigen); bacterial pilin (e.g., pilin from Gram-negative bacterial pili); surfactant protein A; and bacterial flagellin.

In some embodiments, the protozoan (adjuvant) protein comprises one or more of: Tc52 from Trypanosoma cruzi, PFTG from Toxoplasma gondii, protozoan heat shock protein, LelF from Leishmania spp., and profilin-like protein from Toxoplasma gondii.

In some embodiments, the viral (adjuvant) protein comprise one or more of: respiratory syncytial virus fusion glycoprotein (F protein), MMT virus envelop protein, murine leukemia virus protein, and wild-type measles virus hemagglutinin protein.

In some embodiments, the fungal (adjuvant) protein comprises fungal immunomodulatory protein (FIPs, e.g., LZ-8).

In some embodiments, the animal-derived protein comprises keyhole limpet hemocyanin (KLH).

In some embodiments, the polypeptide and/or protein expressed by a nucleotide sequence encoding a polypeptide and/or protein of interest comprises or is a therapeutic protein or polypeptide as therapeutic antibody, for example, one or more of those cited in Tables 1, 2, 3, 4, 5, 6, 7, 8, 9, 10, 11, and 12 of WO2016/170176A1: cytokines, chemokines, suicide enzymes and gene products, apoptosis inducers, endogenous angiogenesis inhibitors, heat shock proteins, tumor antigens, innate immune activators, and antibodies targeting proteins associated with tumor or cancer development.

### V. DNA molecule or vector encoding the recombinant RNA molecule comprising the 5' and/or 3'-UTR of the present disclosure

Provided is a DNA molecule, encoding the 5'-UTR, 3'-UTR and/or recombinant RNA molecule according to the present disclosure. Provided are also a vector comprising the recombinant RNA molecule or DNA molecule according to the present disclosure and a host cell comprising the recombinant RNA molecule, DNA molecule and/or vector according to the present disclosure.

Provided is also a vector, comprising a first nucleotide sequence encoding a 5'-UTR and/or a second nucleotide sequence encoding a 3'-UTR, wherein the first nucleotide sequence comprises at least one of the following polynucleotides: (a) a polynucleotide encoding a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) a polynucleotide encoding a fragment of the 5'-UTR in (a); (c) a polynucleotide encoding a variant of the 5'-UTR in (a); and (d) a polynucleotide encoding a variant of the fragment in (b); the second nucleotide sequence comprises at least one of the following polynucleotides: (e) a polynucleotide encoding a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2; (f) a polynucleotide encoding a fragment of the 3'-UTR in (e); (g) a polynucleotide encoding a variant of the 3'-UTR in (e); and (h) a polynucleotide encoding a variant of the fragment in (f).

In some embodiments, the gene is a human gene.

In some embodiments, the first nucleotide sequence comprises at least one of the following polynucleotides: a polynucleotide having a sequence as set forth in at least one of SEQ ID NOs: 1-21; a fragment of the polynucleotide having a sequence as set forth in at least one of SEQ ID NOs: 1-21; a variant of the polynucleotide having a sequence as set forth in at least one of SEQ ID NOs: 1-21; and a variant of the fragment of the polynucleotide having a sequence as set forth in at least one of SEQ ID NOs: 1-21. In some embodiments, the fragment, the variant, or the variant of the fragment of the polynucleotide having a sequence as set forth in at least one of SEQ ID NOs: 1-21 has at least 40%, 50%, 60%, 70%, 80%, 85%, 90%, 91%, 92%, 93%, 94%, 95%, 96%, 97%, 98%, or 99% identify to the polynucleotide having a sequence as set forth in one of SEQ ID NOs: 1-21. In some embodiments, the fragment, the variant, or the variant of the fragment of the polynucleotide having a sequence as set forth in at least one of SEQ ID NOs: 1-21 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 1-21.

In a preferred embodiment, the gene is selected from at least one of PPIA, HPX, FTCD, CDK5RAP3, HSPA8, HBA1, and HBB. In some embodiments, the first nucleotide sequence comprises at least one of the following polynucleotides:1) a polynucleotide having a sequence as set forth in SEQ ID NO: 9, 7, 18, 12, 8, 1, or 6; 2) a fragment of the polynucleotide in 1); 3) a variant of the polynucleotide in 1); and 4) a variant of the fragment in 2). In some embodiments, the fragment, the variant, or the variant of the fragment of the polynucleotide having a sequence as set forth in SEQ ID NO: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to the polynucleotide having a sequence as set forth in SEQ ID NO: 9, 7, 18, 12, 8, 1, or 6. In some embodiments, the fragment, the variant, or the variant of the fragment of the RNA encoded by a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 9, 7, 18, 12, 8, 1, or 6.

The first nucleotide sequence may comprise two or more tandem polynucleotides encoding 5'-UTRs of the above genes; polynucleotides encoding fragments of 5'-UTRs of the above genes; polynucleotides encoding variants of 5'-UTRs of the above genes; polynucleotides encoding variants of the fragments of 5'-UTRs of the above genes.

In some embodiments, the first nucleotide sequence comprises at least one of the following polynucleotides: (a) a polynucleotide encoding 5'-UTRs derived from at least two genes of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; (b) a polynucleotide encoding fragments of 5'-UTRs of at least two genes of the genes in (a); (c) a polynucleotide encoding variants of 5'-UTRs of at least two genes of the genes in (a); and (d) a polynucleotide encoding variants of the fragments of 5'-UTRs of at least two genes of the genes in (a). In some embodiments, the first nucleotide sequence comprises at least one of the following polynucleotides: (e) a polynucleotide encoding 5'-UTRs derived from at least two genes of genes PPIA, HPX, FTCD, CDK5RAP3, HSPA8, HBA1, and HBB; (b) a polynucleotide encoding fragments of 5'-UTRs in (e); (f) a polynucleotide encoding variants of 5'-UTRs in (e); and (h) a polynucleotide encoding variants of the fragments of 5'-UTRs in (f).

In some embodiments, the first nucleotide sequence comprises at least one of the following polynucleotides: a polynucleotide having a sequence as set forth in at least two of SEQ ID NOs: 1-21; a fragment of the polynucleotide having a sequence as set forth in at least two of SEQ ID NOs: 1-21; a variant of the polynucleotide having a sequence as set forth in at least two of SEQ ID NOs: 1-21; and a variants of the fragment of a polynucleotide having a sequence as set forth in at least two of SEQ ID NOs: 1-21. In some embodiments, the fragment, the variant, or the variant of the fragment of a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to a polynucleotide having a sequence as set forth in one of SEQ ID NOs: 1-21. In some embodiments, the fragment, the variant, or the variant of the fragment of a polynucleotide having a sequence as set forth in one of SEQ ID NOs: 1-21has insertions, additions, deletions, or substitutions of at least 1, 2, 3, 4, 5, 6, 7, 8, 9, 10, or more nucleotides, as compared to a polynucleotide having a sequence as set forth in one of SEQ ID NOs: 1-21.

In some embodiments, the first nucleotide sequence comprises at least one of the following polynucleotides: a polynucleotide having a sequence as set forth in at least two of SEQ ID NOs: 9, 7, 18, 12, 8, 1, and 6; a fragment of the polynucleotide having a sequence as set forth in at least two of SEQ ID NOs: 9, 7, 18, 12, 8, 1, and 6; a variant of the polynucleotide having a sequence as set forth in at least two of SEQ ID NOs: 9, 7, 18, 12, 8, 1, and 6; and a variants of the fragment of a polynucleotide having a sequence as set forth in at least two of SEQ ID NOs: 9, 7, 18, 12, 8, 1, and 6. In some embodiments, the fragment, the variant, or the variant of the fragment of a polynucleotide having a sequence as set forth in SEQ ID NOs: 9, 7, 18, 12, 8, 1, or 6 has at least 40%, 50%, 60%, 70%, 80%, 85%, 90%, 91%, 92%, 93%, 94%, 95%, 96%, 97%, 98%, or 99% identity to a polynucleotide having a sequence as set forth in SEQ ID NOs: 9, 7, 18, 12, 8, 1, or 6. In some embodiments, the fragment, the variant, or the variant of the fragment of a polynucleotide having a sequence as set forth in SEQ ID NOs: 9, 7, 18, 12, 8, 1, or 6 has insertions, additions, deletions, or substitutions of at least 1, 2, 3, 4, 5, 6, 7, 8, 9, 10, or more nucleotides, as compared to a polynucleotide having a sequence as set forth in SEQ ID NOs: 9, 7, 18, 12, 8, 1, or 6.

In some embodiments, the first nucleotide sequence comprises at least one of the following polynucleotides: a): at least two copies of a polynucleotide encoding a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; b) at least two copies of a fragment of the polynucleotide encoding the 5'-UTR of at least one gene from the genes in a); c) at least two copies of a variant of the polynucleotide encoding the 5'-UTR of at least one gene from the genes in a); and d) at least two copies of a variant of the fragment of the polynucleotide encoding the 5'-UTR of at least one gene from the genes in a). In some embodiments, the first nucleotide sequence comprises at least one of the following polynucleotides: e): at least two copies of a polynucleotide encoding a 5'-UTR derived from at least one gene of genes PPIA, HPX, FTCD, CDK5RAP3, HSPA8, HBA1, and HBB; f) at least two copies of a fragment of the polynucleotide encoding the 5'-UTR of at least one gene from the genes in e); g) at least two copies of a variant of the polynucleotide encoding the 5'-UTR of at least one gene from the genes in e); and h) at least two copies of a variant of the fragment of the polynucleotide encoding the 5'-UTR of at least one gene from the genes in e). Preferably, at least two copies are two copies, three copies, four copies, five copies, six copies, seven copies, eight copies, or nine copies.

In some embodiments, the first nucleotide sequence comprises at least one of the following polynucleotides: at least two copies of a polynucleotide having a sequence as set forth in at least one of SEQ ID NOs: 1-21; at least two copies of a fragment of the polynucleotide having a sequence as set forth in at least one of SEQ ID NOs: 1-21; at least two copies of a variant of the polynucleotide having a sequence as set forth in at least one of SEQ ID NOs: 1-21; and at least two copies of a variants of the fragment of a polynucleotide having a sequence as set forth in at least one of SEQ ID NOs: 1-21. Preferably, at least two copies are two copies, three copies, four copies, five copies, six copies, seven copies, eight copies, or nine copies. In some embodiments, the fragment, the variant, or the variant of the fragment of a polynucleotide having a sequence as set forth in one of SEQ ID NOs: 1-21 has at least 40%, 50%, 60%, 70%, 80%, 85%, 90%, 91%, 92%, 93%, 94%, 95%, 96%, 97%, 98%, or 99% identity to a polynucleotide having a sequence as set forth in one of SEQ ID NOs: 1-21. In some embodiments, the fragment, the variant, or the variant of the fragment of a polynucleotide having a sequence as set forth in one of SEQ ID NOs: 1-21 has insertions, additions, deletions, or substitutions of at least 1, 2, 3, 4, 5, 6, 7, 8, 9, 10, or more nucleotides, as compared to a polynucleotide having a sequence as set forth in one of SEQ ID NOs: 1-21.

In some embodiments, the first nucleotide sequence comprises at least one of the following polynucleotides: at least two copies of a polynucleotide having a sequence as set forth in one of SEQ ID NOs: 9, 7, 18, 12, 8, 1, and 6; at least two copies of a fragment of the polynucleotide having a sequence as set forth in one of SEQ ID NOs: 9, 7, 18, 12, 8, 1, and 6; at least two copies of a variant of the polynucleotide having a sequence as set forth in one of SEQ ID NOs: 9, 7, 18, 12, 8, 1, and 6; and at least two copies of a variants of the fragment of a polynucleotide having a sequence as set forth in one of SEQ ID NOs: 9, 7, 18, 12, 8, 1, and 6. Preferably, at least two copies are two copies, three copies, four copies, five copies, six copies, seven copies, eight copies, or nine copies. In some embodiments, the fragment, the variant, or the variant of the fragment of a polynucleotide having a sequence as set forth in one of SEQ ID NOs: 9, 7, 18, 12, 8, 1, and 6 has at least 40%, 50%, 60%, 70%, 80%, 85%, 90%, 91%, 92%, 93%, 94%, 95%, 96%, 97%, 98%, or 99% identity to a polynucleotide having a sequence as set forth in SEQ ID NO: 9, 7, 18, 12, 8, 1, or 6. In some embodiments, the fragment, the variant, or the variant of the fragment of a polynucleotide having a sequence as set forth in SEQ ID NO: 9, 7, 18, 12, 8, 1, or 6 has insertions, additions, deletions, or substitutions of at least 1, 2, 3, 4, 5, 6, 7, 8, 9, 10, or more nucleotides, as compared to a polynucleotide having a sequence as set forth in SEQ ID NO: 9, 7, 18, 12, 8, 1, or 6.

In some embodiments, the second nucleotide sequence comprises at least one of the following polynucleotides: a polynucleotide having a sequence as set forth in at least one of SEQ ID NOs: 22-48; a fragment of the polynucleotide having a sequence as set forth in at least one of SEQ ID NOs: 22-48; a variant of the polynucleotide having a sequence as set forth in at least one of SEQ ID NOs: 22-48; and a variant of the fragment of the polynucleotide having a sequence as set forth in at least one of SEQ ID NOs: 22-48. In some embodiments, the fragment, the variant, or the variant of the fragment of the polynucleotide having a sequence as set forth in at least one of SEQ ID NOs: 22-48 has at least 40%, 50%, 60%, 70%, 80%, 85%, 90%, 91%, 92%, 93%, 94%, 95%, 96%, 97%, 98%, or 99% identify to the polynucleotide having a sequence as set forth in one of SEQ ID NOs: 22-48. In some embodiments, the fragment, the variant, or the variant of the fragment of the polynucleotide having a sequence as set forth in at least one of SEQ ID NOs: 22-48 has insertions, additions, deletions, or substitutions of at least 1, 2, 3, 4, 5, 6, 7, 8, 9, 10, or more nucleotides, as compared to the RNA encoded by a polynucleotide having a sequence as set forth in one of SEQ ID NOs: 22-48.

In a preferred embodiment, the gene is selected from at least one of MPND, FBXW10, FBXW12, and PGLYRP1. In some embodiments, the second nucleotide sequence comprises at least one of the following polynucleotides: a polynucleotide having a sequence as set forth in SEQ ID NO: 24, 22, 23, or 25; a fragment of the polynucleotide having a sequence as set forth in SEQ ID NO: 24, 22, 23, or 25; a variant of the polynucleotide having a sequence as set forth in SEQ ID NO: 24, 22, 23, or 25; and a variant of the fragment of the polynucleotide having a sequence as set forth in SEQ ID NO: 24, 22, 23, or 25. In some embodiments, the fragment, the variant, or the variant of the fragment of the polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to the polynucleotide having a sequence as set forth in SEQ ID NO: 24, 22, 23, or 25. In some embodiments, the fragment, the variant, or the variant of the fragment of the RNA encoded by a polynucleotide having a sequence as set forth in SEQ ID NO: 24, 22, 23, or 25 has insertions, additions, deletions, or substitutions of at least 1, 2, 3, 4, 5, 6, 7, 8, 9, 10, or more nucleotides, as compared to the RNA encoded by a polynucleotide having a sequence as set forth in SEQ ID NO: 24, 22, 23, or 25.

The second nucleotide sequence may comprise two or more tandem polynucleotides encoding 3'-UTRs of the above genes; polynucleotides encoding fragments of 3'-UTRs of the above genes; polynucleotides encoding variants of 3'-UTRs of the above genes; polynucleotides encoding variants of the fragments of 3'-UTRs of the above genes.

In some embodiments, the second nucleotide sequence comprises at least one of the following polynucleotides: (a) a polynucleotide encoding 3'-UTRs derived from at least two genes of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; (b) a polynucleotide encoding fragments of 3'-UTRs of at least two genes of the genes in (a); (c) a polynucleotide encoding variants of 3'-UTRs of at least two genes of the genes in (a); and (d) a polynucleotide encoding variants of the fragments of 3'-UTRs of at least two genes of the genes in (a). In some embodiments, the second nucleotide sequence comprises at least one of the following polynucleotides: (e) a polynucleotide encoding 3'-UTRs derived from at least two genes of genes MPND, FBXW10, FBXW12, and PGLYRP1; (b) a polynucleotide encoding fragments of 3'-UTRs in (e); (f) a polynucleotide encoding variants of 3'-UTRs in (e); and (h) a polynucleotide encoding variants of the fragments of 3'-UTRs in (f).

In some embodiments, the second nucleotide sequence comprises at least one of the following polynucleotides: a polynucleotide having a sequence as set forth in at least two of SEQ ID NOs: 22-48; a fragment of the polynucleotide having a sequence as set forth in at least two of SEQ ID NOs: 22-48; a variant of the polynucleotide having a sequence as set forth in at least two of SEQ ID NOs: 22-48; and a variants of the fragment of a polynucleotide having a sequence as set forth in at least two of SEQ ID NOs: 22-48. In some embodiments, the fragment, the variant, or the variant of the fragment of a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to a polynucleotide having a sequence as set forth in one of SEQ ID NOs: 22-48. In some embodiments, the fragment, the variant, or the variant of the fragment of a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to a polynucleotide having a sequence as set forth in one of SEQ ID NOs: 22-48.

In some embodiments, the second nucleotide sequence comprises at least one of the following polynucleotides: a polynucleotide having a sequence as set forth in at least two of SEQ ID NOs: 24, 22, 23, and 25; a fragment of the polynucleotide having a sequence as set forth in at least two of SEQ ID NOs: 24, 22, 23, and 25; a variant of the polynucleotide having a sequence as set forth in at least two of SEQ ID NOs: 24, 22, 23, and 25; and a variants of the fragment of a polynucleotide having a sequence as set forth in at least two of SEQ ID NOs: 24, 22, 23, and 25. In some embodiments, the fragment, the variant, or the variant of the fragment of a polynucleotide having a sequence as set forth in SEQ ID NO: 24, 22, 23, or 25 has at least 40%, 50%, 60%, 70%, 80%, 85%, 90%, 91%, 92%, 93%, 94%, 95%, 96%, 97%, 98%, or 99% identity to a polynucleotide having a sequence as set forth in SEQ ID NO: 24, 22, 23, or 25. In some embodiments, the fragment, the variant, or the variant of the fragment of a polynucleotide having a sequence as set forth in SEQ ID NO: 24, 22, 23, or 25 has insertions, additions, deletions, or substitutions of at least 1, 2, 3, 4, 5, 6, 7, 8, 9, 10, or more nucleotides, as compared to a polynucleotide having a sequence as set forth in SEQ ID NO: 24, 22, 23, or 25.

In some embodiments, the second nucleotide sequence comprises at least one of the following polynucleotides: a): at least two copies of a polynucleotide encoding a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2; b) at least two copies of a fragment of the polynucleotide encoding the 3'-UTR of at least one gene from the genes in a); c) at least two copies of a variant of the polynucleotide encoding the 3'-UTR of at least one gene from the genes in a); and d) at least two copies of a variant of the fragment of the polynucleotide encoding the 3'-UTR of at least one gene from the genes in a). In some embodiments, the first nucleotide sequence comprises at least one of the following polynucleotides: e): at least two copies of a polynucleotide encoding a 3'-UTR derived from at least one gene of genes MPND, FBXW10, FBXW12, and PGLYRP1; f) at least two copies of a fragment of the polynucleotide encoding the 3'-UTR of at least one gene from the genes in e); g) at least two copies of a variant of the polynucleotide encoding the 3'-UTR of at least one gene from the genes in e); and h) at least two copies of a variant of the fragment of the polynucleotide encoding the 3'-UTR of at least one gene from the genes in e). Preferably, at least two copies are two copies, three copies, four copies, five copies, six copies, seven copies, eight copies, or nine copies.

In some embodiments, the second nucleotide sequence comprises at least one of the following polynucleotides: at least two copies of a polynucleotide having a sequence as set forth in at least one of SEQ ID NOs: 22-48; at least two copies of a fragment of the polynucleotide having a sequence as set forth in at least one of SEQ ID NOs: 22-48; at least two copies of a variant of the polynucleotide having a sequence as set forth in at least one of SEQ ID NOs: 22-48; and at least two copies of a variants of the fragment of a polynucleotide having a sequence as set forth in at least one of SEQ ID NOs: 22-48. Preferably, at least two copies are two copies, three copies, four copies, five copies, six copies, seven copies, eight copies, or nine copies. In some embodiments, the fragment, the variant, or the variant of the fragment of a polynucleotide having a sequence as set forth in one of SEQ ID NOs: 22-48 has at least 40%, 50%, 60%, 70%, 80%, 85%, 90%, 91%, 92%, 93%, 94%, 95%, 96%, 97%, 98%, or 99% identity to a polynucleotide having a sequence as set forth in one of SEQ ID NOs: 22-48. In some embodiments, the fragment, the variant, or the variant of the fragment of a polynucleotide having a sequence as set forth in one of SEQ ID NOs: 22-48 has insertions, additions, deletions, or substitutions of at least 1, 2, 3, 4, 5, 6, 7, 8, 9, 10, or more nucleotides, as compared to a polynucleotide having a sequence as set forth in one of SEQ ID NOs: 22-48.

In some embodiments, the first nucleotide sequence comprises at least one of the following polynucleotides: at least two copies of a polynucleotide having a sequence as set forth in one of SEQ ID NOs: 24, 22, 23, and 25; at least two copies of a fragment of the polynucleotide having a sequence as set forth in one of SEQ ID NOs: 24, 22, 23, and 25; at least two copies of a variant of the polynucleotide having a sequence as set forth in one of SEQ ID NOs: 24, 22, 23, and 25; and at least two copies of a variants of the fragment of a polynucleotide having a sequence as set forth in one of SEQ ID NOs: 24, 22, 23, and 25. Preferably, at least two copies are two copies, three copies, four copies, five copies, six copies, seven copies, eight copies, or nine copies. In some embodiments, the fragment, the variant, or the variant of the fragment of a polynucleotide having a sequence as set forth in one of SEQ ID NOs: 24, 22, 23, and 25 has at least 40%, 50%, 60%, 70%, 80%, 85%, 90%, 91%, 92%, 93%, 94%, 95%, 96%, 97%, 98%, or 99% identity to a polynucleotide having a sequence as set forth in SEQ ID NO: 24, 22, 23, or 25. In some embodiments, the fragment, the variant, or the variant of the fragment of a polynucleotide having a sequence as set forth in one of SEQ ID NOs: 24, 22, 23, and 25 has insertions, additions, deletions, or substitutions of at least 1, 2, 3, 4, 5, 6, 7, 8, 9, 10, or more nucleotides, as compared to a polynucleotide having a sequence as set forth in SEQ ID NOs: 24, 22, 23, or 25.

In some embodiments, the vector is used for producing a recombinant RNA molecule, such as an mRNA molecule. In some embodiments, the vector comprises a first nucleotide sequence and a second nucleotide sequence. In some embodiments, the vector further comprises a third nucleotide sequence encoding a polypeptide and/or protein of interest, between the first nucleotide sequence and the second nucleotide sequence. In some embodiments, the third nucleotide sequence encodes at least one polypeptide of interest, for example, one, two, three, four, five, six, seven, eight, nine, or ten polypeptides of interest. In some embodiments, the third nucleotide sequence encodes at least one protein of interest, for example, one, two, three, four, five, six, seven, eight, nine, or ten proteins of interest. In some embodiments, the third nucleotide sequence encodes at least one polypeptide of interest and at least one protein of interest, for example, one, two, three, four, five, six, seven, eight, nine, or ten polypeptides of interest, and one, two, three, four, five, six, seven, eight, nine, or ten proteins of interest.

In some embodiments, the nucleotides of the poly(A) tail comprise at least 20, at least 40, at least 80, at least 100, or at least 120 A nucleotides. Preferably, the nucleotides of the poly(A) tail comprise at least 20, at least 40, at least 80, at least 100, or at least 120 consecutive A nucleotides. In some embodiments, the poly(A) tail comprises one or more nucleotides other than A nucleotide. In some embodiments, the poly(A) tail comprises two or more consecutive nucleotides other than A nucleotide, wherein the first and last nucleotides of the sequence having two or more consecutive nucleotides are nucleotides other than A nucleotide. Preferably, the poly(A) tail has a segmented configuration, that is, m consecutive A nucleotides are connected to n consecutive A nucleotides via a linker sequence composed of p nucleotides other than A nucleotide, wherein m, n, and p are positive integers. Preferably, m is 30, n is 70, and p is 10.

In some embodiments, the DNA sequence corresponding to the poly(A) tail is as set forth in SEQ ID NO: 53.

Additionally, the vector may further comprise an expression control element for proper expression of the vector in a host. Such control elements are well-known to those skilled in the art and may comprise, but not limited to, a promoter, a splicing cassette, a translation initiation codon, and a translation/insertion site for introducing an insert into the vector.

In some embodiments, the vector of the present disclosure may be, for example, a plasmid, a cosmid, a virus, a bacteriophage, or another vector conventionally used in genetic engineering, and may further comprise an additional gene such as a marker gene that permits selection of the vector in a suitable host cell under appropriate conditions.

The recombinant RNA molecule and vector of the present disclosure can be introduced into a cell either directly or via a delivery system including, but not limited to: liposome, viral vector (e.g., adenovirus, retrovirus), electroporation, ballistic method (e.g., gene gun), or other transfection systems.

Additionally, provided is a method for preparing an mRNA, comprising contacting the vector according to the present disclosure with an RNA polymerase.

In some embodiments, the method of the present disclosure further comprises a step of linearizing the plasmid. In some embodiments, prior to linearization, the plasmid has a supercoiling rate of at least about 90%. In some embodiments, the method of the present disclosure further comprises a step of purifying the linearized plasmid. In some embodiments, the method of the present disclosure further comprises a step of purifying the mRNA.

In some embodiments, the method of the present disclosure further comprises a capping step and optionally a step of purifying the capped product. In some embodiments, the cap is a Cap1-type cap. The Cap1-type cap structure is shown in Formula (I):

cap G1G2 = m7G-5'-ppp-5'-Gm2'-3'-p-[m7 = 7-CH3; m2' = 2'-O-CH3; -ppp- = -PO2H-O-PO2H-O-PO2H)-; -p- = -PO2H-].

The capping reaction is shown as follows:

pppN1(p)Nx-OH(3') → ppN1(pN)x-OH(3') + Pi

ppN1(pN)x-OH(3') + GTP → G(5')ppp(5')N1(pN)x-OH(3') + PPi

G(5')ppp(5')N1(pN)x-OH(3') + AdoMet → m7G(5')ppp(5')N1(pN)x-OH(3') + AdoHyc

m7GpppN1(pN)x-OH(3') + AdoMet → m7Gppp[m2'-O]N1(pN)x-OH(3') + AdoHyc.

Additionally, further provided is a host cell, comprising the recombinant RNA molecule, DNA molecule, and/or vector according to the present disclosure, for example a bacterial cell. The vector according to the present disclosure (e.g., a plasmid) is stored and/or amplified in the host cell.

The host cell of the present disclosure may be prepared by transforming a competent host cell with the vector of the present disclosure. The competent host cell may be those capable of sequence-independent uptake of extracellular genetic materials, e.g., DNA plasmid. Various bacterial cells known to those skilled in the art can naturally take up exogenous DNA from the environment and thus may serve as the bacterial host cells of the present disclosure. Alternatively, a competent bacterial host cell may be obtained from a naturally non-competent bacterial cell by methods for example electroporation or chemical treatment (e.g., calcium ions treatment with thermal exposure). Following uptake, the DNA plasmid is preferably neither degraded nor integrated into the genomic DNA of the bacterial host cell. The bacterial host cell comprises Escherichia coli (E. coli) cells known in the art.

### VI. Lipid nanoparticle and pharmaceutical composition comprising the recombinant RNA molecule comprising the 5' and/or 3'-UTR of the present disclosure, and treatment/prevention of a disease

Provided is a lipid nanoparticle, comprising the recombinant RNA molecule, DNA molecule or vector according to the present disclosure.

In some embodiments, the lipid nanoparticle comprises a protonatable cationic lipid.

In some embodiments, the lipid nanoparticle further comprise one or more of a helper lipid, a structural lipid, and a PEG-lipid. In some further embodiments, the lipid nanoparticle further comprises a helper lipid, a structural lipid, and a PEG-lipid.

In some embodiments as described above herein, the helper lipid is a phospholipidsubstance. The phospholipid substance is typically semi-synthetic, and may also be naturally derived or chemically modified. The phospholipid species comprises, but not limited to DSPC (distearoylphosphocholine), DOPE (dioleoylphosphoethanolamine), DOPC (dioleoylphosphocholine), DOPS (dioleoylphosphoserine), DSPG (1,2-distearoyl-sn-glycero-3-phospho-(1'-rac-glycerol)), DPPG (dipalmitoylphosphoglycerol), DPPC (dipalmitoylphosphocholine), DGTS (1,2-dipalmitoyl-sn-glycero-3-O-4'-(N,N,N-trimethyl)-homoserine), Lysophospholipid, and the like. Preferably, the helper lipid is one or more selected from DSPC, DOPE, DOPC, and DOPS. In some embodiments, the helper lipid is DSPC and/or DOPE.

In some embodiments, the structural lipid is a sterol substance, including but not limited to cholesterol, cholesterol ester, sterol hormone, sterol vitamin, bile acid, cholesterin, ergosterol, β-sitosterol, and oxidized cholesterol derivative or the like. Preferably, the structural lipid is at least one selected from cholesterol, cholesterol ester, steroid hormone, sterol vitamin, and bile acid. In some embodiments, the structural lipid is cholesterol, preferably high-purity cholesterol, and more particularly injectable-grade high-purity cholesterol, such as CHO-HP (manufactured by AVT).

As used herein, the term PEG-lipid (PEGylated lipid) refers to a conjugate of polyethylene glycol and a lipid structure. Preferably, the PEG-lipid is selected from PEG-DMG and PEG-distearoylphosphatidylethanolamine (PEG-DSPE), preferably PEG-DMG. Preferably, the PEG-DMG is a polyethylene glycol (PEG) derivative of 1,2-dimyristoylglycerol. Preferably, the PEG has an average molecular weight of about 2000 - 5000, preferably about 2000.

In some further embodiments, the lipid nanoparticle further comprises the helper lipid, the structural lipid, and the said PEG-lipid. In some embodiments, based on the total amount of the protonatable cationic lipid, the helper lipid, the structural lipid, and the PEG-lipid, the lipid nanoparticle comprises the protonatable cationic lipid in the following amount (molar percentage): 25.0%-75.0%, for example, about 25.0%-28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0%, or 65.0%-75.0%.

Provided is a lipoplex, comprising the recombinant RNA molecule, DNA molecule or vector according to the present disclosure.

Provided is a proteoplex, comprising the recombinant RNA molecule, DNA molecule or vector according to the present disclosure.

Provided is a polylipoplex, comprising the recombinant RNA molecule, DNA molecule or vector according to the present disclosure.

Provided is a pharmaceutical composition, comprising the recombinant RNA molecule, DNA molecule, vector, host cell, lipoplex, proteoplex, polylipoplex or lipid nanoparticle according to the present disclosure and a pharmaceutically acceptable carrier, diluent or excipient.

Additionally, provided is also is use of the recombinant RNA molecule, the DNA molecule, the vector, the host cell, the lipid nanoparticle, or the pharmaceutical composition according to the present disclosure for the manufacture of a medicament.

In some embodiments, the medicament is used for gene therapy, gene vaccination, protein replacement therapy, antisense therapy, or RNA interference therapy.

In some embodiments, the medicament is a nucleic acid medicament, wherein the nucleic acid comprises at least one of: RNA, messenger RNA (mRNA), antisense oligonucleotide, DNA, plasmid, ribosomal RNA (rRNA), microRNA (miRNA), transfer RNA (tRNA), small interfering RNA (siRNA), small nuclear RNA (snRNA), short hairpin RNA (shRNA), single-guide RNA (sgRNA), and Cas9 mRNA.

In some embodiments, the medicament is used for the treatment and/or prevention of a disease. Preferably, the disease is selected from the group consisting of: a rare disease, an infectious disease, a cancer, a genetic disease, an autoimmune disease, a diabete disease, a neurodegenerative disease, a cardiovascular disease, a renal vascular disease, and a metabolic disease; preferably, the cancer comprises one or more of lung cancer, gastric cancer, liver cancer, esophageal cancer, colon cancer, pancreatic cancer, brain cancer, lymphoma, leukemia, or prostate cancer; the genetic disease comprises one or more of hemophilia, thalassemia, and Gaucher's disease.

In some embodiments, the medicament is a vaccine. In some embodiments, the recombinant molecule is used to produce an antigen of a pathogen or a fragment thereof.

In some embodiments, the medicament is a gene therapy agent. In some embodiments, the recombinant molecule is used to express a protein associated with a genetic disease.

In some embodiments, the recombinant molecule is used to produce an antibody, such as an scFv or a nanobody.

Further provided is a method for preventing or treating a disease, comprising administering to a subject in need thereof the recombinant RNA molecule or pharmaceutical composition according to the present disclosure.

In some embodiments, the disease is selected from the group consisting of: a rare disease, an infectious disease, a cancer, a genetic disease, an autoimmune disease, a diabete disease, a neurodegenerative disease, a cardiovascular disease, a renal vascular disease, and a metabolic disease. Preferably, the cancer comprises one or more of lung cancer, gastric cancer, liver cancer, esophageal cancer, colon cancer, pancreatic cancer, brain cancer, lymphoma, leukemia, or prostate cancer; the genetic disease comprises one or more of hemophilia, thalassemia, and Gaucher's disease.

In some embodiments, the recombinant RNA molecule or pharmaceutical composition is used as a prophylactic vaccine against a disease. In some embodiments, the recombinant RNA molecule is used to produce an antigen of a pathogen or a part thereof.

In some embodiments, the recombinant RNA molecule is used to produce a protein associated with a genetic disease.

In some embodiments, the recombinant RNA molecule is used to produce such as a scFv or a nanobody.

### VII. Beneficial effects of the present disclosure

Optimized UTRs are obtained through extensive screening in the present disclosure. The UTRs can increase the translation efficiency and/or stability of mRNA, increase the expression level of polypeptide and/or protein, and is significantly applicable in the development of mRNA vaccine.

### Examples

### Example 1. Construction of plasmid D

The construction procedures are as follows:

### Construction of plasmid B:

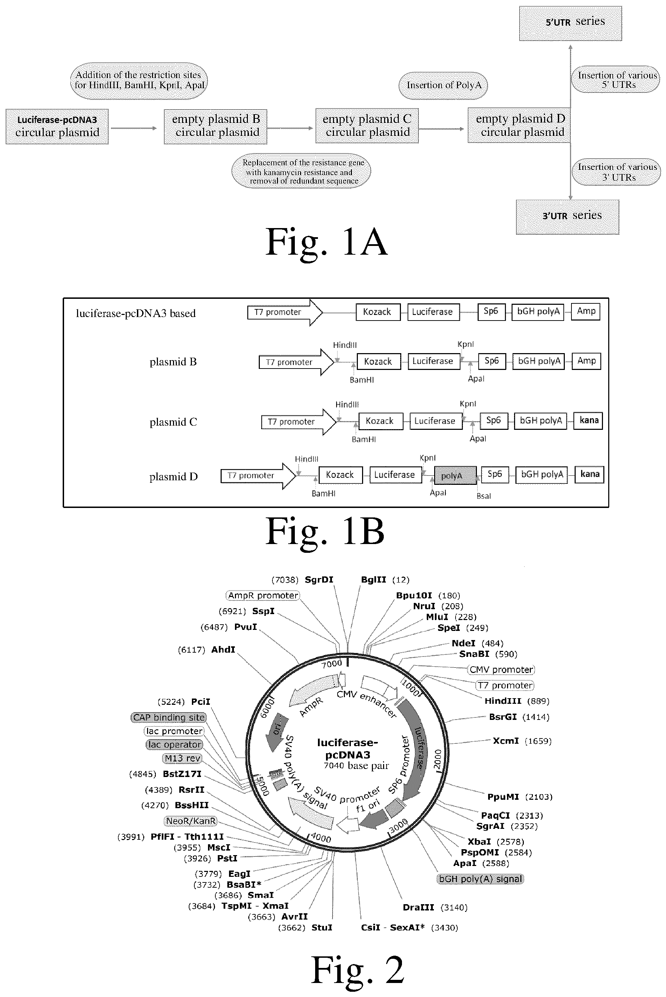

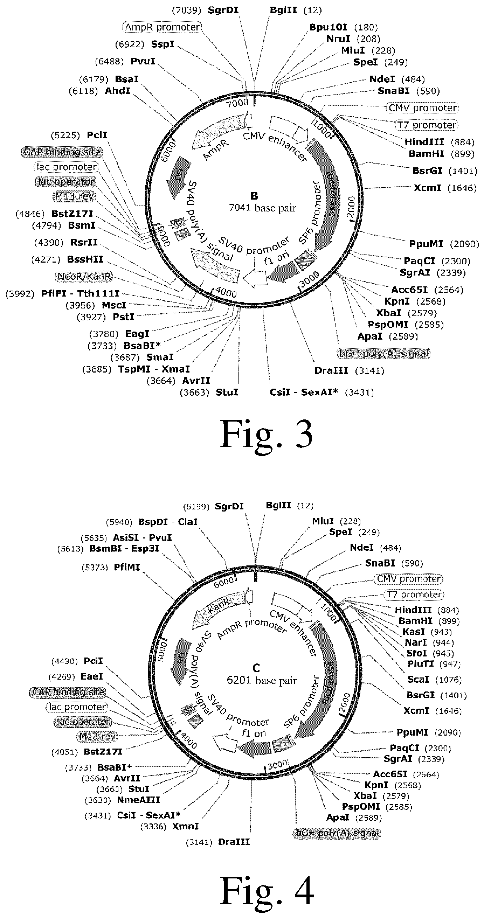

Using the Luciferase-pcDNA3 plasmid (purchased from Addgene, plasmid ID #18964) as the backbone, novel restriction sites Hindlll and BamHI were introduced upstream of the Kozak sequence, and additional restriction sites Kpnl and Apal were inserted downstream of the stop codon for luciferase, resulting in plasmid B. The nucleotide sequence of the Luciferase-pcDNA3 plasmid is provided in SEQ ID NO: 50, and the plasmid map of Luciferase-pcDNA3 is illustrated in Fig. 2. The nucleotide sequence of plasmid B is provided in SEQ ID NO: 51, and the plasmid map of plasmid B is depicted in Fig. 3.

### Construction of plasmid C:

The ampicillin (Amp) resistance gene in plasmid B was replaced with kanamycin (Kana) resistance gene, and the neo/KanR sequence from positions 3746 to 4540 was removed, resulting in plasmid C. The plasmid map of plasmid C is illustrated in Fig. 4, and the nucleotide sequence of plasmid C is provided in SEQ ID NO: 52.

### Construction of plasmid D:

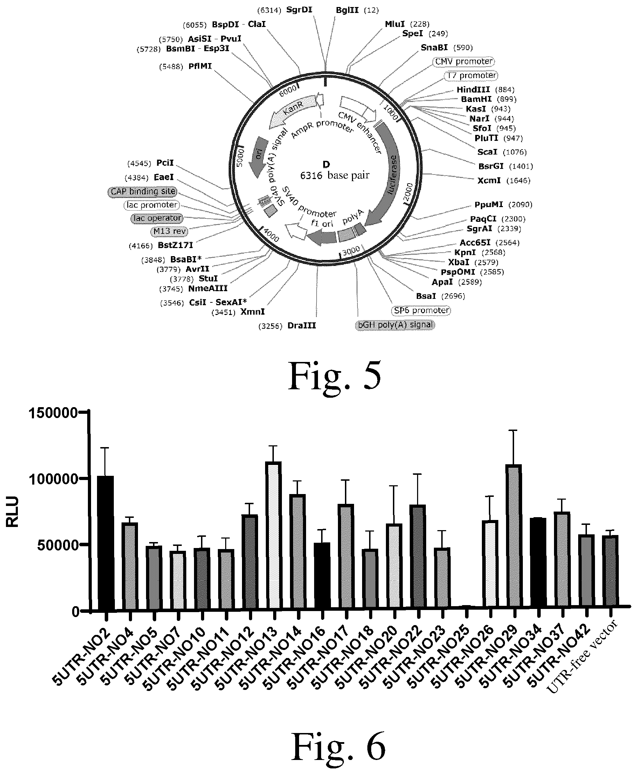

The poly(A) tail shown in SEQ ID NO: 53 was inserted into plasmid C. Specifically, plasmid C was single-digested with Apal, and the product was purified. The purified single-digested product and the poly(A) tail shown in SEQ ID NO: 53 were recombined using homologous recombination. The recombinant product was transformed into DH5α, and clones were screened on LB plates containing 50 µg/mL kanamycin. Clones with correct sequencing were selected, and plasmids were extracted to give plasmid D. The plasmid map of plasmid D is illustrated in Fig. 5, and the nucleotide sequence of plasmid D is provided in SEQ ID NO: 54.

The construction flowchart of plasmid D is shown in Fig. 1A, and the schematic diagram of the construction modifications is depicted in Fig. 1B.

### Example 2. Construction of plasmids containing different 5'-UTRs

5'-UTRs were designed and synthesized according to Table 1.

The 5'-UTR sequences were synthesized by Sangon Biotech (Shanghai) Co., Ltd. According to the quality analysis report provided by the company, the synthesized 5'-UTR sequences were consistent with the theoretically designed sequences.

Construction of plasmids containing different 5'-UTRs:
Plasmid D was double-digested with Hindlll and BamHI, and the gel was excised and purified (Axygen) to obtain fragment A with a molecular weight of approximately 6 kb. Different 5'-UTR sequences were introduced with homologous arm sequences by PCR and subjected to homologous recombination with fragment A using the homologous recombination enzyme system from Takara at 50°C for 15 min. The reaction mixture was transformed into DH5α competent cells (Takara) and plated on LB plates containing 50 µg/mL kanamycin. After 16 h of incubation, 3-4 single colonies were selected and cultured in medium containing 50 µg/mL kanamycin for 8 h. Plasmids were extracted and sequenced by Sangon Biotech (Shanghai) Co., Ltd., resulting in plasmids containing different 5'-UTRs.

### Example 3. Construction of plasmids containing different 3'-UTRs

3'-UTRs were designed and synthesized according to Table 1.

The 3'-UTR sequences were synthesized by Sangon Biotech (Shanghai) Co., Ltd. According to the quality analysis report provided by the company, the synthesized 3'-UTR sequences were consistent with the theoretically designed sequences.

Construction of plasmids containing different 3'-UTRs:
Plasmid D was double-digested with Kpnl and Apal, and the gel was excised and purified (Axygen) to obtain fragment A with a molecular weight of approximately 6 kb. Different 3'-UTR sequences were introduced with homologous arm sequences by PCR and subjected to homologous recombination with fragment A using the homologous recombination enzyme system from Takara at 50°C for 15 min. The reaction mixture was transformed into DH5α competent cells (Takara) and plated on LB plates containing 50 µg/mL kanamycin. After 16 h of incubation, 3-4 single colonies were selected and cultured in medium containing 50 µg/mL kanamycin for 8 h. Plasmids were extracted and sequenced by Sangon Biotech (Shanghai) Co., Ltd., resulting in plasmids containing different 3'-UTRs.

### Example 4. mRNA Synthesis

1. Plasmid extraction with plasmid supercoiling rate ≥90%.
2. Plasmid linearization
(1) Enzymatic linearization: 20 µg of plasmid was was subjected to linearization with corresponding enzyme (Bsal).
   Note: the reaction system was adjsted as needed. Due to columns of the purification kit, the plasmid in each reaction system did not exceed 20 µg.
(2) the reaction system was formulated in a 0.2 mL tube, blended, and incubated at 37°C in a thermostatic chamber overnight or for 2h for enzymatic digestion.
(3) 1 µL of the digested product with the original plasmid sample was subjected to electrophoresis to verify complete enzymatic digestion.

3. Purification of Linearized Plasmid with Takara Recovery kit (catalog no: 9761)
(1) 3× volume of Buffer DC (if the volume of Buffer DC to be added was less than 100 µL, 100 µL of Buffer DC should be added) was added to the PCR reaction solution (or other enzymatic reaction solution) with mixing evenly.
(2) The Spin Column of the kit was placed on the Collection Tube.
(3) The solution obtained from (1) was transferred into the Spin Column, which was centrifuged at 12,000 rpm for 1 min at room temperature, and the filtrate was discarded.
(4) 700 µL of Buffer WB was added in the Spin Column, which was centrifuged at 12,000 rpm for 30 s at room temperature, and the filtrate was discarded.

4. Phenol-Chloroform Extraction (to remove RNase, Proteins, etc.)
(1) The plasmid to be extracted was diluted to 300 µL or 500 µL, to which was add an equal volume of phenol-chloroform, and centrifuged at 12,000 rpm for 10 min.
(2) After centrifugation, the upper layer liquid was aspirated as much as possible, to which was then added an equal volume of phenol-chloroform again, and centrifuged at 12,000 rpm for 10 min.
(3) After centrifugation, the upper layer liquid was aspirated again as much as possible, to which was added 0.1 time of 5M NaCl and 0.7 time of isopropanol, and placed in an ice bath for 15 min. After that, centrifugation wad conducted at 12,000 rpm for 10 min, and the supernatant was discarded.
(4) Washing was conducted once with 200 µL of 70% ethanol (pre-cooled in an ice bath). Centrifugation was conducted at 12,000 rpm for 1 min, and the supernatant was discarded. Centrifugation was conducted at 12,000 rpm for another 1 minute, and the remaining liquid thoroughly aspirated with a small pipette tip.
(5) After air-drying at room temperature, an appropriate amount of UltraPure DNase/RNase-Free- Distilled Water was added with well mixing.
(6) The concentration of the purified sample was determined using OneDrop.
   Identification: 100 ng of the purified plasmid was subjected to electrophoresis with the original plasmid. After confirmation that the plasmid was completely linearized and there was no miscellaneous bands, it can be used for mRNA synthesis.

5. In vitro transcription of mRNA (by reference to the Novoprotein IVT reaction kit)
(1) Cleaning of the experimental area: Firstly, the biological safety cabinet was sterilized with ultraviolet light for 0.5 h. Then, the safety cabinet was wiped and cleaned with alcohol - soaked cotton balls and sprayed with RNase inhibitor. After 5 min, the inhibitor was wiped off with alcohol - soaked cotton balls, and then the RNA experiment was initiated.
(2) Configuration of the reaction system: Before preparation of the reaction system, the reagents were taken out, vortexed and mixed thoroughly on a vortex mixer, centrifuged, and then placed in an ice box for use.
   Note: During the process of the system configuration, all operations should be carried out on ice. The plasmid template and the IVT system should be prepared separately.
(3) Preparation of the IVT system: Each of the components except the plasmid template were added below the liquid surface of the system. Finally, the T7 enzyme was added with vortex for evenly mixing, wherein uracil (U) was replaced with N1-methylpseudouridine.
(4) The required amount of plasmid was taken out and transferred into a new PCR tube and incubated with the IVT system for 5 min. Finally, the two systems were mixed, vortexed and shaken for thoroughly mixing. After centrifugation, the mixture was incubated at 37°C for 2 h.
(5) The reaction system was prepared in a 0.2 mL flat - cap thin - wall tube. After mixing, it was placed it in a PCR instrument for reaction. The reaction conditions were set at 37°C for 2 h. Note: The reaction system can also be scaled up synchronously according to the required production volume.
(6) The linearized plasmid template was removed using DNase. To the reaction system was added (20µL system)1 µL/(100µL system)5µL DNase and the reaction system was incubated at 37°C for 15 min.

6. Purification procedures (by reference to Thermo MEGAclear Kit)
Column Purification
(1)Each of the reaction samples were transferred to a 1.5 - mL EP tube with gentle mixing. If the volume of the reaction system was less than 100 µL, the Elution Solution was used to make up the volume to 100 µL.
(2) 350 µL of Binding Solution Concentrate was added, which was gently mixed using a pipette.
(3) 250 µL of absolute ethanol was added, which was gently mixed using a pipette..
(4) The filter cartridge provided in the kit was inserted into the collection tube. 700 µL of the mixed solution was added to the filter cartridge, allowing binding at room temperature for 10 min. Centrifugation was conducted at 12,000 g for 1 min and the the filtrate was discarded and the collection tube was reused. Subsequently, the RNA was washed.
(5) 500 µL of Wash Solution was added, which was filtered through the filter cartridge (centrifugation at 12,000 g for 1 min).
(6) Step (5) was repeated.
(7) The Wash Solution was removed, and centrifugation was conducted at the maximum speed for 1 min to remove any residual Wash Solution.
(8) An appropriate amount (80 - 100 µL) of pre-heated RNase-Free Distilled Water was added to the filter cartridge. The lid was put on and allowed to stand at 70°C for 10 min, followed by centrifugation at 12,000 g for 1 min. The elution times can be increased as needed.
(9) Concentration determination:
The concentration was determined using onedrop or Qubit (measurement after dilution of the sample by a factor of 10).
7. Identification:
5 µL of the diluted purified sample was taken and mixed with NorthernMax^{™} Formaldehyde Load Dye, which was incubate at 75°C for 10 min, and then stored on ice.
Gel electrophoresis was conducted using 1% agarose gel to ensure that size of the mRNA was correct and the bands showed no smearing before the subsequent step of capping reaction.

8. Capping reaction (by reference to cellscript kit)
mRNACap1 type capping reaction:
Cap1 type cap structure and reaction principle were as follows:

pppN1(p)Nx-OH(3') → ppN1(pN)x-OH(3') + Pi

ppN1(pN)x-OH(3') + GTP → G(5')ppp(5')N1(pN)x-OH(3') + PPi

G(5')ppp(5')N1(pN)x-OH(3') + AdoMet → m7G(5')ppp(5')N1(pN)x-OH(3') + AdoHyc

m7GpppN1(pN)x-OH(3') + AdoMet → m7Gppp[m2'-O]N1(pN)x-OH(3') + AdoHyc
5'-Cap1 type cap structure:

cap G¹G² = m⁷G⁺-5'-ppp-5'-Gm^{2'}-3'-p-[m⁷ = 7-CH₃; m^{2'} = 2'-O-CH₃; -ppp- = -PO₂H-O-PO₂H-O-PO₂H)-; -p- = -PO₂H-], 37°C 5min or 65°C 5min(CELL SCRIPT, the reaction system was shown in Table 4).

**Table 3: Cap1 type capping reaction system**

| H₂O | Balanced to 67µL |
|---|---|
| No capping mRNA | 25 pmol |

| | |
|---|---|
| Note: Depending on the reaction system, the amount of mRNA capped in a single reaction (100 µL system) should not exceed 60 µg. | |

The capping (100 µL system) (referring to CELL SCRIPT capping reaction kit) was shown in Table 4.

**Table 4: 100µL capping system**

| | |
|---|---|
| 10X ScriptCap Capping Buffer | 10 µL |
| 10 mM GTP | 10 µL |
| 20 mM SAM | 2.5 µL |
| ScriptGuard RNase Inhibitor | 2.5 µL |
| ScriptCap 2'-O-Methyltransferase (100 U/µl) | 4 µL |
| ScriptCap Capping Enzyme (10 U/µl) | 4 µL |

The pre-heated mRNA was mixed with the above reaction system, and incubated at 37°C for 1 h.
9. Purification procedures: The same as the purification procedures for the products after in vitro transcription.

Characterization of data: The concentration was determined using onedrop or Qubit.

Experimental results: The final mRNA products containing Cap1 type cap and different UTRs were obtained, in which all uracil (U) nucleosides in the final mRNA products were replaced by N1-methyl pseudouridine.

### Example 5. Screening for 5'-UTR

The effect of the 5'-UTR on the expression level of luciferase was compared through the cellular luciferase reporter assay.

HEK293 cells were transfected with mRNAs containing different 5'-UTRs. Specifically, HEK293 cells were added to a 96-well plate at a density of 40,000 cells/well one day in advance. When the cell confluency reached 70%-90% on the next day, the cell transfection was carried out.

The transfection reagent lipoMAX (Invitrogen) was used according to the instructions of lipoMAX. mRNAs containing different 5'-UTRs were transfected into HEK293 cells respectively, with 100 ng of mRNA transfected in each well. After incubation in a 37°C CO₂ incubator for 16 h, the luciferase reporter assay was carried out (according to the instructions of Promega kit).

The experimental results were shown in Fig. 6. As compared to the mRNA without UTR, the mRNAs with 5UTR-NO2, 5UTR-NO4, 5UTR-NO12, 5UTR-NO13, 5UTR-NO14, 5UTR-NO17, 5UTR-NO20, 5UTR-NO22, 5UTR-NO26, 5UTR-NO29, 5UTR-NO34, and 5UTR-NO37 added could significantly increase the expression level of protein.

### Example 6. Screening for 3'-UTR

The effect of the 3'-UTR on the expression level of luciferase was compared through the cellular luciferase reporter assay.

HEK293 cells were transfected with mRNAs containing different 3'-UTRs. Specifically, HEK293 cells were added to a 96-well plate at a density of 40,000 cells/well one day in advance. When the cell confluency reached 70%-90% on the next day, the cell transfection was carried out.

The transfection reagent lipoMAX (Invitrogen) was used according to the instructions of lipoMAX. mRNAs containing different 3'-UTRs were transfected into HEK293 cells respectively, with 100 ng of mRNA transfected in each well. After incubation in a 37°C CO₂ incubator for 16 h, the luciferase reporter assay was carried out (according to the instructions of Promega kit).

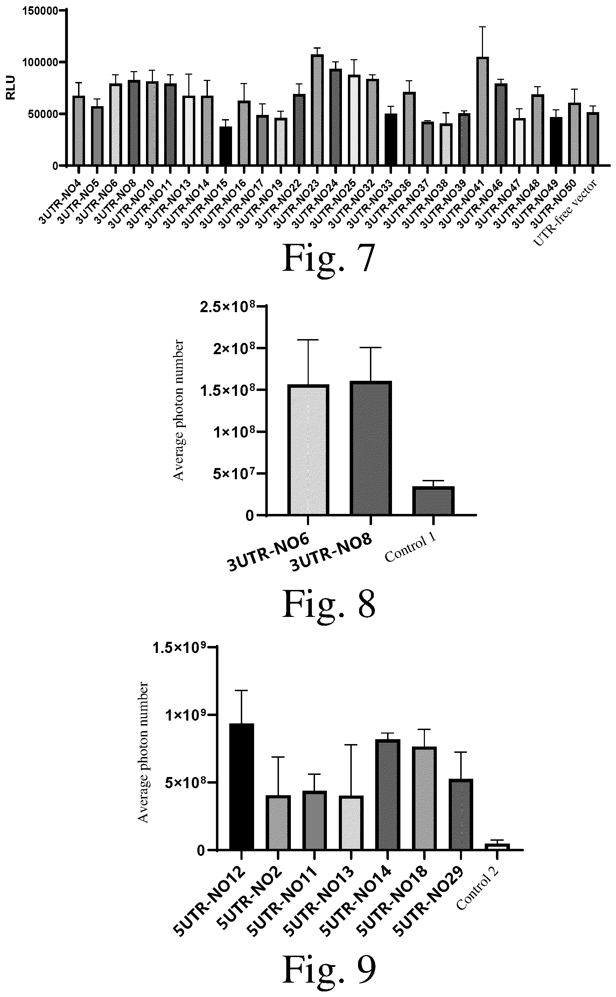

The experimental results were shown in Fig. 7. As compared to the mRNA without UTR, the mRNAs with 3UTR-NO4, 3UTR-NO5, 3UTR-NO6, 3UTR-NO8, 3UTR-NO10, 3UTR-NO11, 3UTR-NO13, 3UTR-NO14, 3UTR-NO16, 3UTR-NO22, 3UTR-NO23, 3UTR-NO24, 3UTR-NO25, 3UTR-NO32, 3UTR-NO36, 3UTR-NO41, 3UTR-NO46, 3UTR-NO48, 3UTR-NO50 added could significantly increase the expression level of protein.

### Example 7. Screening for the combination of 5'-UTR and 3'-UTR

I. The expressions of a series of mRNAs containing 5UTR-NO54 and one of different 3'-UTRs in mice were compared through in vivo imaging (IVIS, in vivo imaging). The specific procedures included:
   1. 5UTR-NO54 (DNA sequence as shown in SEQ ID NO: 55, synthesized by Sangon Biotech (Shanghai) Co., Ltd.) was inserted between Hindlll and BamHI of the blank plasmid D (referring to Example 1 for the construction procedures of the blank plasmid D). After fixation of the 5'-UTR sequence, 3UTR-NO6 and 3UTR-NO8 were inserted respectively between Apal and Kpnl of the plasmid containing 5UTR-NO54 to give a series of plasmids containing 5UTR-NO54 and one of different 3'-UTRs.
   2. By using the series of plasmids in step 1, a series of mRNAs containing 5UTR-NO54 and one of different 3'-UTRs were prepared with reference to Example 4, and then their respective LNP-mRNA formulations were prepared. The steps for preparing the LNP-mRNA formulations included:
      (1) LNP encapsulation: The total volume of the prepared solution (the sum of the aqueous phase and the alcohol phase in each system) was 1.5 mL, wherein the mass ratio of mRNA to lipid was 1:10, and the concentration of HAc-NaOAc buffer solution (0.2 M, pH 5.0) in the final aqueous phase was 0.025 M. A microfluidic device (MPE-L2) and a chip (SN.000035) were used to prepare each sample at aqueous phase: alcohol phase = 9 mL/min: 3 mL/min. The two phases were injected into the microfluidic chip for mixing according to the interface requirements. The aqueous phase contained 0.3 mg of mRNA with a total volume of 1125 µL; the alcohol phase contained lipids dissolved in ethanol, SM-102:DSPC:CHO-HP: DMG-PEG2000(Mol%)=50:10:38.5:1.5, with a total volume of 375 µL. The chemical structure of SM-102 was as follows: 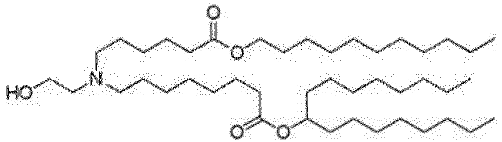 SM-102 was commercially purchased or prepared according to techniques well-known in the art.
      (2) Replacing the solution through dialysis to prepare the LNP-mRNA formulation:
         a. Preparation of the dialysis solution:
            1×PBS +8%(m/V) sucrose solution: 2 packages of 1×PBS prefabricated powder were taken and put into a beaker, which were dissolved and mixed thoroughly with 2 L of DEPC water. Then, 160 g of sucrose was added and mixed well to give the 1×PBS+8%(m/V) sucrose solution.
         b. Dialysis: The medicinal solutions of each group were loaded into 100KD dialysis bags respectively, which were immersed in a beaker filled with 1 L of the dialysis solution. The beaker was wrapped with aluminum foil paper and dialysis was conducted at room temperature for 1 h at a rotation speed of 100 rpm. Then, the dialysis solution was replaced, and the dialysis was continued for another 1 h to give different LNP-mRNA formulations.
   3. In vivo imaging experiments were conducted on different LNP-mRNA formulations obtained in step 2 respectively. The mice used in the experiments were female Balb/c mice at 6 weeks, 3 mice in each group. Each mouse was injected with 12 µg of the LNP-mRNA formulation via tail vein. After 12 h, the mice were anesthetized by inhaling isoflurane and injected with the luciferase imaging substrate D-Luciferin (150mg/kg). Then, the animals were placed in supine position and observed the signal distribution and intensity of luciferin in the mice using the IVIS in vivo imaging system.
   The results of the luciferase expression in mice for various LNP-mRNA formulations were shown in Fig. 8. The unit of the average photon number in Fig. 8 was p/s/cm²/sr. The only difference between Control 1 in Fig. 8 and other groups was that Control 1 did not contain 3'-UTR. The preparation procedures of the LNP-mRNA formulation of Control 1 were referred to the above. The results showed that, as compared to Control 1, the mRNAs containing 5UTR-NO54 and 3'-UTR selected from one of 3UTR-NO6 and 3UTR-NO8 achieved higher luciferase expression in mice.
II. The expressions of a series of mRNAs containing 3UTR-NO3 and one of different 5'-UTRs were compared in mice through in vivo imaging. The specific steps included:
   1. 3UTR-NO3 (DNA sequence as shown in SEQ ID NO: 56, synthesized by Sangon Biotech (Shanghai) Co., Ltd.) was inserted between Apal and Kpnl of the blank plasmid D (referring to Example 1 for the construction procedures of the blank plasmid D). After fixation of the 3'-UTR sequence, 5UTR-NO12, 5UTR-NO2, 5UTR-NO11, 5UTR-NO13, 5UTR-NO14, 5UTR-NO18, and 5UTR-NO29 were inserted respectively between Hindlll and BamHI of the plasmid containing 3UTR-NO3 to give a series of plasmids containing 3UTR-NO3 and one of different 5'-UTRs.
   2. By using the series of plasmids in step 1, a series of mRNAs containing 3UTR-NO3 and one of different 5'-UTRs were prepared with reference to the above, and then respective LNP-mRNA formulations were prepared.
   3. In vivo imaging experiments were conducted on different LNP-mRNA formulations obtained in step 2 respectively. The mice used in the experiments were female Balb/c mice at 6 weeks, 3 mice in each group. Each mouse was injected with 12 µg of the LNP-mRNA formulation via tail vein. After 12 h, the mice were anesthetized by inhaling isoflurane and injected with the luciferase imaging substrate D-Luciferin (150mg/kg). Then, the animals were placed in supine position and observed the signal distribution and intensity of luciferin in the mice using the IVIS in vivo imaging system.

The results of the luciferase expression in mice for various LNP-mRNA formulations were shown in Fig. 9. The unit of the average photon number in Fig. 9 was p/s/cm²/sr. The only difference between Control 2 in Fig. 9 and other groups was that Control 2 did not contain 5'-UTR. The preparation procedures of the LNP-mRNA formulation of Control 2 were referred to the above. The results showed that, as compared to Control 2, the mRNAs containing 3UTR-NO3 and 5'-UTR selected from one of 5UTR-NO12, 5UTR-NO2, 5UTR-NO11, 5UTR-NO13, 5UTR-NO14, 5UTR-NO18, and 5UTR-NO29 achieved higher luciferase expression in mice.

SEQ ID NO: 50

gacggatcgggagatctcccgatcccctatggtgcactctcagtacaatctgctctgatgccgcatagttaagccagtatctgctccctgcttgtg

tgttggaggtcgctgagtagtgcgcgagcaaaatttaagctacaacaaggcaaggcttgaccgacaattgcatgaagaatctgcttagggtt

aggcgttttgcgctgcttcgcgatgtacgggccagatatacgcgttgacattgattattgactagttattaatagtaatcaattacggggtcattagt

tcatagcccatatatggagttccgcgttacataacttacggtaaatggcccgcctggctgaccgcccaacgacccccgcccattgacgtcaat

aatgacgtatgttcccatagtaacgccaatagggactttccattgacgtcaatgggtggagtatttacggtaaactgcccacttggcagtacatc

aagtgtatcatatgccaagtacgccccctattgacgtcaatgacggtaaatggcccgcctggcattatgcccagtacatgaccttatgggacttt

cctacttggcagtacatctacgtattagtcatcgctattaccatggtgatgcggttttggcagtacatcaatgggcgtggatagcggtttgactcac

ggggatttccaagtctccaccccattgacgtcaatgggagtttgttttggcaccaaaatcaacgggactttccaaaatgtcgtaacaactccgc

cccattgacgcaaatgggcggtaggcgtgtacggtgggaggtctatataagcagagctctctggctaactagagaacccactgcttactggct

tatcgaaattaatacgactcactatagggagacccaagcttggcattccggtactgttggtaaagccaccatggaagacgccaaaaacata

aagaaaggcccggcgccattctatccgctggaagatggaaccgctggagagcaactgcataaggctatgaagagatacgccctggttcct

ggaacaattgcttttacagatgcacatatcgaggtggacatcacttacgctgagtacttcgaaatgtccgttcggttggcagaagctatgaaac

gatatgggctgaatacaaatcacagaatcgtcgtatgcagtgaaaactctcttcaattctttatgccggtgttgggcgcgttatttatcggagttgc

agttgcgcccgcgaacgacatttataatgaacgtgaattgctcaacagtatgggcatttcgcagcctaccgtggtgttcgtttccaaaaagggg

ttgcaaaaaattttgaacgtgcaaaaaaagctcccaatcatccaaaaaattattatcatggattctaaaacggattaccagggatttcagtcgat

gtacacgttcgtcacatctcatctacctcccggttttaatgaatacgattttgtgccagagtccttcgatagggacaagacaattgcactgatcatg

aactcctctggatctactggtctgcctaaaggtgtcgctctgcctcatagaactgcctgcgtgagattctcgcatgccagagatcctatttttggca

atcaaatcattccggatactgcgattttaagtgttgttccattccatcacggttttggaatgtttactacactcggatatttgatatgtggatttcgagtc

gtcttaatgtatagatttgaagaagagctgtttctgaggagccttcaggattacaagattcaaagtgcgctgctggtgccaaccctattctccttctt

cgccaaaagcactctgattgacaaatacgatttatctaatttacacgaaattgcttctggtggcgctcccctctctaaggaagtcggggaagcg

gttgccaagaggttccatctgccaggtatcaggcaaggatatgggctcactgagactacatcagctattctgattacacccgagggggatgat

aaaccgggcgcggtcggtaaagttgttccattttttgaagcgaaggttgtggatctggataccgggaaaacgctgggcgttaatcaaagagg

cgaactgtgtgtgagaggtcctatgattatgtccggttatgtaaacaatccggaagcgaccaacgccttgattgacaaggatggatggctacat

tctggagacatagcttactgggacgaagacgaacacttcttcatcgttgaccgcctgaagtctctgattaagtacaaaggctatcaggtggctc

ccgctgaattggaatccatcttgctccaacaccccaacatcttcgacgcaggtgtcgcaggtcttcccgacgatgacgccggtgaacttcccg

ccgccgttgttgttttggagcacggaaagacgatgacggaaaaagagatcgtggattacgtcgccagtcaagtaacaaccgcgaaaaagtt

gcgcggaggagttgtgtttgtggacgaagtaccgaaaggtcttaccggaaaactcgacgcaagaaaaatcagagagatcctcataaaggc

caagaagggcggaaagatcgccgtgtaattctagagggccctattctatagtgtcacctaaatgctagagctcgctgatcagcctcgactgtg

ccttctagttgccagccatctgttgtttgcccctcccccgtgccttccttgaccctggaaggtgccactcccactgtcctttcctaataaaatgagga

aattgcatcgcattgtctgagtaggtgtcattctattctggggggtggggtggggcaggacagcaagggggaggattgggaagacaatagca
ggcatgctggggatgcggtgggctctatggcttctgaggcggaaagaaccagctggggctctagggggtatccccacgcgccctgtagcgg
cgcattaagcgcggcgggtgtggtggttacgcgcagcgtgaccgctacacttgccagcgccctagcgcccgctcctttcgctttcttcccttcctt
tctcgccacgttcgccggctttccccgtcaagctctaaatcgggggctccctttagggttccgatttagtgctttacggcacctcgaccccaaaaa
acttgattagggtgatggttcacgtagtgggccatcgccctgatagacggtttttcgccctttgacgttggagtccacgttctttaatagtggactctt
gttccaaactggaacaacactcaaccctatctcggtctattcttttgatttataagggattttgccgatttcggcctattggttaaaaaatgagctgat
ttaacaaaaatttaacgcgaattaattctgtggaatgtgtgtcagttagggtgtggaaagtccccaggctccccagcaggcagaagtatgcaa
agcatgcatctcaattagtcagcaaccaggtgtggaaagtccccaggctccccagcaggcagaagtatgcaaagcatgcatctcaattagt
cagcaaccatagtcccgcccctaactccgcccatcccgcccctaactccgcccagttccgcccattctccgccccatggctgactaatttttttta
tttatgcagaggccgaggccgcctctgcctctgagctattccagaagtagtgaggaggcttttttggaggcctaggcttttgcaaaaagctcccg
ggagcttgtatatccattttcggatctgatcaagagacaggatgaggatcgtttcgcatgattgaacaagatggattgcacgcaggttctccggc
cgcttgggtggagaggctattcggctatgactgggcacaacagacaatcggctgctctgatgccgccgtgttccggctgtcagcgcaggggc
gcccggttctttttgtcaagaccgacctgtccggtgccctgaatgaactgcaggacgaggcagcgcggctatcgtggctggccacgacgggc
gttccttgcgcagctgtgctcgacgttgtcactgaagcgggaagggactggctgctattgggcgaagtgccggggcaggatctcctgtcatctc
accttgctcctgccgagaaagtatccatcatggctgatgcaatgcggcggctgcatacgcttgatccggctacctgcccattcgaccaccaag
cgaaacatcgcatcgagcgagcacgtactcggatggaagccggtcttgtcgatcaggatgatctggacgaagagcatcaggggctcgcgc
cagccgaactgttcgccaggctcaaggcgcgcatgcccgacggcgaggatctcgtcgtgacccatggcgatgcctgcttgccgaatatcat
ggtggaaaatggccgcttttctggattcatcgactgtggccggctgggtgtggcggaccgctatcaggacatagcgttggctacccgtgatatt
gctgaagagcttggcggcgaatgggctgaccgcttcctcgtgctttacggtatcgccgctcccgattcgcagcgcatcgccttctatcgccttctt
gacgagttcttctgagcgggactctggggttcgaaatgaccgaccaagcgacgcccaacctgccatcacgagatttcgattccaccgccgc
cttctatgaaaggttgggcttcggaatcgttttccgggacgccggctggatgatcctccagcgcggggatctcatgctggagttcttcgcccacc
ccaacttgtttattgcagcttataatggttacaaataaagcaatagcatcacaaatttcacaaataaagcatttttttcactgcattctagttgtggttt
gtccaaactcatcaatgtatcttatcatgtctgtataccgtcgacctctagctagagcttggcgtaatcatggtcatagctgtttcctgtgtgaaattgt
tatccgctcacaattccacacaacatacgagccggaagcataaagtgtaaagcctggggtgcctaatgagtgagctaactcacattaattgc
gttgcgctcactgcccgctttccagtcgggaaacctgtcgtgccagctgcattaatgaatcggccaacgcgcggggagaggcggtttgcgtat
tgggcgctcttccgcttcctcgctcactgactcgctgcgctcggtcgttcggctgcggcgagcggtatcagctcactcaaaggcggtaatacgg
ttatccacagaatcaggggataacgcaggaaagaacatgtgagcaaaaggccagcaaaaggccaggaaccgtaaaaaggccgcgttg
ctggcgtttttccataggctccgcccccctgacgagcatcacaaaaatcgacgctcaagtcagaggtggcgaaacccgacaggactataaa
gataccaggcgtttccccctggaagctccctcgtgcgctctcctgttccgaccctgccgcttaccggatacctgtccgcctttctcccttcgggaa
gcgtggcgctttctcatagctcacgctgtaggtatctcagttcggtgtaggtcgttcgctccaagctgggctgtgtgcacgaaccccccgttcagc

ccgaccgctgcgccttatccggtaactatcgtcttgagtccaacccggtaagacacgacttatcgccactggcagcagccactggtaacagg

attagcagagcgaggtatgtaggcggtgctacagagttcttgaagtggtggcctaactacggctacactagaagaacagtatttggtatctgc

gctctgctgaagccagttaccttcggaaaaagagttggtagctcttgatccggcaaacaaaccaccgctggtagcggtggtttttttgtttgcaa

gcagcagattacgcgcagaaaaaaaggatctcaagaagatcctttgatcttttctacggggtctgacgctcagtggaacgaaaactcacgtt

aagggattttggtcatgagattatcaaaaaggatcttcacctagatccttttaaattaaaaatgaagttttaaatcaatctaaagtatatatgagta

aacttggtctgacagttaccaatgcttaatcagtgaggcacctatctcagcgatctgtctatttcgttcatccatagttgcctgactccccgtcgtgta

gataactacgatacgggagggcttaccatctggccccagtgctgcaatgataccgcgagacccacgctcaccggctccagatttatcagca

ataaaccagccagccggaagggccgagcgcagaagtggtcctgcaactttatccgcctccatccagtctattaattgttgccgggaagctag

agtaagtagttcgccagttaatagtttgcgcaacgttgttgccattgctacaggcatcgtggtgtcacgctcgtcgtttggtatggcttcattcagctc

cggttcccaacgatcaaggcgagttacatgatcccccatgttgtgcaaaaaagcggttagctccttcggtcctccgatcgttgtcagaagtaag

ttggccgcagtgttatcactcatggttatggcagcactgcataattctcttactgtcatgccatccgtaagatgcttttctgtgactggtgagtactca

accaagtcattctgagaatagtgtatgcggcgaccgagttgctcttgcccggcgtcaatacgggataataccgcgccacatagcagaacttta

aaagtgctcatcattggaaaacgttcttcggggcgaaaactctcaaggatcttaccgctgttgagatccagttcgatgtaacccactcgtgcac

ccaactgatcttcagcatcttttactttcaccagcgtttctgggtgagcaaaaacaggaaggcaaaatgccgcaaaaaagggaataagggc

gacacggaaatgttgaatactcatactcttcctttttcaatattattgaagcatttatcagggttattgtctcatgagcggatacatatttgaatgtattt

agaaaaataaacaaataggggttccgcgcacatttccccgaaaagtgccacctgacgtc

SEQ ID NO: 51

gacggatcgggagatctcccgatcccctatggtgcactctcagtacaatctgctctgatgccgcatagttaagccagtatctgctccctgcttgtg

tgttggaggtcgctgagtagtgcgcgagcaaaatttaagctacaacaaggcaaggcttgaccgacaattgcatgaagaatctgcttagggtt

aggcgttttgcgctgcttcgcgatgtacgggccagatatacgcgttgacattgattattgactagttattaatagtaatcaattacggggtcattagt

tcatagcccatatatggagttccgcgttacataacttacggtaaatggcccgcctggctgaccgcccaacgacccccgcccattgacgtcaat

aatgacgtatgttcccatagtaacgccaatagggactttccattgacgtcaatgggtggagtatttacggtaaactgcccacttggcagtacatc

aagtgtatcatatgccaagtacgccccctattgacgtcaatgacggtaaatggcccgcctggcattatgcccagtacatgaccttatgggacttt

cctacttggcagtacatctacgtattagtcatcgctattaccatggtgatgcggttttggcagtacatcaatgggcgtggatagcggtttgactcac

ggggatttccaagtctccaccccattgacgtcaatgggagtttgttttggcaccaaaatcaacgggactttccaaaatgtcgtaacaactccgc

cccattgacgcaaatgggcggtaggcgtgtacggtgggaggtctatataagcagagctctctggctaactagagaacccactgcttactggct

tatcgaaattaatacgactcactatagggaaagcttgatcagaacggatccgccaccatggaagacgccaaaaacataaagaaaggccc

ggcgccattctatccgctggaagatggaaccgctggagagcaactgcataaggctatgaagagatacgccctggttcctggaacaattgcttt

tacagatgcacatatcgaggtggacatcacttacgctgagtacttcgaaatgtccgttcggttggcagaagctatgaaacgatatgggctgaat

acaaatcacagaatcgtcgtatgcagtgaaaactctcttcaattctttatgccggtgttgggcgcgttatttatcggagttgcagttgcgcccgcga
acgacatttataatgaacgtgaattgctcaacagtatgggcatttcgcagcctaccgtggtgttcgtttccaaaaaggggttgcaaaaaattttga
acgtgcaaaaaaagctcccaatcatccaaaaaattattatcatggattctaaaacggattaccagggatttcagtcgatgtacacgttcgtcac
atctcatctacctcccggttttaatgaatacgattttgtgccagagtccttcgatagggacaagacaattgcactgatcatgaactcctctggatct
actggtctgcctaaaggtgtcgctctgcctcatagaactgcctgcgtgagattctcgcatgccagagatcctatttttggcaatcaaatcattccg
gatactgcgattttaagtgttgttccattccatcacggttttggaatgtttactacactcggatatttgatatgtggatttcgagtcgtcttaatgtataga
tttgaagaagagctgtttctgaggagccttcaggattacaagattcaaagtgcgctgctggtgccaaccctattctccttcttcgccaaaagcact
ctgattgacaaatacgatttatctaatttacacgaaattgcttctggtggcgctcccctctctaaggaagtcggggaagcggttgccaagaggtt
ccatctgccaggtatcaggcaaggatatgggctcactgagactacatcagctattctgattacacccgagggggatgataaaccgggcgcg
gtcggtaaagttgttccattttttgaagcgaaggttgtggatctggataccgggaaaacgctgggcgttaatcaaagaggcgaactgtgtgtga
gaggtcctatgattatgtccggttatgtaaacaatccggaagcgaccaacgccttgattgacaaggatggatggctacattctggagacatag
cttactgggacgaagacgaacacttcttcatcgttgaccgcctgaagtctctgattaagtacaaaggctatcaggtggctcccgctgaattgga
atccatcttgctccaacaccccaacatcttcgacgcaggtgtcgcaggtcttcccgacgatgacgccggtgaacttcccgccgccgttgttgtttt
ggagcacggaaagacgatgacggaaaaagagatcgtggattacgtcgccagtcaagtaacaaccgcgaaaaagttgcgcggaggagt
tgtgtttgtggacgaagtaccgaaaggtcttaccggaaaactcgacgcaagaaaaatcagagagatcctcataaaggccaagaagggcg
gaaagatcgccgtgtaaggtacccgatcagattctagagggccctattctatagtgtcacctaaatgctagagctcgctgatcagcctcgactg
tgccttctagttgccagccatctgttgtttgcccctcccccgtgccttccttgaccctggaaggtgccactcccactgtcctttcctaataaaatgag
gaaattgcatcgcattgtctgagtaggtgtcattctattctggggggtggggtggggcaggacagcaagggggaggattgggaagacaata
gcaggcatgctggggatgcggtgggctctatggcttctgaggcggaaagaaccagctggggctctagggggtatccccacgcgccctgtag
cggcgcattaagcgcggcgggtgtggtggttacgcgcagcgtgaccgctacacttgccagcgccctagcgcccgctcctttcgctttcttccctt
cctttctcgccacgttcgccggctttccccgtcaagctctaaatcgggggctccctttagggttccgatttagtgctttacggcacctcgaccccaa
aaaacttgattagggtgatggttcacgtagtgggccatcgccctgatagacggtttttcgccctttgacgttggagtccacgttctttaatagtgga
ctcttgttccaaactggaacaacactcaaccctatctcggtctattcttttgatttataagggattttgccgatttcggcctattggttaaaaaatgagc
tgatttaacaaaaatttaacgcgaattaattctgtggaatgtgtgtcagttagggtgtggaaagtccccaggctccccagcaggcagaagtatg
caaagcatgcatctcaattagtcagcaaccaggtgtggaaagtccccaggctccccagcaggcagaagtatgcaaagcatgcatctcaatt
agtcagcaaccatagtcccgcccctaactccgcccatcccgcccctaactccgcccagttccgcccattctccgccccatggctgactaatttt
ttttatttatgcagaggccgaggccgcctctgcctctgagctattccagaagtagtgaggaggcttttttggaggcctaggcttttgcaaaaagctc
ccgggagcttgtatatccattttcggatctgatcaagagacaggatgaggatcgtttcgcatgattgaacaagatggattgcacgcaggttctcc
ggccgcttgggtggagaggctattcggctatgactgggcacaacagacaatcggctgctctgatgccgccgtgttccggctgtcagcgcagg
ggcgcccggttctttttgtcaagaccgacctgtccggtgccctgaatgaactgcaggacgaggcagcgcggctatcgtggctggccacgacg

ggcgttccttgcgcagctgtgctcgacgttgtcactgaagcgggaagggactggctgctattgggcgaagtgccggggcaggatctcctgtca

tctcaccttgctcctgccgagaaagtatccatcatggctgatgcaatgcggcggctgcatacgcttgatccggctacctgcccattcgaccacc

aagcgaaacatcgcatcgagcgagcacgtactcggatggaagccggtcttgtcgatcaggatgatctggacgaagagcatcaggggctc

gcgccagccgaactgttcgccaggctcaaggcgcgcatgcccgacggcgaggatctcgtcgtgacccatggcgatgcctgcttgccgaat

atcatggtggaaaatggccgcttttctggattcatcgactgtggccggctgggtgtggcggaccgctatcaggacatagcgttggctacccgtg

atattgctgaagagcttggcggcgaatgggctgaccgcttcctcgtgctttacggtatcgccgctcccgattcgcagcgcatcgccttctatcgc

cttcttgacgagttcttctgagcgggactctggggttcgaaatgaccgaccaagcgacgcccaacctgccatcacgagatttcgattccaccg

ccgccttctatgaaaggttgggcttcggaatcgttttccgggacgccggctggatgatcctccagcgcggggatctcatgctggagttcttcgcc

caccccaacttgtttattgcagcttataatggttacaaataaagcaatagcatcacaaatttcacaaataaagcatttttttcactgcattctagttgt

ggtttgtccaaactcatcaatgtatcttatcatgtctgtataccgtcgacctctagctagagcttggcgtaatcatggtcatagctgtttcctgtgtgaa

attgttatccgctcacaattccacacaacatacgagccggaagcataaagtgtaaagcctggggtgcctaatgagtgagctaactcacattaa

ttgcgttgcgctcactgcccgctttccagtcgggaaacctgtcgtgccagctgcattaatgaatcggccaacgcgcggggagaggcggtttgc

gtattgggcgctcttccgcttcctcgctcactgactcgctgcgctcggtcgttcggctgcggcgagcggtatcagctcactcaaaggcggtaata

cggttatccacagaatcaggggataacgcaggaaagaacatgtgagcaaaaggccagcaaaaggccaggaaccgtaaaaaggccgc

gttgctggcgtttttccataggctccgcccccctgacgagcatcacaaaaatcgacgctcaagtcagaggtggcgaaacccgacaggactat

aaagataccaggcgtttccccctggaagctccctcgtgcgctctcctgttccgaccctgccgcttaccggatacctgtccgcctttctcccttcgg

gaagcgtggcgctttctcatagctcacgctgtaggtatctcagttcggtgtaggtcgttcgctccaagctgggctgtgtgcacgaaccccccgttc

agcccgaccgctgcgccttatccggtaactatcgtcttgagtccaacccggtaagacacgacttatcgccactggcagcagccactggtaac

aggattagcagagcgaggtatgtaggcggtgctacagagttcttgaagtggtggcctaactacggctacactagaagaacagtatttggtatc

tgcgctctgctgaagccagttaccttcggaaaaagagttggtagctcttgatccggcaaacaaaccaccgctggtagcggtggtttttttgtttgc

aagcagcagattacgcgcagaaaaaaaggatctcaagaagatcctttgatcttttctacggggtctgacgctcagtggaacgaaaactcac

gttaagggattttggtcatgagattatcaaaaaggatcttcacctagatccttttaaattaaaaatgaagttttaaatcaatctaaagtatatatgag

taaacttggtctgacagttaccaatgcttaatcagtgaggcacctatctcagcgatctgtctatttcgttcatccatagttgcctgactccccgtcgtg

tagataactacgatacgggagggcttaccatctggccccagtgctgcaatgataccgcgagacccacgctcaccggctccagatttatcagc

aataaaccagccagccggaagggccgagcgcagaagtggtcctgcaactttatccgcctccatccagtctattaattgttgccgggaagcta

gagtaagtagttcgccagttaatagtttgcgcaacgttgttgccattgctacaggcatcgtggtgtcacgctcgtcgtttggtatggcttcattcagc

tccggttcccaacgatcaaggcgagttacatgatcccccatgttgtgcaaaaaagcggttagctccttcggtcctccgatcgttgtcagaagta

agttggccgcagtgttatcactcatggttatggcagcactgcataattctcttactgtcatgccatccgtaagatgcttttctgtgactggtgagtact

caaccaagtcattctgagaatagtgtatgcggcgaccgagttgctcttgcccggcgtcaatacgggataataccgcgccacatagcagaact

ttaaaagtgctcatcattggaaaacgttcttcggggcgaaaactctcaaggatcttaccgctgttgagatccagttcgatgtaacccactcgtgc

acccaactgatcttcagcatcttttactttcaccagcgtttctgggtgagcaaaaacaggaaggcaaaatgccgcaaaaaagggaataagg

gcgacacggaaatgttgaatactcatactcttcctttttcaatattattgaagcatttatcagggttattgtctcatgagcggatacatatttgaatgta

tttagaaaaataaacaaataggggttccgcgcacatttccccgaaaagtgccacctgacgtc

SEQ ID NO: 52

gacggatcgggagatctcccgatcccctatggtgcactctcagtacaatctgctctgatgccgcatagttaagccagtatctgctccctgcttgtg

tgttggaggtcgctgagtagtgcgcgagcaaaatttaagctacaacaaggcaaggcttgaccgacaattgcatgaagaatctgcttagggtt

aggcgttttgcgctgcttcgcgatgtacgggccagatatacgcgttgacattgattattgactagttattaatagtaatcaattacggggtcattagt

tcatagcccatatatggagttccgcgttacataacttacggtaaatggcccgcctggctgaccgcccaacgacccccgcccattgacgtcaat

aatgacgtatgttcccatagtaacgccaatagggactttccattgacgtcaatgggtggagtatttacggtaaactgcccacttggcagtacatc

aagtgtatcatatgccaagtacgccccctattgacgtcaatgacggtaaatggcccgcctggcattatgcccagtacatgaccttatgggacttt

cctacttggcagtacatctacgtattagtcatcgctattaccatggtgatgcggttttggcagtacatcaatgggcgtggatagcggtttgactcac

ggggatttccaagtctccaccccattgacgtcaatgggagtttgttttggcaccaaaatcaacgggactttccaaaatgtcgtaacaactccgc

cccattgacgcaaatgggcggtaggcgtgtacggtgggaggtctatataagcagagctctctggctaactagagaacccactgcttactggct

tatcgaaattaatacgactcactatagggaaagcttgatcagaacggatccgccaccatggaagacgccaaaaacataaagaaaggccc

ggcgccattctatccgctggaagatggaaccgctggagagcaactgcataaggctatgaagagatacgccctggttcctggaacaattgcttt

tacagatgcacatatcgaggtggacatcacttacgctgagtacttcgaaatgtccgttcggttggcagaagctatgaaacgatatgggctgaat

acaaatcacagaatcgtcgtatgcagtgaaaactctcttcaattctttatgccggtgttgggcgcgttatttatcggagttgcagttgcgcccgcga

acgacatttataatgaacgtgaattgctcaacagtatgggcatttcgcagcctaccgtggtgttcgtttccaaaaaggggttgcaaaaaattttga

acgtgcaaaaaaagctcccaatcatccaaaaaattattatcatggattctaaaacggattaccagggatttcagtcgatgtacacgttcgtcac

atctcatctacctcccggttttaatgaatacgattttgtgccagagtccttcgatagggacaagacaattgcactgatcatgaactcctctggatct

actggtctgcctaaaggtgtcgctctgcctcatagaactgcctgcgtgagattctcgcatgccagagatcctatttttggcaatcaaatcattccg

gatactgcgattttaagtgttgttccattccatcacggttttggaatgtttactacactcggatatttgatatgtggatttcgagtcgtcttaatgtataga

tttgaagaagagctgtttctgaggagccttcaggattacaagattcaaagtgcgctgctggtgccaaccctattctccttcttcgccaaaagcact

ctgattgacaaatacgatttatctaatttacacgaaattgcttctggtggcgctcccctctctaaggaagtcggggaagcggttgccaagaggtt

ccatctgccaggtatcaggcaaggatatgggctcactgagactacatcagctattctgattacacccgagggggatgataaaccgggcgcg

gtcggtaaagttgttccattttttgaagcgaaggttgtggatctggataccgggaaaacgctgggcgttaatcaaagaggcgaactgtgtgtga

gaggtcctatgattatgtccggttatgtaaacaatccggaagcgaccaacgccttgattgacaaggatggatggctacattctggagacatag

cttactgggacgaagacgaacacttcttcatcgttgaccgcctgaagtctctgattaagtacaaaggctatcaggtggctcccgctgaattgga

atccatcttgctccaacaccccaacatcttcgacgcaggtgtcgcaggtcttcccgacgatgacgccggtgaacttcccgccgccgttgttgttt

ggagcacggaaagacgatgacggaaaaagagatcgtggattacgtcgccagtcaagtaacaaccgcgaaaaagttgcgcggaggagt
tgtgtttgtggacgaagtaccgaaaggtcttaccggaaaactcgacgcaagaaaaatcagagagatcctcataaaggccaagaagggcg
gaaagatcgccgtgtaaggtacccgatcagattctagagggccctattctatagtgtcacctaaatgctagagctcgctgatcagcctcgactg
tgccttctagttgccagccatctgttgtttgcccctcccccgtgccttccttgaccctggaaggtgccactcccactgtcctttcctaataaaatgag
gaaattgcatcgcattgtctgagtaggtgtcattctattctggggggtggggtggggcaggacagcaagggggaggattgggaagacaata
gcaggcatgctggggatgcggtgggctctatggcttctgaggcggaaagaaccagctggggctctagggggtatccccacgcgccctgtag
cggcgcattaagcgcggcgggtgtggtggttacgcgcagcgtgaccgctacacttgccagcgccctagcgcccgctcctttcgctttcttccctt
cctttctcgccacgttcgccggctttccccgtcaagctctaaatcgggggctccctttagggttccgatttagtgctttacggcacctcgaccccaa
aaaacttgattagggtgatggttcacgtagtgggccatcgccctgatagacggtttttcgccctttgacgttggagtccacgttctttaatagtgga
ctcttgttccaaactggaacaacactcaaccctatctcggtctattcttttgatttataagggattttgccgatttcggcctattggttaaaaaatgagc
tgatttaacaaaaatttaacgcgaattaattctgtggaatgtgtgtcagttagggtgtggaaagtccccaggctccccagcaggcagaagtatg
caaagcatgcatctcaattagtcagcaaccaggtgtggaaagtccccaggctccccagcaggcagaagtatgcaaagcatgcatctcaatt
agtcagcaaccatagtcccgcccctaactccgcccatcccgcccctaactccgcccagttccgcccattctccgccccatggctgactaattttt
tttatttatgcagaggccgaggccgcctctgcctctgagctattccagaagtagtgaggaggcttttttggaggcctaggcttttgcaaaaagctc
ccgggagcttgtatatccattttcggatctgatcaagagacaggatgaggatcgtttcgcgcgggactctggggttcgaaatgaccgaccaag
cgacgcccaacctgccatcacgagatttcgattccaccgccgccttctatgaaaggttgggcttcggaatcgttttccgggacgccggctggat
gatcctccagcgcggggatctcatgctggagttcttcgcccaccccaacttgtttattgcagcttataatggttacaaataaagcaatagcatca
caaatttcacaaataaagcatttttttcactgcattctagttgtggtttgtccaaactcatcaatgtatcttatcatgtctgtataccgtcgacctctagct
agagcttggcgtaatcatggtcatagctgtttcctgtgtgaaattgttatccgctcacaattccacacaacatacgagccggaagcataaagtgt
aaagcctggggtgcctaatgagtgagctaactcacattaattgcgttgcgctcactgcccgctttccagtcgggaaacctgtcgtgccagctgc
attaatgaatcggccaacgcgcggggagaggcggtttgcgtattgggcgctcttccgcttcctcgctcactgactcgctgcgctcggtcgttcgg
ctgcggcgagcggtatcagctcactcaaaggcggtaatacggttatccacagaatcaggggataacgcaggaaagaacatgtgagcaaa
aggccagcaaaaggccaggaaccgtaaaaaggccgcgttgctggcgtttttccataggctccgcccccctgacgagcatcacaaaaatcg
acgctcaagtcagaggtggcgaaacccgacaggactataaagataccaggcgtttccccctggaagctccctcgtgcgctctcctgttccga
ccctgccgcttaccggatacctgtccgcctttctcccttcgggaagcgtggcgctttctcatagctcacgctgtaggtatctcagttcggtgtaggtc
gttcgctccaagctgggctgtgtgcacgaaccccccgttcagcccgaccgctgcgccttatccggtaactatcgtcttgagtccaacccggtaa
gacacgacttatcgccactggcagcagccactggtaacaggattagcagagcgaggtatgtaggcggtgctacagagttcttgaagtggtg
gcctaactacggctacactagaagaacagtatttggtatctgcgctctgctgaagccagttaccttcggaaaaagagttggtagctcttgatccg
gcaaacaaaccaccgctggtagcggtggtttttttgtttgcaagcagcagattacgcgcagaaaaaaaggatctcaagaagatcctttgatctt
ttctacggggtctgacgctcagtggaacgaaaactcacgttaagggattttggtcatgagattatcaaaaaggatcttcacctagatccttttaaa

ttaaaaatgaagttttaaatcaatctaaagtatatatgagtaaacttggtctgacagttagaaaaactcatcgagcatcaaatgaaactgcaattt
attcatatcaggattatcaataccatatttttgaaaaagccgtttctgtaatgaaggagaaaactcaccgaggcagttccataggatggcaaga
tcctggtatcggtctgcgattccgactcgtccaacatcaatacaacctattaatttcccctcgtcaaaaataaggttatcaagtgagaaatcacc
atgagtgacgactgaatccggtgagaatggcaaaagtttatgcatttctttccagacttgttcaacaggccagccattacgctcgtcatcaaaat
cactcgcatcaaccaaaccgttattcattcgtgattgcgcctgagcgagacgaaatacgcgatcgctgttaaaaggacaattacaaacagga
atcgaatgcaaccggcgcaggaacactgccagcgcatcaacaatattttcacctgaatcaggatattcttctaatacctggaatgctgttttccc
ggggatcgcagtggtgagtaaccatgcatcatcaggagtacggataaaatgcttgatggtcggaagaggcataaattccgtcagccagttta
gtctgaccatctcatctgtaacatcattggcaacgctacctttgccatgtttcagaaacaactctggcgcatcgggcttcccatacaatcgataga
ttgtcgcacctgattgcccgacattatcgcgagcccatttatacccatataaatcagcatccatgttggaatttaatcgcggcctagagcaagac
gtttcccgttgaatatggctcatactcttcctttttcaatattattgaagcatttatcagggttattgtctcatgagcggatacatatttgaatgtatttaga
aaaataaacaaataggggttccgcgcacatttccccgaaaagtgccacctgacgtc

SEQ ID NO: 53

aaaaaaaaaaaaaaaaaaaagcatatgactaaaaaaaaaaaaaaaaaaaaaaaaaaaaaaaaaaaaaaaaaaaaaaaaaaaa

aaaaaaaaaaaaaaaaaaaaaaaaaaaaaa

SEQ ID NO: 54

gacggatcgggagatctcccgatcccctatggtgcactctcagtacaatctgctctgatgccgcatagttaagccagtatctgctccctgcttgtg

tgttggaggtcgctgagtagtgcgcgagcaaaatttaagctacaacaaggcaaggcttgaccgacaattgcatgaagaatctgcttagggtt

aggcgttttgcgctgcttcgcgatgtacgggccagatatacgcgttgacattgattattgactagttattaatagtaatcaattacggggtcattagt

tcatagcccatatatggagttccgcgttacataacttacggtaaatggcccgcctggctgaccgcccaacgacccccgcccattgacgtcaat

aatgacgtatgttcccatagtaacgccaatagggactttccattgacgtcaatgggtggagtatttacggtaaactgcccacttggcagtacatc

aagtgtatcatatgccaagtacgccccctattgacgtcaatgacggtaaatggcccgcctggcattatgcccagtacatgaccttatgggacttt

cctacttggcagtacatctacgtattagtcatcgctattaccatggtgatgcggttttggcagtacatcaatgggcgtggatagcggtttgactcac

ggggatttccaagtctccaccccattgacgtcaatgggagtttgttttggcaccaaaatcaacgggactttccaaaatgtcgtaacaactccgc

cccattgacgcaaatgggcggtaggcgtgtacggtgggaggtctatataagcagagctctctggctaactagagaacccactgcttactggct

tatcgaaattaatacgactcactatagggaaagcttgatcagaacggatccgccaccatggaagacgccaaaaacataaagaaaggccc

ggcgccattctatccgctggaagatggaaccgctggagagcaactgcataaggctatgaagagatacgccctggttcctggaacaattgcttt

tacagatgcacatatcgaggtggacatcacttacgctgagtacttcgaaatgtccgttcggttggcagaagctatgaaacgatatgggctgaat

acaaatcacagaatcgtcgtatgcagtgaaaactctcttcaattctttatgccggtgttgggcgcgttatttatcggagttgcagttgcgcccgcga

acgacatttataatgaacgtgaattgctcaacagtatgggcatttcgcagcctaccgtggtgttcgtttccaaaaaggggttgcaaaaaattttga
acgtgcaaaaaaagctcccaatcatccaaaaaattattatcatggattctaaaacggattaccagggatttcagtcgatgtacacgttcgtcac
atctcatctacctcccggttttaatgaatacgattttgtgccagagtccttcgatagggacaagacaattgcactgatcatgaactcctctggatct
actggtctgcctaaaggtgtcgctctgcctcatagaactgcctgcgtgagattctcgcatgccagagatcctatttttggcaatcaaatcattccg
gatactgcgattttaagtgttgttccattccatcacggttttggaatgtttactacactcggatatttgatatgtggatttcgagtcgtcttaatgtataga
tttgaagaagagctgtttctgaggagccttcaggattacaagattcaaagtgcgctgctggtgccaaccctattctccttcttcgccaaaagcact
ctgattgacaaatacgatttatctaatttacacgaaattgcttctggtggcgctcccctctctaaggaagtcggggaagcggttgccaagaggtt
ccatctgccaggtatcaggcaaggatatgggctcactgagactacatcagctattctgattacacccgagggggatgataaaccgggcgcg
gtcggtaaagttgttccattttttgaagcgaaggttgtggatctggataccgggaaaacgctgggcgttaatcaaagaggcgaactgtgtgtga
gaggtcctatgattatgtccggttatgtaaacaatccggaagcgaccaacgccttgattgacaaggatggatggctacattctggagacatag
cttactgggacgaagacgaacacttcttcatcgttgaccgcctgaagtctctgattaagtacaaaggctatcaggtggctcccgctgaattgga
atccatcttgctccaacaccccaacatcttcgacgcaggtgtcgcaggtcttcccgacgatgacgccggtgaacttcccgccgccgttgttgtttt
ggagcacggaaagacgatgacggaaaaagagatcgtggattacgtcgccagtcaagtaacaaccgcgaaaaagttgcgcggaggagt
tgtgtttgtggacgaagtaccgaaaggtcttaccggaaaactcgacgcaagaaaaatcagagagatcctcataaaggccaagaagggcg
gaaagatcgccgtgtaaggtacccgatcagattctagagggcccaaaaaaaaaaaaaaaaaaaaaaaaaaaaaagcatatgactaaa
aaaaaaaaaaaaaaaaaaaaaaaaaaaaaaaaaaaaaaaaaaaaaaaaaaaaaaaaaaaaaaaaaaaaaaagagaccgtctatag
tgtcacctaaatgctagagctcgctgatcagcctcgactgtgccttctagttgccagccatctgttgtttgcccctcccccgtgccttccttgaccct
ggaaggtgccactcccactgtcctttcctaataaaatgaggaaattgcatcgcattgtctgagtaggtgtcattctattctggggggtggggtggg
gcaggacagcaagggggaggattgggaagacaatagcaggcatgctggggatgcggtgggctctatggcttctgaggcggaaagaacc
agctggggctctagggggtatccccacgcgccctgtagcggcgcattaagcgcggcgggtgtggtggttacgcgcagcgtgaccgctacac
ttgccagcgccctagcgcccgctcctttcgctttcttcccttcctttctcgccacgttcgccggctttccccgtcaagctctaaatcgggggctcccttt
agggttccgatttagtgctttacggcacctcgaccccaaaaaacttgattagggtgatggttcacgtagtgggccatcgccctgatagacggtttt
tcgccctttgacgttggagtccacgttctttaatagtggactcttgttccaaactggaacaacactcaaccctatctcggtctattcttttgatttataa
gggattttgccgatttcggcctattggttaaaaaatgagctgatttaacaaaaatttaacgcgaattaattctgtggaatgtgtgtcagttagggtgt
ggaaagtccccaggctccccagcaggcagaagtatgcaaagcatgcatctcaattagtcagcaaccaggtgtggaaagtccccaggctcc
ccagcaggcagaagtatgcaaagcatgcatctcaattagtcagcaaccatagtcccgcccctaactccgcccatcccgcccctaactccgc
ccagttccgcccattctccgccccatggctgactaattttttttatttatgcagaggccgaggccgcctctgcctctgagctattccagaagtagtg
aggaggcttttttggaggcctaggcttttgcaaaaagctcccgggagcttgtatatccattttcggatctgatcaagagacaggatgaggatcgtt
tcgcgcgggactctggggttcgaaatgaccgaccaagcgacgcccaacctgccatcacgagatttcgattccaccgccgccttctatgaaa
ggttgggcttcggaatcgttttccgggacgccggctggatgatcctccagcgcggggatctcatgctggagttcttcgcccaccccaacttgttta

ttgcagcttataatggttacaaataaagcaatagcatcacaaatttcacaaataaagcatttttttcactgcattctagttgtggtttgtccaaactca
tcaatgtatcttatcatgtctgtataccgtcgacctctagctagagcttggcgtaatcatggtcatagctgtttcctgtgtgaaattgttatccgctcac
aattccacacaacatacgagccggaagcataaagtgtaaagcctggggtgcctaatgagtgagctaactcacattaattgcgttgcgctcact
gcccgctttccagtcgggaaacctgtcgtgccagctgcattaatgaatcggccaacgcgcggggagaggcggtttgcgtattgggcgctcttc
cgcttcctcgctcactgactcgctgcgctcggtcgttcggctgcggcgagcggtatcagctcactcaaaggcggtaatacggttatccacaga
atcaggggataacgcaggaaagaacatgtgagcaaaaggccagcaaaaggccaggaaccgtaaaaaggccgcgttgctggcgtttttc
cataggctccgcccccctgacgagcatcacaaaaatcgacgctcaagtcagaggtggcgaaacccgacaggactataaagataccagg
cgtttccccctggaagctccctcgtgcgctctcctgttccgaccctgccgcttaccggatacctgtccgcctttctcccttcgggaagcgtggcgct
ttctcatagctcacgctgtaggtatctcagttcggtgtaggtcgttcgctccaagctgggctgtgtgcacgaaccccccgttcagcccgaccgct
gcgccttatccggtaactatcgtcttgagtccaacccggtaagacacgacttatcgccactggcagcagccactggtaacaggattagcaga
gcgaggtatgtaggcggtgctacagagttcttgaagtggtggcctaactacggctacactagaagaacagtatttggtatctgcgctctgctga
agccagttaccttcggaaaaagagttggtagctcttgatccggcaaacaaaccaccgctggtagcggtggtttttttgtttgcaagcagcagatt
acgcgcagaaaaaaaggatctcaagaagatcctttgatcttttctacggggtctgacgctcagtggaacgaaaactcacgttaagggattttg
gtcatgagattatcaaaaaggatcttcacctagatccttttaaattaaaaatgaagttttaaatcaatctaaagtatatatgagtaaacttggtctga
cagttagaaaaactcatcgagcatcaaatgaaactgcaatttattcatatcaggattatcaataccatatttttgaaaaagccgtttctgtaatga
aggagaaaactcaccgaggcagttccataggatggcaagatcctggtatcggtctgcgattccgactcgtccaacatcaatacaacctatta
atttcccctcgtcaaaaataaggttatcaagtgagaaatcaccatgagtgacgactgaatccggtgagaatggcaaaagtttatgcatttctttc
cagacttgttcaacaggccagccattacgctcgtcatcaaaatcactcgcatcaaccaaaccgttattcattcgtgattgcgcctgagcgagac
gaaatacgcgatcgctgttaaaaggacaattacaaacaggaatcgaatgcaaccggcgcaggaacactgccagcgcatcaacaatatttt
cacctgaatcaggatattcttctaatacctggaatgctgttttcccggggatcgcagtggtgagtaaccatgcatcatcaggagtacggataaa
atgcttgatggtcggaagaggcataaattccgtcagccagtttagtctgaccatctcatctgtaacatcattggcaacgctacctttgccatgtttc
agaaacaactctggcgcatcgggcttcccatacaatcgatagattgtcgcacctgattgcccgacattatcgcgagcccatttatacccatata
aatcagcatccatgttggaatttaatcgcggcctagagcaagacgtttcccgttgaatatggctcatactcttcctttttcaatattattgaagcattt
atcagggttattgtctcatgagcggatacatatttgaatgtatttagaaaaataaacaaataggggttccgcgcacatttccccgaaaagtgcca
cctgacgtc

SEQ ID NO: 55

TAATACGACTCACTATAGGACAGATCGCCTGGAGACGCCATCCACGCTGTTTTGACCTCCATAGA

AGACACCGGGACCGATCCAGCCTCCGCGGCCGGGAACGGTGCATTGGAACGCGGATTCCCCG

TGCCAAGAGTGACGTAAGTACCGCAGTCACCGTCCTTGACACG

SEQ ID NO: 56

TCTTCACCCAGTCTGTCTCTTAGAATTTATTTCCCACTGACACAAACAAGCCATTTTGTCTGCTTG

TATAACCC

## Claims

1. A recombinant RNA molecule, comprising:
(1) a first nucleotide sequence encoding a polypeptide and/or protein of interest; and
(2) a second nucleotide sequence comprising a 5'-untranslated region (5'-UTR);
wherein the 5'-UTR comprises at least one polynucleotide selected from:
(a) a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7;
(b) a fragment of the 5'-UTR in (a);
(c) a variant of the 5'-UTR in (a); and
(d) a variant of the fragment in (b);
the first nucleotide sequence and the second nucleotide sequence are not naturally occurring in the same RNA molecule.

2. The recombinant RNA molecule according to claim 1, wherein the gene is a human gene.

3. The recombinant RNA molecule of claim 1 or 2, wherein the second nucleotide sequence comprises at least one of the following polynucleotides:
(a) a 5'-UTR derived from at least one gene of genes PPIA, HPX, FTCD, CDK5RAP3, HSPA8, HBA1, and HBB;
(b) a fragment of the 5'-UTR in (a);
(c) a variant of the 5'-UTR in (a); and
(d) a variant of the fragment in (b).

4. The recombinant RNA molecule according to any one of claims 1-3, wherein the second nucleotide sequence comprises at least one of the following polynucleotides:
an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21;
preferably, the variant of the RNA encoded by a polynucleotide having a sequence as set forth in at least one of SEQ ID NOs: 1-21, the fragment of the RNA encoded by a polynucleotide having a sequence as set forth in at least one of SEQ ID NOs: 1-21, and the variant of the fragment of the RNA encoded by a polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 1-21.

5. The recombinant RNA molecule according to any one of claims 1-4, wherein the second nucleotide sequence comprises at least one of:
the 5'-UTRs of at least two genes from the genes in (a); the fragments of the 5'-UTRs of at least two genes from the genes in (a); the variants of the 5'-UTRs of at least two genes from the genes in (a) and the variants of the fragments of the 5'-UTRs of at least two genes from the genes in (a).

6. The recombinant RNA molecule according to any one of claims 1-4, wherein the second nucleotide sequence comprises at least one of:
at least two copies of the 5'-UTR in (a); at least two copies of the fragment of the 5'-UTR in (a); at least two copies of the variant of the 5'-UTR in (a), and at least two copies of the variant of the fragment of the 5'-UTR in (a).

7. The recombinant RNA molecule according to any one of claims 1-6, further comprising at least one of a promoter, a 5'-cap structure, a 3'-UTR, and a poly(A) tail.

8. The recombinant RNA molecule according to claim 7, wherein the 5'-cap structure comprises at least one of m⁷GpppG, m₂^{7,3'-O}GpppG, m⁷Gppp(5')N1 and m⁷Gppp(m^{2'-O})N1.

9. The recombinant RNA molecule according to claim 7 or 8, wherein the 3'-UTR comprises at least one of:
i) a 3'-UTR derived from at least one gene of albumin gene, α-globin gene, β-globin gene, tyrosine hydroxylase gene, lipoxygenase gene, and collagen α gene;
ii) a variant of the 3'-UTR in i);
iii) a 3'-UTR derived from at least one gene of genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, NFKB2, and GH1, a fragment, a variant, or a variant of a fragment thereof;
preferably, at least one of an RNA encoded by a polynucleotide having a sequence as set forth in at least one of SEQ ID NOs: 22-49, a fragment of the RNA encoded by a polynucleotide having a sequence as set forth in at least one of SEQ ID NOs: 22-49, a variant of the RNA encoded by a polynucleotide having a sequence as set forth in at least one of SEQ ID NOs: 22-49, and a variant of the fragment of the RNA encoded by a polynucleotide having a sequence as set forth in at least one of SEQ ID NOs: 22-49;
preferably, a fragment of the RNA encoded by a polynucleotide having a sequence as set forth in at least one of SEQ ID NOs: 22-49, a variant of the RNA encoded by a polynucleotide having a sequence as set forth in at least one of SEQ ID NOs: 22-49, and a variant of the fragment of the RNA encoded by a polynucleotide having a sequence as set forth in at least one of SEQ ID NOs: 22-49 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 22-49;
iv) at least two copies of one of the polynucleotides in i), ii), or iii); or
v) at least two polynucleotides selected from the group consisting of polynucleotides in i)-iii).

10. The recombinant RNA molecule according to any one of claims 7-9, wherein the nucleotides of the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides.

11. The recombinant RNA molecule according to any one of claims 7-10, wherein the nucleotides forming the poly(A) tail comprise one or more nucleotides other than A nucleotide;
optionally, the nucleotides of the poly(A) tail comprise two or more consecutive nucleotides other than A nucleotide.

12. A recombinant RNA molecule, comprising:
(1) a first nucleotide sequence encoding a polypeptide and/or protein of interest; and
(2) a second nucleotide sequence comprising a 3'-untranslated region (3'-UTR);
wherein the 3'-UTR comprises at least one polynucleotide selected from:
(a) a 3'-UTR derived from at least one gene selected from genes MPND, FBXW10, FBXW12, PGLYRP1, HPX, CDK7, APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2;
(b) a fragment of the 3'-UTR in (a);
(c) a variant of the 3'-UTR in (a); and
(d) a variant of the fragment in (b);
wherein the first nucleotide sequence and the second nucleotide sequence are not naturally occurring in the same RNA molecule.

13. The recombinant RNA molecule according to claim 12, wherein the gene is a human gene.

14. The recombinant RNA molecule of claim 12 or 13, wherein the second nucleotide sequence comprises at least one of the following polynucleotides:
(a) a 3'-UTR derived from at least one gene of genes MPND, FBXW10, FBXW12, and PGLYRP1;
(b) a fragment of the 3'-UTR in (a);
(c) a variant of the 3'-UTR in (a); and
(d) a variant of the fragment in (b).

15. The recombinant RNA molecule according to any one of claims 12-14, wherein the second nucleotide sequence comprises at least one of the following polynucleotides:
an RNA encoded by a polynucleotide having a sequence as set forth in at least one of SEQ ID NOs: 22-48; a fragment of the RNA encoded by a polynucleotide having a sequence as set forth in at least one of SEQ ID NOs: 22-48; a variant of the RNA encoded by a polynucleotide having a sequence as set forth in at least one of SEQ ID NOs: 22-48; and a variant of the fragment of the RNA encoded by a polynucleotide having a sequence as set forth in at least one of SEQ ID NOs: 22-48;
preferably, the variant of the RNA encoded by a polynucleotide having a sequence as set forth in at least one of SEQ ID NOs: 22-48, the fragment of the RNA encoded by a polynucleotide having a sequence as set forth in at least one of SEQ ID NOs: 22-48, and the variant of the fragment of the RNA encoded by a polynucleotide having a sequence as set forth in at least one of SEQ ID NOs: 22-48 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 22-48.

16. The recombinant RNA molecule according to any one of claims 12-15, wherein the second nucleotide sequence comprises at least one of:
the 3'-UTRs of at least two genes from the genes in (a); the fragments of the 3'-UTRs of at least two genes from the genes in (a); the variants of the 3'-UTRs of at least two genes from the genes in (a) and the fragments of the variants of the 3'-UTRs of at least two genes from the genes in (a).

17. The recombinant RNA molecule according to any one of claims 12-15, wherein the second nucleotide sequence comprises at least one of:
at least two copies of the 3'-UTR in (a); at least two copies of the fragment of the 3'-UTR in (a); at least two copies of the variant of the 3'-UTR in (a) and at least two copies of the variant of the fragment of the 3'-UTR in (a).

18. The recombinant RNA molecule according to any one of claims 12-17, further comprising at least one of a promoter, a 5'-cap structure, a 5'-UTR, and a poly(A) tail.

19. The recombinant RNA molecule according to claim 17, wherein the 5'-cap structure comprises at least one of m⁷GpppG, m₂^{7,3'-O}GpppG, m⁷Gppp(5')N1 or m⁷Gppp(m^{2'-O})N1.

20. The recombinant RNA molecule according to claim 18 or 19, wherein the 5'-UTR comprises:
i) at least one of a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7; a fragment, a variant or a variant of a fragment thereof;
preferably, an RNA encoded by a polynucleotide having a sequence as set forth in at least one of SEQ ID NOs: 1-21; a fragment of the RNA encoded by a polynucleotide having a sequence as set forth in at least one of SEQ ID NOs: 1-21; a variant of the RNA encoded by a polynucleotide having a sequence as set forth in at least one of SEQ ID NOs: 1-21; and a variant of the fragment of the RNA encoded by a polynucleotide having a sequence as set forth in at least one of SEQ ID NOs: 1-21;
preferably, the variant of the RNA encoded by a polynucleotide having a sequence as set forth in at least one of SEQ ID NOs: 1-21, the fragment of the RNAencoded by a polynucleotide having a sequence as set forth in at least one of SEQ ID NOs: 1-21, and the variant of the fragment of the RNA encoded by a polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% identity to the RNA encoded by a polynucleotide having a sequence as set forth in at least one of SEQ ID NOs: 1-21;
ii) at least two copies of one of the polynucleotides in i); or
iii) at least two polynucleotides in i).

21. The recombinant RNA molecule according to any one of claims 18-20, wherein the nucleotides of the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides.

22. The recombinant RNA molecule according to any one of claims 18-21, wherein the nucleotides of the poly(A) tail comprise one or more nucleotides other than A nucleotide.

23. A DNA molecule, encoding the recombinant RNA molecule according to any one of claims 1-22.

24. A vector, comprising the DNA molecule according to claim 23.

25. A host cell, comprising the recombinant RNA molecule according to any one of claims 1-22, the DNA molecule according to claim 23 or the vector according to claim 24.

26. A lipid nanoparticle, comprising the recombinant RNA molecule according to any one of claims 1-22.

27. A pharmaceutical composition comprising the recombinant RNA molecule according to any one of claims 1-22, the DNA molecule according to claim 23, the vector according to claim 24, the host cell according to claim 25, or the lipid nanoparticle according to claim 26, and a pharmaceutically acceptable carrier.

28. A vector, comprising a first nucleotide sequence encoding a 5'-UTR and/or a second nucleotide sequence encoding a 3'-UTR, wherein:
the first nucleotide sequence comprises at least one of the following polynucleotides:
(a) a polynucleotide encoding a 5'-UTR derived from at least one gene of genes PPIA, HPX, FTCD, CDK5RAP3, HSPA8, HBA1, HBB, MYSM1, LENG1, TMSB4X, CASP4, IFNA1, PGLYRP1, UCHL1, CPAMD8, TTR, APOA2, GH1, DTYMK, APOC2, and CDK7;
(b) a polynucleotide encoding a fragment of the 5'-UTR in (a);
(c) a polynucleotide encoding a variant of the 5'-UTR in (a); and
(d) a polynucleotide encoding a variant of the fragment in (b);
the second nucleotide sequence comprises at least one of the following polynucleotides:
(e) a polynucleotide encoding a 3'-UTR derived from at least one gene of genes MPND, FBXW10, FBXW12, PGLYRP1, HPX, CDK7,APOC2, PFN1, RBP4, FTCD, NAAA, ALB, GSDMD, FBXL8, ORM1, CASP4, CHMP2A, LENG1, MYCBPAP, APOC1, GAPDH, HSPA8, APOA2, UCHL1, TSG101, NAE1, and NFKB2;
(f) a polynucleotide encoding a fragment of the 3'-UTR in (e);
(g) a polynucleotide encoding a variant of the 3'-UTR in (e); and
(h) a polynucleotide encoding a variant of the fragment in (f).

29. The vector according to claim 28, wherein the gene is a human gene.

30. The vector according to claim 28 or 29, wherein the first nucleotide sequence comprises a 5'-UTR derived from at least one gene of genes PPIA, HPX, FTCD, CDK5RAP3, HSPA8, HBA1, and HBB, or a variant thereof.

31. The vector according to any one of claims 28-30, wherein the second nucleotide sequence comprises a 3'-UTR derived from at least one gene of genes MPND, FBXW10, FBXW12, and PGLYRP1, or a variant thereof.

32. The vector according to any one of claims 28-31, comprising the first nucleotide sequence and the second nucleotide sequence.

33. The vector according to any one of claims 28-32, wherein the first nucleotide sequence comprises:
a polynucleotide having a sequence as set forth in at least one of SEQ ID NOs: 1-21; a fragment of the polynucleotide having a sequence as set forth in at least one of SEQ ID NOs: 1-21; a variant of the polynucleotide having a sequence as set forth in at least one of SEQ ID NOs: 1-21; and a variant of the fragment of the polynucleotide having a sequence as set forth in at least one of SEQ ID NOs: 1-21;
preferably, the variant of the polynucleotide having a sequence as set forth in at least one of SEQ ID NOs: 1-21, the fragment of the polynucleotide having a sequence as set forth in at least one of SEQ ID NOs: 1-21, and the variant of the fragment of the polynucleotide having a sequence as set forth in at least one of SEQ ID NOs: 1-21 have at least 70%, 80%, 85%, 90%, 91%, 92%, 93%, 94%, 95%, 96%, 97%, 98%, or 99% homology to the polynucleotide having a sequence as set forth in at least one of SEQ ID NOs: 1-21;
ii) at least two copies of one of the polynucleotides in i); or
iii) at least two polynucleotides in i).

34. The vector according to any one of claims 28-33, wherein the second nucleotide sequence comprises:
(1) a polynucleotide encoding a 3'-UTR derived from at least one gene of albumin gene, α-globin gene, β-globin gene, tyrosine hydroxylase gene, lipoxygenase gene, and collagen α gene;
(2) a polynucleotide encoding a variant of the 3'-UTR in (1);
(3) at least one of a polynucleotide having a sequence as set forth in at least one of SEQ ID NOs: 22-48; a fragment of the polynucleotide having a sequence as set forth in at least one of SEQ ID NOs: 22-48; a variant of the polynucleotide having a sequence as set forth in at least one of SEQ ID NOs: 22-48; and a variant of the fragment of the polynucleotide having a sequence as set forth in at least one of SEQ ID NOs: 22-48;
preferably, the RNA encoded by the polynucleotide having a sequence as set forth in at least one of SEQ ID NOs: 22-48, the fragment encoded by the polynucleotide having a sequence as set forth in at least one of SEQ ID NOs: 22-48, and the variant of the fragment encoded by the polynucleotide having a sequence as set forth in at least one of SEQ ID NOs: 22-48 have at least 70%, 80%, 85%, 90%, 91%, 92%, 93%, 94%, 95%, 96%, 97%, 98%, or 99% identity to the polynucleotide having a sequence as set forth in at least one of SEQ ID NOs: 22-48;
(4) at least two copies of one of the polynucleotides in (1), (2) or (3); or
(5) at least two polynucleotides selected from the group consisting of polynucleotides in (1)-(3).

35. The vector according to any one of claims 28-34, further comprising a polynucleotide encoding a poly(A) tail.

36. The vector according to claims 35, wherein the nucleotides of the poly(A) tail comprise at least 20, at least 40, at least 80, at least 100, or at least 120 A nucleotides; preferably, the nucleotides of the poly(A) tail comprise at least 20, at least 40, at least 80, at least 100, or at least 120 consecutive A nucleotides.

37. The vector according to claims 35, wherein the nucleotides of the poly(A) tail comprise one or more nucleotides other than A nucleotide.

38. Use of the recombinant RNA molecule according to any one of claims 1-22, the DNA molecule according to claim 23, the vector according to claim 24 or 28, the host cell according to claim 25, the lipid nanoparticle according to claim 26, or the pharmaceutical composition according to claim 27 in the manufacture of a medicament;
preferably, the medicament is used for gene therapy, gene vaccination, or protein replacement therapy.

39. The use according to claim 38, wherein the medicament is a nucleic acid medicament, and wherein the nucleic acid comprises at least one of RNA, messenger RNA (mRNA), DNA, plasmid, ribosomal RNA (rRNA), single-guide RNA (sgRNA), and Cas9 mRNA.

40. The use according to any one of claims 38-39, wherein the medicament is used for the treatment and/or prevention of a disease;
preferably, the disease is selected from the group consisting of: a rare disease, an infectious disease, a cancer, a genetic disease, an autoimmune disease, a diabete disease, a neurodegenerative disease, a cardiovascular disease, a renal vascular disease, and a metabolic disease;
preferably, the cancer comprises one or more of lung cancer, gastric cancer, liver cancer, esophageal cancer, colon cancer, pancreatic cancer, brain cancer, lymphoma, leukemia, or prostate cancer; the genetic disease comprises one or more of hemophilia, thalassemia, and Gaucher's disease.
